# EUROPEAN PATENT SPECIFICATION

(11) **EP 4 077 721 B1**
(45) Date of publication and mention of the grant of the patent: **05.03.2025**
(21) Application number: 20903846.2
(22) Date of filing: 18.12.2020
(51) Int. Cl.: C12Q 1/6827, C12Q 1/6883, C12Q 1/6869, C12Q 1/6886, G16B 20/20

(54) **CELL-FREE DNA FRAGMENTATION AND NUCLEASES**
ZELLFREIE DNA-FRAGMENTIERUNG UND NUKLEASEN
FRAGMENTATION D'ADN ACELLULAIRE ET NUCLÉASES

(30) Priority: 18.12.2019 US 201962949867 P; 08.01.2020 US 202062958651 P
(43) Date of publication of application: 26.10.2022
(60) Divisional application: 25154452.4
(73) Proprietor: The Chinese University Of Hong Kong, Hong Kong (HK); Grail, Inc., Menlo Park, California 94025 (US)
(72) Inventor: LO, Yuk-Ming Dennis, Hong Kong (HK); CHIU, Rossa, Wai Kwun, Hong Kong (HK); HAN, Diana, Siao Cheng, Hong Kong (HK); NI, Meng, Hong Kong (HK)
(74) Representative: J A Kemp LLP
(86) International application number: PCT/CN2020/137516
(87) International publication number: WO 2021/121368

(56) References cited:
- WO-A1-2013/177086
- WO-A1-2016/065295
- WO-A1-2017/012592
- WO-A1-2017/012592
- WO-A1-2017/218512
- WO-A1-2018/031808
- CN-A- 107 002 122
- CN-A- 108 884 491
- DINEIKA CHANDRANANDA ET AL: "High-resolution characterization of sequence signatures due to non-random cleavage of cell-free DNA", BMC MEDICAL GENOMICS, vol. 8, no. 1, 17 June 2015 (2015-06-17), XP055450660, DOI: 10.1186/s12920-015-0107-z
- SERPAS LEE ET AL: "Dnase1l3 deletion causes aberrations in length and end-motif frequencies in plasma DNA", PROCEEDINGS OF THE NATIONAL ACADEMY OF SCIENCES, vol. 116, no. 2, 8 January 2019 (2019-01-08), pages 641 - 649, XP055822531, ISSN: 0027-8424, Retrieved from the Internet <URL:https://www.pnas.org/content/116/2/641.short> DOI: 10.1073/pnas.1815031116
- HAN DIANA S.C. ET AL: "The Biology of Cell-free DNA Fragmentation and the Roles of DNASE1, DNASE1L3, and DFFB", THE AMERICAN JOURNAL OF HUMAN GENETICS, vol. 106, no. 2, 6 February 2020 (2020-02-06), US, pages 202 - 214, XP055822533, ISSN: 0002-9297, Retrieved from the Internet <URL:https://www.sciencedirect.com/science/article/pii/S0002929720300082> DOI: 10.1016/j.ajhg.2020.01.008
- HAN DIANA S.C., NI MENG, CHAN REBECCA W.Y., CHAN VICKEN W.H., LUI KATHY O., CHIU ROSSA W.K., LO Y.M. DENNIS: "The Biology of Cell-free DNA Fragmentation and the Roles of DNASE1, DNASE1L3, and DFFB", THE AMERICAN JOURNAL OF HUMAN GENETICS, vol. 106, no. 2, 6 February 2020 (2020-02-06), pages 202 - 214, XP055822533, ISSN: 0002-9297, DOI: 10.1016/j.ajhg.2020.01.008
- SERPAS LEE, CHAN REBECCA W. Y., JIANG PEIYONG, NI MENG, SUN KUN, RASHIDFARROKHI ALI, SONI CHETNA, SISIRAK VANJA, LEE WING-SHAN, CH: "Dnase1l3 deletion causes aberrations in length and end-motif frequencies in plasma DNA", PROCEEDINGS OF THE NATIONAL ACADEMY OF SCIENCES, vol. 116, no. 2, 8 January 2019 (2019-01-08), pages 641 - 649, XP055822531, ISSN: 0027-8424, DOI: 10.1073/pnas.1815031116
- MARKUS NAPIREI, SEBASTIAN LUDWIG, JAMAL MEZRHAB, THOMAS KLÖCKL, HANS G. MANNHERZ: "Murine serum nucleases – contrasting effects of plasmin and heparin on the activities of DNase1 and DNase1-like 3 (DNase1l3)", FEBS JOURNAL, vol. 276, 23 January 2009 (2009-01-23), pages 1059 - 1073, XP055822527, ISSN: 1742-464X, DOI: 10.1111/j.1742-4658.2008.06849.x
- WATANABE TAIKI; TAKADA SHUHEI; MIZUTA RYUSHIN: "Cell-free DNA in blood circulation is generated by DNase1L3 and caspase-activated DNase", BIOCHEMICAL AND BIOPHYSICAL RESEARCH COMMUNICATIONS, vol. 516, no. 3, 27 June 2019 (2019-06-27), pages 790 - 795, XP085743316, ISSN: 0006-291X, DOI: 10.1016/j.bbrc.2019.06.069

## Description

### CROSS-REFERENCES TO RELATED APPLICATIONS

### BACKGROUND

Cell-free DNA (cfDNA) is a rich source of information that can be applied to the diagnosis and prognostication of many physiological and pathological conditions such as pregnancy and cancer (Chan, K.C.A. et al. (2017), New England Journal of Medicine 377, 513-522; Chiu, R.W.K. et al. (2008), Proceedings of the National Academy of Sciences of the United States of America 105, 20458-20463; Lo, Y.M.D. et al., (1997), The Lancet 350, 485-487). Though circulating cfDNA is now commonly used as a non-invasive biomarker and is known to circulate in the form of short fragments, the physiological factors governing the fragmentation and molecular profile of cfDNA remain elusive.

Recent works have suggested that the fragmentation of cfDNA is a non-random process associated with the positioning of nucleosomes (Chandrananda, D. et al., (2015), BMC Medical Genomics 8, 29; Ivanov, M. et al., (2015), BMC genomics 16, S1; Lo, Y.M.D. et al. (2010), Science Translational Medicine 2, 61ra91-61ra91; Snyder, M.W. et al., (2016), Cell 164, 57-68; Sun, K. et al., (2019), Genome Research 29, 418-427)). Previously, we have demonstrated that the DNASE1L3 nuclease contributes to the size profile of cfDNA in plasma (Serpas, L. et al. (2019), Proceedings of the National Academy of Sciences 116, 641-649).

### BRIEF SUMMARY

Various embodiments use quantitative fragmentation information of cell-free DNA (cfDNA) for detecting a genetic disorder in a gene associated with a nuclease, for determining an efficacy of a dosage of an anticoagulant, and for monitoring an activity of a nuclease. Measured parameter values can be compared to a reference value to determine classifications of a genetic disorder, efficiency, or activity. An amount of a particular base (e.g., in an end motif) at fragment ends, an amount of a particular base at fragment ends of a particular size, or a total amount of cell-free DNA fragments (e.g., as a concentration) can be used. Certain samples may be treated with an anticoagulant, and different incubation times can be used in some embodiments,

Some embodiments are provided for detecting a genetic disorder for a gene, e.g., using an amount of a particular base at fragment ends relative to a reference value, using an amount of a particular base at fragment ends of a particular size in a sample treated with an anticoagulant, and comparing amounts of a particular base at fragment ends for samples incubated with an anticoagulant over different times.

Some embodiments are provided for determining an efficacy of a dosage of an anticoagulant, e.g., using an amount of a particular base at fragment ends in a sample of a subject administered an anticoagulant and using an amount of a particular base at fragment ends of a particular size in a sample of a subject administered an anticoagulant.

Some embodiments are provided for monitoring an activity of a nuclease, e.g., using an amount of a particular base at fragment ends in a sample relative to a reference value and using an amount of a particular base at fragment ends of a particular size in a sample.

These embodiments and other embodiments of the disclosure are described in detail below. For example, other embodiments are directed to systems, devices, and computer readable media associated with methods described herein.

A better understanding of the nature and advantages of embodiments of the present disclosure may be gained with reference to the following detailed description and the accompanying drawings.

### BRIEF DESCRIPTION OF THE DRAWINGS

FIG. 1 shows examples for end motifs, including a single base at an end of a DNA fragment, according to embodiments of the present disclosure.
FIGS. 2A-2E show base content of the 5' end of WT cfDNA fragments compared with the reference genomic content in different regions according to embodiments of the present disclosure.
FIGS. 3A-3D show base content proportions in TSS and Pol II regions according to embodiments of the present disclosure. The reference genomic content in TSS (3A) and Pol II (3C) regions compared to the 5' end base content of cfDNA in WT EDTA 0 h samples (3B & 3D).
FIG. 4 shows base content of the 5' end of WT cfDNA across the range of fragment sizes according to embodiments of the present disclosure.
FIGS. 5A-5B show collection of EDTA 6 h samples enriched with fresh cfDNA according to embodiments of the present disclosure.
FIG. 6 shows size profiles for EDTA 0 h vs 6 h samples in WT mice according to embodiments of the present disclosure.
FIGS. 7A-7D show base content percentages of EDTA 6 h samples enriched with fresh cfDNA in mice for random, CTCF, TSS, and Pol II regions according to embodiments of the present disclosure.
FIG. 8A shows A<>A fragment proportions compared between baseline cfDNA (EDTA 0 h) and samples enriched with fresh cfDNA (EDTA 6 h) in WT mice among short, intermediate, and long fragments according to embodiments of the present disclosure. FIG. 8B) shows size profiles for G<>G, and FIGS. 9A-9B show size profiles for C<>C, T<>T fragment proportions in WT mice compared between EDTA 0 h and EDTA 6 h among short, intermediate and long fragments. P-value calculated by Mann-Whitney *U* test.
FIGS. 10A-10B show base content percentages of EDTA 0 h vs. EDTA 6 h samples enriched with fresh cfDNA in WT and *Dffb*-deficient mice according to embodiments of the present disclosure.
FIG. 11A shows a concentration of cfDNA in EDTA 0 h vs 6 h samples in *Dffb-*deficient mice according to embodiments of the present disclosure. FIG. 11B shows size profiles in EDTA 0 h vs 6 h samples in *Dffb*-deficient mice according to embodiments of the present disclosure. FIG. 11C shows A<>A fragment proportions in *Dffb*-deficient mice compared between EDTA 0 h and EDTA 6 h among short, intermediate and long fragments according to embodiments of the present disclosure.
FIGS. 12A-12D show base content proportions in *Dffb*-deficient mice in EDTA 0 h and 6 h samples for random regions and CTCF regions according to embodiments of the present disclosure.
FIGS. 13A-13D shows base content proportions in *Dffb*-deficient mice in EDTA 0 h and 6 h samples for TSS regions and Pol II regions according to embodiments of the present disclosure.
FIGS. 14A shows the construction of an A<>A fragment according to embodiments of the present disclosure. FIG. 14B shows end base contents of *Dnase1l3*-deficient samples compared to WT samples according to embodiments of the present disclosure.
FIG. 15 shows end base contents of *Dnase1l3*-deficient samples compared to WT samples per fragment size according to embodiments of the present disclosure.
FIG. 16A shows percentages of A<>A, A<>G, and A<>C fragments in *Dnase1l3-*deficient EDTA 0 h cfDNA compared with the baseline representation of WT EDTA 0 h cfDNA (gray) according to embodiments of the present disclosure. FIG. 16B shows percentages of A<>A, A<>G, and A<>C fragments in WT EDTA 6 h samples enriched with fresh cfDNA compared to the baseline representation of WT EDTA 0 h cfDNA (gray) according to embodiments of the present disclosure.
FIGS. 17A-17B show size profiles of cfDNA of WT, *Dnase1*^{*+*/*-*}, and *Dnase1*^{*-*/*-*} mice with incubation in heparin in regular and logarithmic scales according to embodiments of the present disclosure.
FIGS. 18A-18B show size profiles and base content of cfDNA of WT and *Dnase1*^{-/-}mice with incubation in heparin according to embodiments of the present disclosure.
FIG. 19 shows size profiles and base content of cfDNA of *Dnase1*^{+/-} mice with incubation in heparin according to embodiments of the present disclosure
FIG. 20 shows cfDNA quantity for WT, *Dnase1*^{*+*/*-*}, and *Dnase1*^{*-*/*-*} mice with in 0 h and 6 h samples in heparin according to embodiments of the present disclosure.
FIG. 21A shows a cfDNA size profile of A-end, G-end, C-end, and T-end fragments in an EDTA 0 h WT sample according to embodiments of the present disclosure. FIG. 21B shows a cfDNA size profile of A-end, G-end, C-end, and T-end fragments in a Heparin 6 h WT sample according to embodiments of the present disclosure.
FIGS. 22A-22D show cfDNA size profiles of A-end, G-end, C-end, and T-end fragments in EDTA 0 h sample of *Dffb*^{*-*/}*⁻, Dnase1l3*^{*-*/}*⁻, Dnase1*^{*+*/*-*}, and *Dnase1*^{*-*/*-*} mice according to embodiments of the present disclosure.
FIG. 23A shows fragment end density in the CTCF region in the Heparin 6 h sample (red) compared to the baseline samples (EDTA 0 h and 6 h, Heparin 0 h) (gray) according to embodiments of the present disclosure. FIGS. 23B-23C show 5' end base representation in the CTCF region of Heparin 0 h and 6 h samples of WT (D) according to embodiments of the present disclosure.
FIGS. 24A-24B show 5' end base representation in the CTCF region of Heparin 0 h and 6 h samples of *Dnase1*^{*-*/*-*} mice according to embodiments of the present disclosure.
FIG. 25 shows FIGS. 23A and 23C overlaid to show the T-end fragment peaks correspond to the intranucleosomal areas with increased end density in Heparin 6 h according to embodiments of the present disclosure.
FIG. 26 shows a model of cfDNA generation and digestion with cutting preferences shown for nucleases DFFB, DNASE1, and DNASE1L3 according to embodiments of the present disclosure.
FIG. 27 shows a flowchart illustrating a method for detecting a genetic disorder for a gene associated with a nuclease using biological samples including cell-free DNA according to embodiments of the present disclosure.
FIG. 28 shows a flowchart illustrating a method for detecting a genetic disorder for a gene associated with a nuclease using biological samples including cell-free DNA according to embodiments of the present disclosure.
FIG. 29 shows a flowchart illustrating a method for detecting a genetic disorder for a gene associated with a nuclease using biological samples including cell-free DNA according to embodiments of the present disclosure.
FIG. 30 shows a flowchart illustrating a method for determining an efficacy of a treatment of a subject having blood disorder according to embodiments of the present disclosure.
FIG. 31 shows a flowchart illustrating a method 1300 for determining an efficacy of a treatment of a subject having blood disorder according to embodiments of the present disclosure.
FIG. 32A shows data for four cases treated with heparin according to embodiments of the present disclosure. FIGS. 32B-32C show data for two samples of a patient with deep vein thrombosis (DVT) who has been treated with heparin according to embodiments of the present disclosure.
FIG. 33 shows plots of content percentage for the different ends vs. size of the fragment for different dosages of DNASE1 according to embodiments of the present disclosure. FIG. 33 also shows a frequency plot for the size of all fragments according to embodiments of the present disclosure.
FIG. 34A shows a size profile for serum that is treated with DNASE1 compared to untreated and to EDTA treated (at 0 and 6 hours) according to embodiments of the present disclosure. FIG. 34B shows a size profile in plasma.
FIG. 35 shows the effect of different doses of DNASE1 on serum after 6 hours according to embodiments of the present disclosure.
FIG. 36 shows the frequency vs. size and base content vs size in a urine sample according to embodiments of the present disclosure.
FIG. 37 shows the DNASE1 expression for different tissues.
FIG. 38 is a flowchart illustrating a method for monitoring activity of a nuclease using biological samples including cell-free DNA according to embodiments of the present disclosure.
FIG. 39 is a flowchart illustrating a method for monitoring activity of a nuclease using biological samples including cell-free DNA according to embodiments of the present disclosure.
FIG. 40 summarizes the number of non-duplicate fragments obtained for each condition according to embodiments of the present disclosure.
FIG. 41A shows a deletion in the Dnase1 gene for both copies (Dnase1^{-/-}). FIG. 41B shows the deletions for the Dffb gene in both copies.
FIG. 42 illustrates a measurement system according to an embodiment of the present invention.
FIG. 43 shows a block diagram of an example computer system usable with systems and methods according to embodiments of the present invention.

### TERMS

*A "tissue"* corresponds to a group of cells that group together as a functional unit. More than one type of cells can be found in a single tissue. Different types of tissue may consist of different types of cells (e.g., hepatocytes, alveolar cells or blood cells), but also may correspond to tissue from different organisms (mother vs. fetus) or to healthy cells vs. tumor cells. "Reference tissues" can correspond to tissues used to determine tissue-specific methylation levels. Multiple samples of a same tissue type from different individuals may be used to determine a tissue-specific methylation level for that tissue type.

*A "biological sample"* refers to any sample that is taken from a subject (*e.g*., a human (or other animal), such as a pregnant woman, a person with cancer or other disorder, or a person suspected of having cancer or other disorder, an organ transplant recipient or a subject suspected of having a disease process involving an organ (e.g., the heart in myocardial infarction, or the brain in stroke, or the hematopoietic system in anemia) and contains one or more nucleic acid molecule(s) of interest. The biological sample can be a bodily fluid, such as blood, plasma, serum, urine, vaginal fluid, fluid from a hydrocele (e.g. of the testis), vaginal flushing fluids, pleural fluid, ascitic fluid, cerebrospinal fluid, saliva, sweat, tears, sputum, bronchoalveolar lavage fluid, discharge fluid from the nipple, aspiration fluid from different parts of the body (e.g. thyroid, breast), intraocular fluids (e.g. the aqueous humor), etc. Stool samples can also be used. In various embodiments, the majority of DNA in a biological sample that has been enriched for cell-free DNA (e.g., a plasma sample obtained via a centrifugation protocol) can be cell-free, e.g., greater than 50%, 60%, 70%, 80%, 90%, 95%, or 99% of the DNA can be cell-free. The centrifugation protocol can include, for example, 3,000 g x 10 minutes, obtaining the fluid part, and re-centrifuging at for example, 30,000 g for another 10 minutes to remove residual cells. As part of an analysis of a biological sample, a statistically significant number of cell-free DNA molecules can be analyzed (e.g., to provide an accurate measurement) for a biological sample. In some embodiments, at least 1,000 cell-free DNA molecules are analyzed. In other embodiments, at least 10,000 or 50,000 or 100,000 or 500,000 or 1,000,000 or 5,000,000 cell-free DNA molecules, or more, can be analyzed. At least a same number of sequence reads can be analyzed.

*A "sequence read"* refers to a string of nucleotides sequenced from any part or all of a nucleic acid molecule. For example, a sequence read may be a short string of nucleotides (e.g., 20-150 nucleotides) sequenced from a nucleic acid fragment, a short string of nucleotides at one or both ends of a nucleic acid fragment, or the sequencing of the entire nucleic acid fragment that exists in the biological sample. A sequence read may be obtained in a variety of ways, e.g., using sequencing techniques or using probes, e.g., in hybridization arrays or capture probes as may be used in microarrays, or amplification techniques, such as the polymerase chain reaction (PCR) or linear amplification using a single primer or isothermal amplification. As part of an analysis of a biological sample, at least 1,000 sequence reads can be analyzed. As other examples, at least 10,000 or 50,000 or 100,000 or 500,000 or 1,000,000 or 5,000,000 sequence reads, or more, can be analyzed.

A sequence read can include an *"ending sequence"* associated with an end of a fragment. The ending sequence can correspond to the outermost N bases of the fragment, e.g., 1-30 bases at the end of the fragment. If a sequence read corresponds to an entire fragment, then the sequence read can include two ending sequences. When paired-end sequencing provides two sequence reads that correspond to the ends of the fragments, each sequence read can include one ending sequence.

*A "sequence motif'* may refer to a short, recurring pattern of bases in DNA fragments (e.g., cell-free DNA fragments). A sequence motif can occur at an end of a fragment, and thus be part of or include an ending sequence. An *"end motif'* can refer to a sequence motif for an ending sequence that preferentially occurs at ends of DNA fragments, potentially for a particular type of tissue. An end motif may also occur just before or just after ends of a fragment, thereby still corresponding to an ending sequence. A nuclease can have a specific cutting preference for a particular end motif, as well as a second most preferred cutting preference for a second end motif.

The term *"alleles"* refers to alternative DNA sequences at the same physical genomic locus, which may or may not result in different phenotypic traits. In any particular diploid organism, with two copies of each chromosome (except the sex chromosomes in a male human subject), the genotype for each gene comprises the pair of alleles present at that locus, which are the same in homozygotes and different in heterozygotes. A population or species of organisms typically include multiple alleles at each locus among various individuals. A genomic locus where more than one allele is found in the population is termed a polymorphic site. Allelic variation at a locus is measurable as the number of alleles (i.e., the degree of polymorphism) present, or the proportion of heterozygotes (i.e., the heterozygosity rate) in the population. As used herein, the term *"polymorphism"* refers to any inter-individual variation in the human genome, regardless of its frequency. Examples of such variations include, but are not limited to, single nucleotide polymorphism, simple tandem repeat polymorphisms, insertion-deletion polymorphisms, mutations (which may be disease causing) and copy number variations. The term *"haplotype"* as used herein refers to a combination of alleles at multiple loci that are transmitted together on the same chromosome or chromosomal region. A haplotype may refer to as few as one pair of loci or to a chromosomal region, or to an entire chromosome or chromosome arm.

*A "relative frequency"* (also referred to just as "frequency") may refer to a proportion (e.g., a percentage, fraction, or concentration). In particular, a relative frequency of a particular end motif (e.g., CCGA or just a single base) can provide a proportion of cell-free DNA fragments in a sample that are associated with the end motif CCGA, e.g., by having an ending sequence of CCGA.

An *"aggregate value"* may refer to a collective property, e.g., of relative frequencies of a set of end motifs. Examples include a mean, a median, a sum of relative frequencies, a variation among the relative frequencies (e.g., entropy, standard deviation (SD), the coefficient of variation (CV), interquartile range (IQR) or a certain percentile cutoff (e.g. 95^{th} or 99^{th} percentile) among different relative frequencies), or a difference (e.g., a distance) from a reference pattern of relative frequencies, as may be implemented in clustering.

A *"calibration sample"* can correspond to a biological sample whose desired measured value (e.g., nuclease activity, classification of a genetic disorder, or other desired property) is known or determined via a calibration method, e.g., using other measurement techniques such as clotting measurements for effective dosage or ELISA for measuring nuclease quantity or assays quantifying the rate of DNA digestion by nucleases for measuring nuclease activity. An example measurement can involve fluorometric or spectrophotometric measurement of cfDNA quantity, which may be done on its own or before, after, and/or in real-time with, the addition of a nuclease-containing sample. Another example is using radial enzyme diffusion methods. A calibration sample can have separate measured values (e.g., an amount of fragments with a particular end motif or with a particular size) can be determined to which the desired measure value can be correlated.

A *"calibration data point"* includes a *"calibration value"* (e.g., an amount of fragments with a particular end motif or with a particular size) and a measured or known value that is desired to be determined for other test samples. The calibration value can be determined from various types of data measured from DNA molecules of the sample, (e.g., an amount of fragments with an end motif or with a particular size). The calibration value corresponds to a parameter that correlates to the desired property, e.g., classification of a genetic disorder, nuclease activity, or efficacy of anticoagulant dosage. For example, a calibration value can be determined from measured values as determined for a calibration sample, for which the desired property is known. The calibration data points may be defined in a variety of ways, e.g., as discrete points or as a calibration function (also called a calibration curve or calibration surface). The calibration function could be derived from additional mathematical transformation of the calibration data points.

*A "site"* (also called a *"genomic site*") corresponds to a single site, which may be a single base position or a group of correlated base positions, e.g., a CpG site, TSS site, Dnase hypersensitivity site, or larger group of correlated base positions. A "locus" may correspond to a region that includes multiple sites. A locus can include just one site, which would make the locus equivalent to a site in that context.

A *"cfDNA profile"* may refer to the relationship of ending sequences (e.g., 1- 30 bases) of cfDNA fragments (also just referred to as DNA fragments) in a sample. Various relationships can be provided, e.g., an amount of cfDNA fragments with a particular ending sequence (end motif), a relative frequency of cfDNA fragments with a particular ending sequence compared to one or more other ending sequences, as well as include other parameters, such as size. A cfDNA profile can be provided for various sizes of cfDNA fragments. Such a cfDNA profile (sometimes referred to as a cfDNA size profile) can be provided in various ways that illustrate an amount of cfDNA fragments having one or more particular ending sequences for a given size (single length or size range).

*A "separation value"* corresponds to a difference or a ratio involving two values, e.g., two fractional contributions or two methylation levels. The separation value could be a simple difference or ratio. As examples, a direct ratio of x/y is a separation value, as well as x/(x+y). The separation value can include other factors, e.g., multiplicative factors. As other examples, a difference or ratio of functions of the values can be used, e.g., a difference or ratio of the natural logarithms (ln) of the two values. A separation value can include a difference and a ratio.

A *"separation value"* and an *"aggregate value"* (e.g., of relative frequencies) are two examples of a parameter (also called a metric) that provides a measure of a sample that varies between different classifications (states), and thus can be used to determine different classifications. An aggregate value can be a separation value, e.g., when a difference is taken between a set of relative frequencies of a sample and a reference set of relative frequencies, as may be done in clustering.

The term *"classification"* as used herein refers to any number(s) or other characters(s) that are associated with a particular property of a sample. For example, a "+" symbol (or the word "positive") could signify that a sample is classified as having deletions or amplifications. The classification can be binary (e.g., positive or negative) or have more levels of classification (e.g., a scale from 1 to 10 or 0 to 1).

The terms "*cutoff*" and *"threshold"* refer to predetermined numbers used in an operation. For example, a cutoff size can refer to a size above which fragments are excluded. A threshold value may be a value above or below which a particular classification applies. Either of these terms can be used in either of these contexts. A cutoff or threshold may be "a reference value" or derived from a reference value that is representative of a particular classification or discriminates between two or more classifications. Such a reference value can be determined in various ways, as will be appreciated by the skilled person. For example, metrics can be determined for two different cohorts of subjects with different known classifications, and a reference value can be selected as representative of one classification (e.g., a mean) or a value that is between two clusters of the metrics (e.g., chosen to obtain a desired sensitivity and specificity). As another example, a reference value can be determined based on statistical simulations of samples. A particular value for a cutoff, threshold, reference, etc. can be determined based on a desired accuracy (e.g., a sensitivity and specificity).

*A "level of pathology"* (or level of a disorder) can refer to the amount, degree, or severity of pathology associated with an organism. An example is a cellular disorder in expressing a nuclease. Another example of pathology is a rejection of a transplanted organ. Other example pathologies can include autoimmune attack (e.g., lupus nephritis damaging the kidney or multiple sclerosis), inflammatory diseases (e.g., hepatitis), fibrotic processes (e.g. cirrhosis), fatty infiltration (e.g. fatty liver diseases), degenerative processes (e.g. Alzheimer's disease) and ischemic tissue damage (e.g., myocardial infarction or stroke). A heathy state of a subject can be considered a classification of no pathology. The pathology can be cancer.

The term *"level of cancer"* can refer to whether cancer exists (i.e., presence or absence), a stage of a cancer, a size of tumor, whether there is metastasis, the total tumor burden of the body, the cancer's response to treatment, and/or other measure of a severity of a cancer (e.g. recurrence of cancer). The level of cancer may be a number or other indicia, such as symbols, alphabet letters, and colors. The level may be zero. The level of cancer may also include premalignant or precancerous conditions (states). The level of cancer can be used in various ways. For example, screening can check if cancer is present in someone who is not previously known to have cancer. Assessment can investigate someone who has been diagnosed with cancer to monitor the progress of cancer over time, study the effectiveness of therapies or to determine the prognosis. In one embodiment, the prognosis can be expressed as the chance of a patient dying of cancer, or the chance of the cancer progressing after a specific duration or time, or the chance or extent of cancer metastasizing. Detection can mean 'screening' or can mean checking if someone, with suggestive features of cancer (e.g. symptoms or other positive tests), has cancer.

The term "about" or "approximately" can mean within an acceptable error range for the particular value as determined by one of ordinary skill in the art, which will depend in part on how the value is measured or determined, i.e., the limitations of the measurement system. For example, "about" can mean within 1 or more than 1 standard deviation, per the practice in the art. Alternatively, "about" can mean a range of up to 20%, up to 10%, up to 5%, or up to 1% of a given value. Alternatively, particularly with respect to biological systems or processes, the term "about" or "approximately" can mean within an order of magnitude, within 5-fold, and more preferably within 2-fold, of a value. Where particular values are described in the application and claims, unless otherwise stated the term "about" meaning within an acceptable error range for the particular value should be assumed. The term "about" can have the meaning as commonly understood by one of ordinary skill in the art. The term "about" can refer to ±10%. The term "about" can refer to ±5%.

### DETAILED DESCRIPTION

Cell-free DNA (cfDNA) is a powerful non-invasive biomarker for cancer and prenatal testing and circulates in plasma (as well as other cell-free samples) as short fragments. In this disclosure, we investigated the respective roles of DNASE1, DNASE1L3, and DNA fragmentation factor subunit beta (DFFB, also known as Caspase-Activated DNase) in cfDNA fragmentation. To elucidate the biology of cfDNA fragmentation, we analyzed the roles of DNASE1, DNASE1L3, and DNA fragmentation factor subunit beta (DFFB) with mice deficient in each of these nucleases.

In an example analysis, we compared the cfDNA profiles (including cfDNA size profiles) between mice deficient in each type of nuclease and their wildtype counterparts, including the ending base of cfDNA fragments. The ending base of a DNA fragment is a type of end motif, and measurements of relative amounts (e.g., proportions) of cfDNA fragments ending with a particular base can provide information about cfDNA fragments, the source of cfDNA fragments related to the tissue nuclease activity, nucleases function, and disorders affecting nucleases. We found that each nuclease served a different but complementary role in cfDNA fragmentation.

By analyzing the ends of cfDNA fragments in each type of nuclease-deficient mice with those in wildtype mice, we show that each nuclease has a specific cutting preference (e.g., a particular end motif) that reveals the stepwise process of cfDNA fragmentation. We demonstrate that cfDNA is generated first intracellularly with DFFB, intracellularly with DNASE1L3, and other nucleases. Then, cfDNA fragmentation continues extracellularly with circulating DNASE1L3 and DNASE1. With the use of heparin to disrupt the nucleosomal structure, we also showed that the 10 bp periodicity originated from the cutting of DNA within an intact nucleosomal structure. Altogether, this disclosure establishes a model of cfDNA fragmentation.

Various embodiments are provided for detecting a genetic disorder in a gene associated with a nuclease, for determining an efficacy of a dosage of an anticoagulant, and for monitoring an activity of a nuclease.

Various techniques are provided for detecting a genetic disorder for a gene, e.g., using an amount of a particular base at fragment ends relative to a reference value, using an amount of a particular base at fragment ends of a particular size in a sample treated with an anticoagulant, and comparing amounts of a particular base at fragment ends for samples incubated with an anticoagulant over different times.

Various techniques are provided for determining an efficacy of a dosage of an anticoagulant, e.g., using an amount of a particular base at fragment ends in a sample of a subject administered an anticoagulant and using an amount of a particular base at fragment ends of a particular size in a sample of a subject administered an anticoagulant.

Various techniques are provided for monitoring an activity of a nuclease, e.g., using an amount of a particular base at fragment ends in a sample relative to a reference value and using an amount of a particular base at fragment ends of a particular size in a sample.

### I. CELL-FREE DNA END MOTIFS

An end motif relates to the ending sequence of a cell-free DNA fragment, e.g., the sequence for the K bases at either end of the fragment. The ending sequence can be a k-mer having various numbers of bases, e.g., 1, 2, 3, 4, 5, 6, 7, etc. The end motif (or "sequence motif") relates to the sequence itself as opposed to a particular position in a reference genome. Thus, a same end motif may occur at numerous positions throughout a reference genome. The end motif may be determined using a reference genome, e.g., to identify bases just before a start position or just after an end position. Such bases will still correspond to ends of cell-free DNA fragments, e.g., as they are identified based on the ending sequences of the fragments.

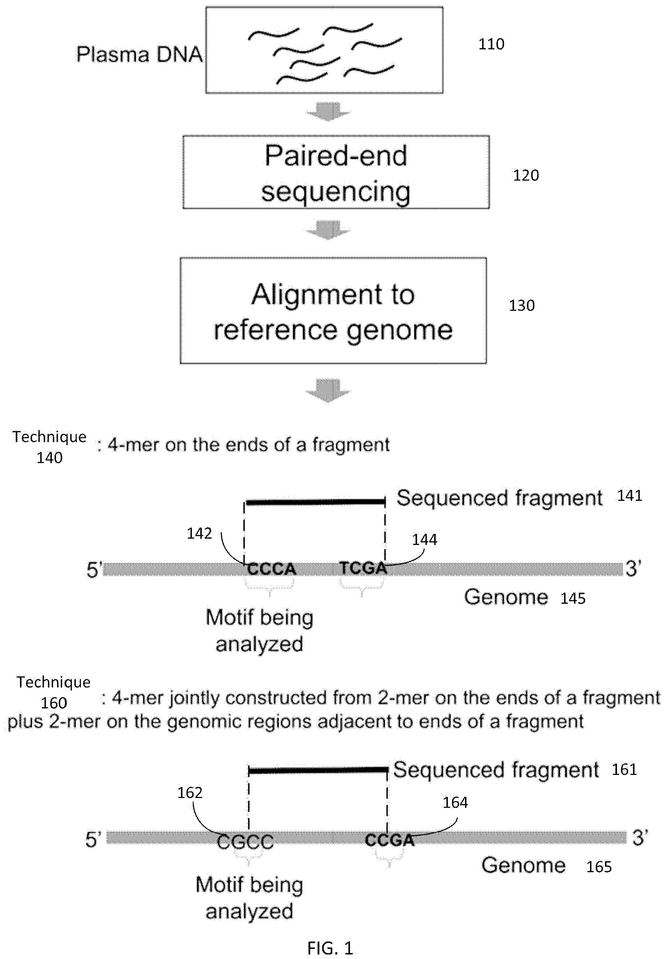

FIG. 1 shows examples for end motifs according to embodiments of the present disclosure. FIG. 1 depicts two ways to define 4-mer end motifs to be analyzed. In technique 140, the 4-mer end motifs are directly constructed from the first 4-bp sequence on each end of a plasma DNA molecule. For example, the first 4 nucleotides or the last 4 nucleotides of a sequenced fragment could be used. In technique 160, the 4-mer end motifs are jointly constructed by making use of the 2-mer sequence from the sequenced ends of fragments and the other 2-mer sequence from the genomic regions adjacent to the ends of that fragment. In other embodiments, other types of motifs can be used, e.g., 1-mer, 2-mer, 3-mer, 5-mer, 6-mer, 7-mer end motifs.

As shown in FIG. 1, cell-free DNA fragments 110 are obtained, e.g., using a purification process on a blood sample, such as by centrifuging. Besides plasma DNA fragments, other types of cell-free DNA molecules can be used, e.g., from serum, urine, saliva, and other samples mentioned herein. In one embodiment, the DNA fragments may be blunt-ended.

At block 120, the DNA fragments are subjected to paired-end sequencing. In some embodiments, the paired-end sequencing can produce two sequence reads from the two ends of a DNA fragment, e.g., 30-120 bases per sequence read. These two sequence reads can form a pair of reads for the DNA fragment (molecule), where each sequence read includes an ending sequence of a respective end of the DNA fragment. In other embodiments, the entire DNA fragment can be sequenced, thereby providing a single sequence read, which includes the ending sequences of both ends of the DNA fragment. The two ending sequences at both ends can still be considered paired sequence reads, even if generated together from a single sequencing operation.

At block 130, the sequence reads can be aligned to a reference genome. This alignment is to illustrate different ways to define a sequence motif, and may not be used in some embodiments. For example, the sequences at the end of a fragment can be used directly without needing to align to a reference genome. However, alignment can be desired to have uniformity of an ending sequence, which does not depend on variations (e.g., SNPs) in the subject. For instance, the ending base could be different from the reference genome due to a variation or a sequencing error, but the base of in the reference may be the one counted. Alternatively, the base on the end of the sequence read can be used, so as to be tailored to the individual. The alignment procedure can be performed using various software packages, such as (but not limited to) BLAST, FASTA, Bowtie, BWA, BFAST, SHRiMP, SSAHA2, NovoAlign, and SOAP.

Technique 140 shows a sequence read of a sequenced fragment 141, with an alignment to a genome 145. With the 5' end viewed as the start, a first end motif 142 (CCCA) is at the start of sequenced fragment 141. A second end motif 144 (TCGA) is at the tail of the sequenced fragment 141. When analyzing the end predominance of cfDNA fragments, this sequence read would contribute to a C-end count for the 5' end. Such end motifs might, in one embodiment, occur when an enzyme recognizes CCCA and then makes a cut just before the first C. If that is the case, CCCA will preferentially be at the end of the plasma DNA fragment. For TCGA, an enzyme might recognize it, and then make a cut after the A. When a count is determined for the A, this sequence read would contribute to an A-end count.

Technique 160 shows a sequence read of a sequenced fragment 161, with an alignment to a genome 165. With the 5' end viewed as the start, a first end motif 162 (CGCC) has a first portion (CG) that occurs just before the start of sequenced fragment 161 and a second portion (CC) that is part of the ending sequence for the start of sequenced fragment 161. A second end motif 164 (CCGA) has a first portion (GA) that occurs just after the tail of sequenced fragment 161 and a second portion (CC) that is part of the ending sequence for the tail of sequenced fragment 161. Such end motifs might, in one embodiment, occur when an enzyme recognizes CGCC and then makes a cut just before the G and the C. If that is the case, CC will preferentially be at the end of the plasma DNA fragment with CG occurring just before it, thereby providing an end motif of CGCC. As for the second end motif 164 (CCGA), an enzyme can cut between C and G. If that is the case, CC will preferentially be at the end of the plasma DNA fragment. For technique 160, the number of bases from the adjacent genome regions and sequenced plasma DNA fragments can be varied and are not necessarily restricted to a fixed ratio, e.g., instead of 2:2, the ratio can be 2:3, 3:2, 4:4, 2:4, etc.

The higher the number of nucleotides included in the cell-free DNA end signature, the higher the specificity of the motif because the probability of having 6 bases ordered in an exact configuration in the genome is lower than the probability of having 2 bases ordered in an exact configuration in the genome. Thus, the choice of the length of the end motif can be governed by the needed sensitivity and/or specificity of the intended use application.

As the ending sequence is used to align the sequence read to the reference genome, any sequence motif determined from the ending sequence or just before/after is still determined from the ending sequence. Thus, technique 160 makes an association of an ending sequence to other bases, where the reference is used as a mechanism to make that association. A difference between techniques 140 and 160 would be to which two end motifs a particular DNA fragment is assigned, which affects the particular values for the relative frequencies. But, the overall result (e.g., detecting a genetic disorder, determining efficacy of a dosage, monitoring activity of a nuclease, etc.) would not be affected by how the a DNA fragment is assigned to an end motif, as long as a consistent technique is used, e.g., for any training data to determine a reference value, as may occur using a machine learning model.

The counted numbers of DNA fragments having an ending sequence corresponding to a particular end motif (e.g., a particular base) may be counted (e.g., stored in an array in memory) to determine an amount of the particular end motif. The amount can be measured in various ways, such as a raw count or a frequency, where the amount is normalized. The normalization may be done using (e.g., dividing by) a total number of DNA fragments or a number in a specified group of DNA fragments (e.g., from a specified region, having a specified size, or having one or more specified end motifs). Differences in amounts of end motifs have been detected when a genetic disorder exists, as well as when an effective dose of an anticoagulant has been administered, as well as when the activity of a nuclease changes (e.g., increases or decreased).

### II. ENDING PREFERENCES IN CIRCULATING AND FRESH CFDNA

Circulating cfDNA can be found directly from a sample obtained from a subject, e.g., blood or plasma. Such circulating cfDNA exists in cell-free form in the body. Thus, the cell-free DNA was produced (e.g., via apoptosis or necrosis) from cells within the body, and then the cell-free DNA began to circulate (e.g., in blood). In contrast, fresh cfDNA is obtained from cells from the body, and then the cell-free DNA is generated while the cell is outside the body, e.g., by having the cell die in any of various ways, such as incubation. Differences in preferred ending sequence(s) were observed.

### A. C-end preference in typical circulating cfDNA

We analyzed the base content proportions at the 5' end of cfDNA fragments in different genomic regions in wildtype (WT) mice to test the hypothesis that cfDNA fragmentation is not random. For blood samples, EDTA can be used as an anticoagulant and inhibit plasma nucleases to preserve the size profile, frequencies of end motifs, and the concentration of cell-free DNA relatively close to an initial state when kept at cool temperatures, e.g., standard refrigerator temperatures, such as between -5° C to 20° C. If incubated at a higher temperature (e.g., room temperature), fresh cfDNA will be generated at an amount dependent on the amount of incubation time. A time of 0 indicates that no incubation at room temperature.

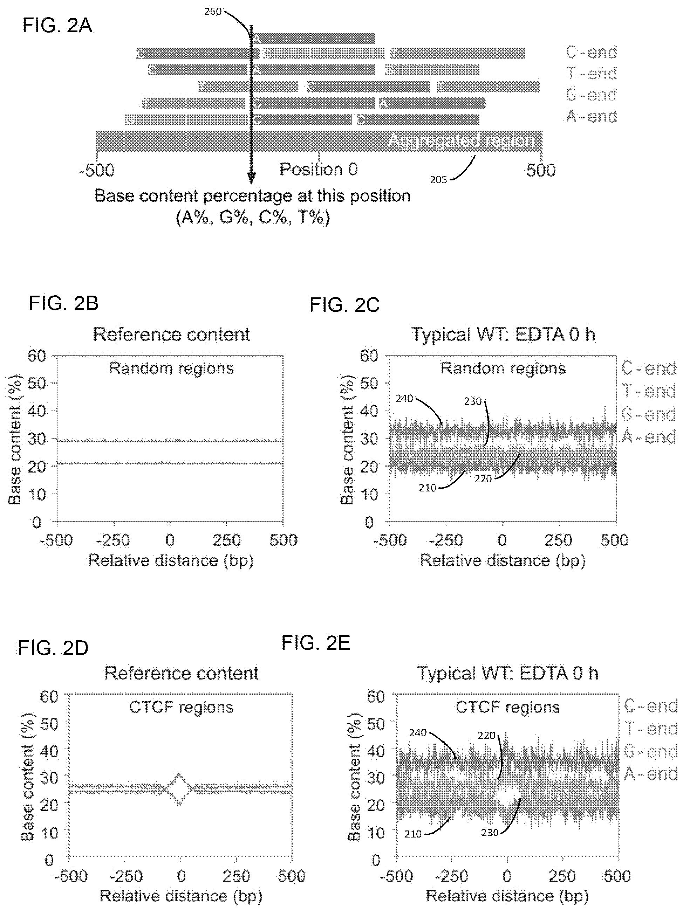

FIGS. 2A-2E show base content of the 5' end of WT cfDNA fragments compared with the reference genomic content in different regions according to embodiments of the present disclosure. These figures show a preference for fragmenting at C/G relative to T/A using based content percentage of end motif (single base in this example) relative to general base content of reference genome.

### 1. Defining base content percentage for end motif of fragments

FIG. 2A shows an aggregated region 205 to which fragments are aligned, where the fragments are labeled based on the ending base at the 5' end. The horizontal axis shows a relative position to a center of the region. Example types of such regions include open chromatin regions; CTCF regions; regions associated with hypersensitive sites, e.g., for a particular nuclease (e.g., a DNase); Pol II regions (RNA polymerase II); and regions associated with transcription start sites (TSS). Since there are many instances of each the type of region in a reference genome, the aligned count data (e.g., counts of end motif for each position in a given instance of region) is aggregated across the many instances of the region type. A position 0 is selected for each instance, so that the counts may be aggregated for a given position for each end motif, a particular base in this example.

A vertical line 260 illustrates how a percentage is determined for each position. The percentage is of reads labeled with a particular base, which as mentioned above, corresponds to the ending base at the 5' end. Thus, the calculation of the percentage at a given position uses all of the fragments that end at that position. In FIG. 2A, the base content is 50% A and 50% C at the position corresponding to vertical line 260. If the end motif was more than a 1-mer, then the determination of the percentage can account for the number possible end motifs being more than two, e.g., 16 for the end motif being a 2-mer.

### 2. End base content relative to general base content of reference

FIG. 2B shows a plot of base content percentage at positions in random regions for reference genomic content (i.e., of a reference genome). The random regions were generated by randomly selecting a position 0, which defines a region of 1000 bases, and then determining the base content in the reference genome relative to that position. Thus, FIG. 2B is not determined using the ending base of a DNA fragment, but instead the base content of the reference genome relative to the randomly selected position is used. FIG. 2B shows no variation in the percentage for an ending base for the relative distance to position 0. The percentages for the different bases do have a difference, as a result of differences of occurrence of a base in the reference genome, but the percentage for a given base is constant. For the particular data in FIG. 2B, about 10,000 random positions were selected, where the bases around those positions were analyzed. These positions are shown at position 0. In random regions of the reference genomic content, A and T proportions are equal, and C and G proportions are equal. For random regions, the base content percentage is uniform. The percentages for T and A are just under 30% and are just above 20% for G and C.

FIG. 2D shows a plot of base content percentage at positions in CTCF regions for reference genomic content. CTCF regions are known to be flanked by nucleosomes that have largely invariant positions in the eukaryotic genome, thereby showing any preferences depending on the function of the genomic region. For CTCF regions, the base content percentage flips at the CTCF site, with the content of G/C being higher than T/A.

FIG. 2C shows the base content of the 5' end of cfDNA fragments in WT EDTA 0 h samples in random regions. Thus, no incubation has occurred for FIG. 2C. The count data for the ending base was aggregated at each position to a randomly selected position, and the percentages were determined for the relative frequency of each base ending at that position. The base content percentage is shown for A-end 210, G-end 220, T-end 230, and C-end 240. If fragmentation were completely random, the end nucleotide proportions should reflect the composition of the mouse genome, which is 28.8% A, 28.8% T, 21.2% C, and 21.2% G, as shown in FIG. 2B. However, the 5' end of cfDNA fragments in randomly selected genomic regions show a substantial overrepresentation of C (32.6%), a slight overrepresentation of G (24.4%), and an underrepresentation of A (19.8%) and T (23.2%), as shown in FIG. 2C. Such changes indicate that the DNA is disproportionately fragmented at C and G positions, since A/T sites are more prevalent in the reference genome but appear less often at fragment ends..

FIG. 2E shows the base content of the 5' end of cfDNA fragments in WT EDTA 0 h samples in CTCF regions. The base content percentage is shown for A-end 210, G-end 220, T-end 230, and C-end 240. In these samples, C and G are overrepresented while A and T are underrepresented at the 5' ends of cfDNA fragments compared to the reference genomic content. Thus again, there is a preference for the natural fragmentation of circulating cfDNA to be at C/G sites than at A/T sites. Such asymmetric representation can also be seen for other regions.

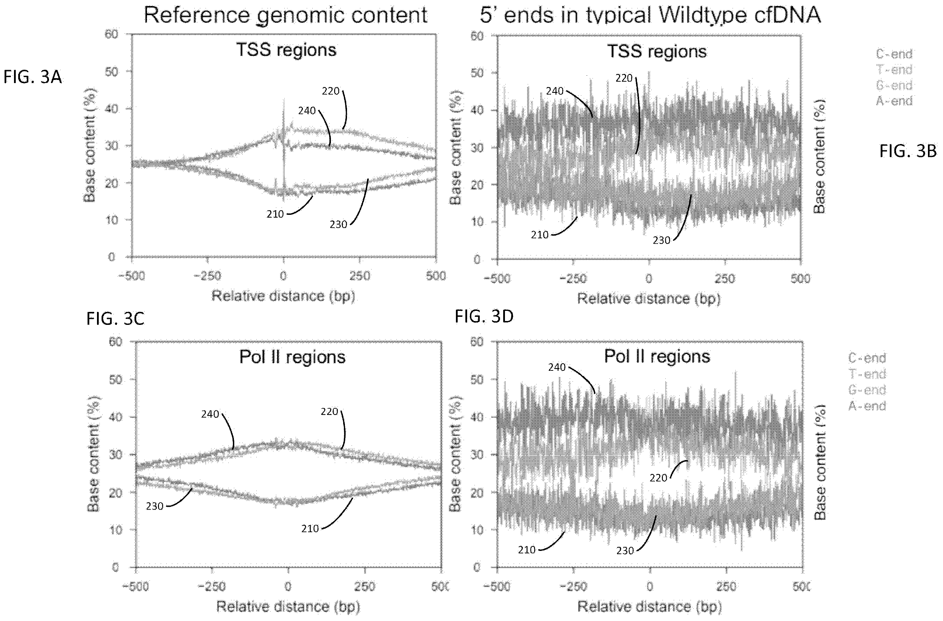

FIGS. 3A-3B show base content proportions in TSS regions according to embodiments of the present disclosure. The reference genomic content in TSS (FIG. 3A) regions is compared to the 5' end base content of cfDNA in WT EDTA 0 h samples (FIG. 3B). FIG. 3B shows an increase in C-ends relative to the reference content. The A-ends and T-ends are generally lower, and the G-ends are roughly the same.

FIGS. 3C-3D show base content proportions in Pol II regions according to embodiments of the present disclosure. The reference genomic content in Pol II (FIG. 3C) regions is compared to the 5' end base content of cfDNA in WT EDTA 0 h samples (FIG. 3D). FIG. 3D shows a large increase in C-ends relative to the reference content and a smaller increase for the G-ends. The A-ends and T-ends are generally lower. As with other figures, the base content percentage is shown for A-end 210, G-end 220, T-end 230, and C-end 240.

Accordingly, this pattern of asymmetric representation was also seen in cfDNA aligning to TSS and Pol II regions. Because CTCF regions contain an array of well-positioned nucleosomes flanking the CTCF binding site and because TSS and Pol II regions are known open chromatin regions, both nucleosomal and open regions of the genome display the same C-end overrepresentation.

### 3. End base content for different fragment sizes

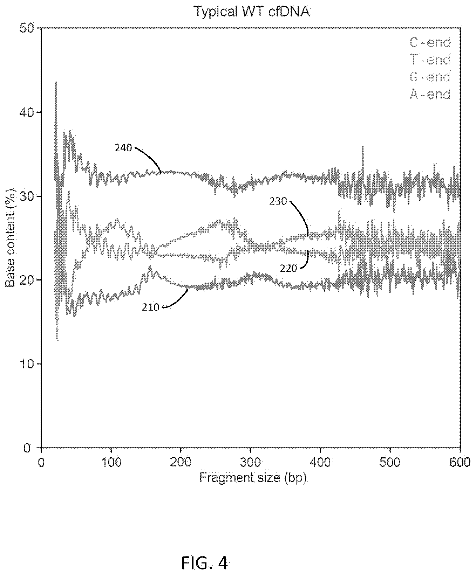

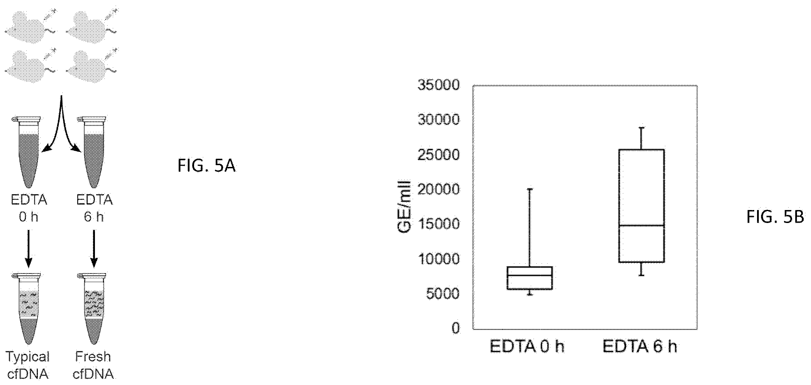

FIG. 4 shows base content of the 5' end of WT cfDNA across the range of fragment sizes according to embodiments of the present disclosure. The vertical axis is base content percentage, and the horizontal axis is fragment size. Each end of a fragment is counted independently. In different fragment sizes, C and G are overrepresented while A and T are underrepresented at the 5' ends of cfDNA fragments. As shown in FIG. 5, when the 5' ends are plotted across the 0 - 600 bp range of cfDNA fragment sizes, the over-representation of C-ends and underrepresentation of A-ends is evident and relatively uniform across all fragment sizes in wildtype cfDNA. Thus, C-end predominant cfDNA appears to be the typical cfDNA profile in WT mice across all fragment sizes.

### B. Fragmentation pattern in fresh cfDNA (e.g., for DFFB)

Fresh DNA can be obtained from cells in a whole blood sample, where the cells are caused to die by incubating the whole blood at room temperature in EDTA for a period of time. In this manner, the resulting plasma sample can be enriched for fresh DNA.

We explored whether, or not, this typical cfDNA profile (i.e., as shown in previous section) was created 'as is' from cellular sources, or produced after further digestion within the plasma. Thus, we sought to capture and analyze cfDNA that was freshly generated from dying cells and to compare its profile with the typical C-end predominant cfDNA profile that are shown above.

### 1. Changes in amounts of cfDNA with incubation

FIGS. 5A-5B show collection of EDTA 6 h samples enriched with fresh cfDNA according to embodiments of the present disclosure.

FIG. 5A shows cfDNA from WT mice being treated with EDTA over two time periods. Samples were enriched with fresh cfDNA by incubating whole blood in EDTA at room temperature for 6 hours. The incubation at room temperature with EDTA causes cells to die, thereby releasing fresh cfDNA (i.e., DNA that was not cell-free when the sample was first collected but has become cell-free). The influx of fresh cfDNA after incubation in each paired sample was confirmed by an increase in plasma cfDNA quantity of 1.1 to 5.9-fold.

FIG. 5B shows the increase in the concentration (genomic equivalents GE/ml) of cfDNA from no incubation to 6 hours of incubation at room temperature. An increase in long cfDNA fragments is also observed.

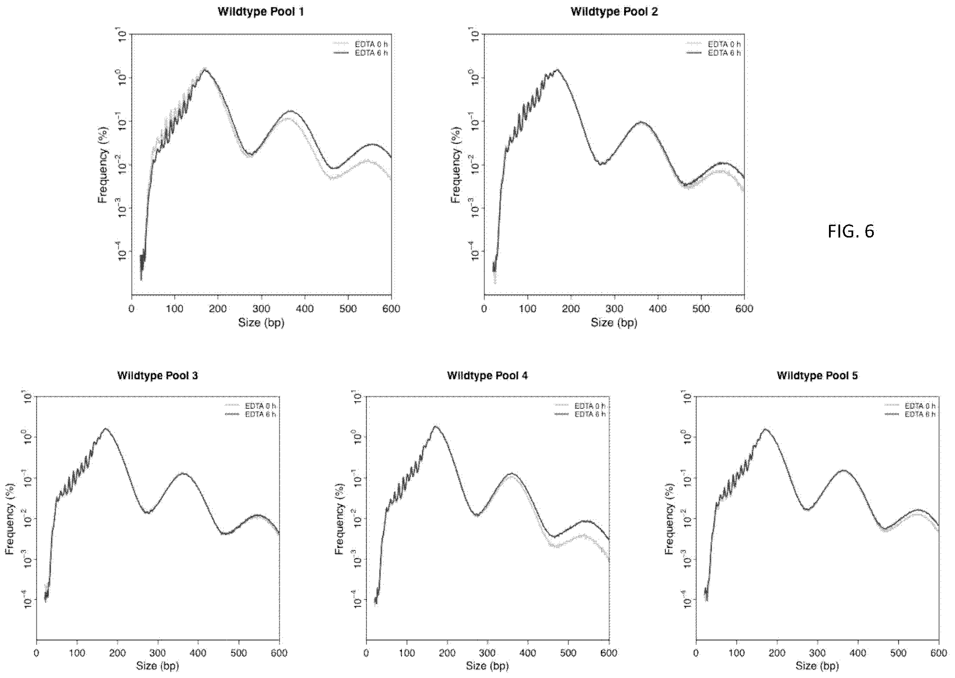

FIG. 6 shows size profiles 710 of samples without incubation (0 h) and size profiles 720 with incubation (6 h) for five different wildtype pools. Each pool contains DNA from a different group of mice that have the wild type (WT). The size profiles show a size (bp) of the DNA fragments on the horizontal axis, and a frequency (as a percentage) of the DNA fragments at a given size. The frequency of long DNA fragments (e.g., 350-600 bp) generally increases with the incubation, as shown by size profiles 720 being greater than size profiles 710 for the long DNA fragments.

Such behavior in FIGS. 5A, 5B, and 6 show that if whole blood is kept for a prolonged period of time, some of the blood cells that are present in the sample may start to leak cell-free DNA. Such leakage can be accounted for in any analysis and be used for applications, such as detection and other measurement.

### 2. A-end and G-end preference in fresh cfDNA

Besides an increase in cfDNA as a result of incubation with EDTA, changes in base end content was also investigated. The incubation of the blood sample with EDTA results in increases to the A-end and G-end content relative to the typical base end content in blood samples that have not been incubated. This increase is seen in various regions, including random regions, CTCF regions, TSS regions, and Pol II regions.

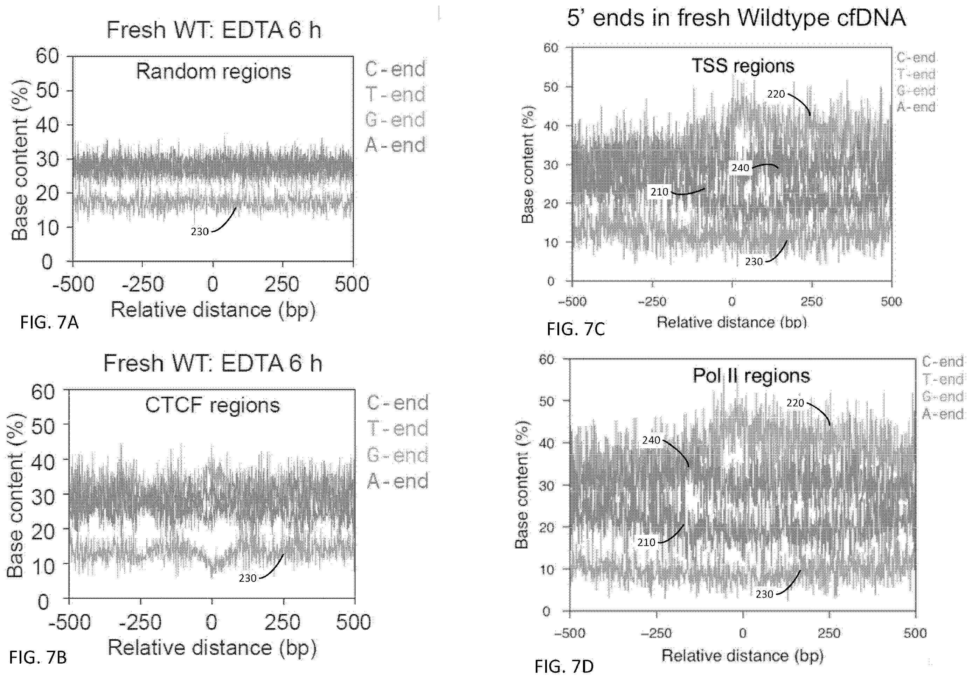

FIGS. 7A-7D show base content percentages of EDTA 6 h samples enriched with fresh cfDNA in mice for random, CTCF, TSS, and Pol II regions according to embodiments of the present disclosure. Relative to FIGS. 2C and 2E, the incubation increases the frequency of A-end and G-end, indicating a preference for A and G in the fragmentation that occurs during incubation.

FIG. 7A shows the base content percentage in random regions for fresh cfDNA samples as prepared by incubating blood samples with EDTA over 6 hours according to embodiments of the present disclosure. Analyzing the 5' ends of cfDNA in the 6 h EDTA sample, the C-end predominance seen in typical cfDNA was greatly diminished in the presence of fresh cfDNA, as compared with its baseline 0 h incubation, as shown in FIG. 2C. C-end and T-end fragments decreased to 28.3% and 17.0%, respectively. A-end and G-end fragments increased substantially to 27.7% and 27.0%, respectively, in randomly selected genomic regions.

FIG. 7B shows the base content percentage in CTCF regions for fresh cfDNA samples according to embodiments of the present disclosure. The changes in base content for random regions were also consistently visualized in the CTCF regions with nucleosomal arrays. In comparing FIG. 7B with FIG. 2E, one can see that A-end content increases from just under 20% to between ~20-30%, and G-end content increases from generally under 30% to above 30%.

FIG. 7C shows base content proportions in TSS regions in EDTA 6 h samples enriched with fresh cfDNA according to embodiments of the present disclosure. In comparison to FIG. 3B, one can see an increase in A-end content from below 20% to above 20%, and an increase in G-end content from below 30% to above 30%.

FIG. 7D shows base content proportions in Pol II regions in EDTA 6 h samples enriched with fresh cfDNA according to embodiments of the present disclosure. In comparison to FIG. 3D, one can see an increase in A-end content from below 20% to above 20%, and an increase in G-end content from generally about 30% to around 40% and above.

Therefore, fresh cfDNA after whole blood incubation were enriched for A- and G-end fragments when compared to typical cfDNA. Since the fresh cfDNA profile from dying cells does not appear similar to the typical C-end predominant cfDNA found in baseline samples, we inferred that the typical C-end predominant cfDNA would be created in a subsequent step. Since the fragment end preference (e.g., for enrichment of A-ends) after incubation is different (e.g., A-end vs C-end), we also reasoned that the generation of fresh cfDNA likely originated from a different mechanism than that which created the typical cfDNA. The enrichment for A-ends occurs in longer cfDNA as shown in later sections.

### 3. A-ends and G-ends among fresh cfDNA of different sizes

We also explored the base end preference by fragment size. We identified fragments by their two end nucleotides and analyzed the fragments in which both ends terminated with A, G, C, or T. These fragments where both ends were identified were denoted with their end nucleotides and the symbol <> in between, such that a fragment with both ends as A would be designated as A<>A. We compared the proportional representation of A<>A, G<>G, C<>C, and T<>T fragments among different sizes reasoning that any preference for cutting a particular nucleotide would be most well-visualized with these fragment types where both ends encompassed the same nucleotide preference. Of these four types of fragments, 6 h samples enriched with fresh cfDNA had a significantly higher proportion of A<>A fragments in sizes >150 bp and increased further in long fragments ≥250 bp. On the other hand, G<>G, C<>C, and T<>T fragments did not differ significantly by size. Thus, fresh cfDNA was enriched for A-end fragments that were longer than 150 bp.

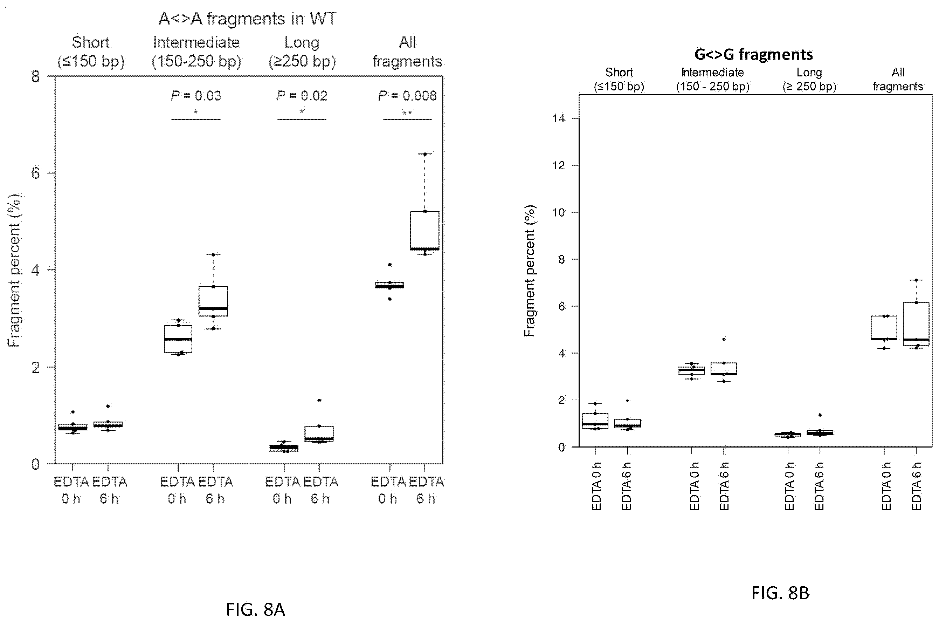

FIG. 8A shows A<>A fragment proportions compared between baseline cfDNA (EDTA 0 h) and samples enriched with fresh cfDNA (EDTA 6 h) in WT mice among short, intermediate, and long fragments according to embodiments of the present disclosure. P-value calculated by Mann-Whitney *U* test. In FIG. 8A, four categories show analysis for short (≤150 bp), intermediate (150-250 bp), long (≥250 bp), and all fragments. For each category, measurements for 0 h and 6 h of EDTA are shown. The percent increases noticeably for intermedia and long, as well as all A<>A fragments. The increase in the A<>A might be related to the DNA fragmentation factor subunit beta (DFFB) nuclease cutting intracellular DNA (i.e., inside the cell) from the blood and then releasing that cell-free DNA into the plasma, as is analyzed below.

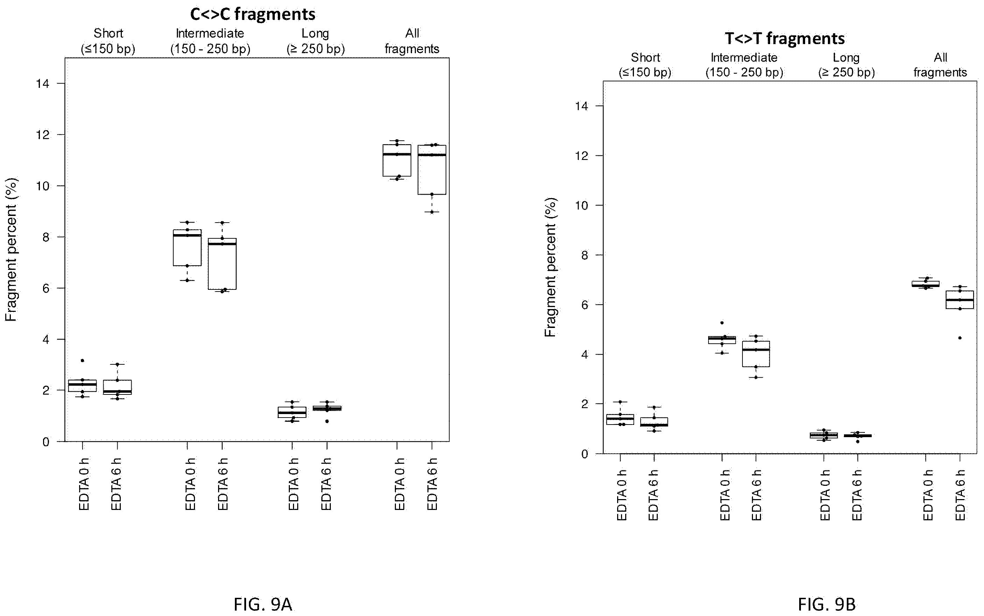

FIG. 8B shows size profiles for G<>G, and FIGS. 9A-9B show size profiles for C<>C, T<>T fragment proportions in WT mice compared between EDTA 0 h and EDTA 6 h among short, intermediate and long fragments. P-value calculated by Mann-Whitney U test. As mentioned above, the amounts of G<>G, C<>C, and T<>T fragments did not differ significantly by size.

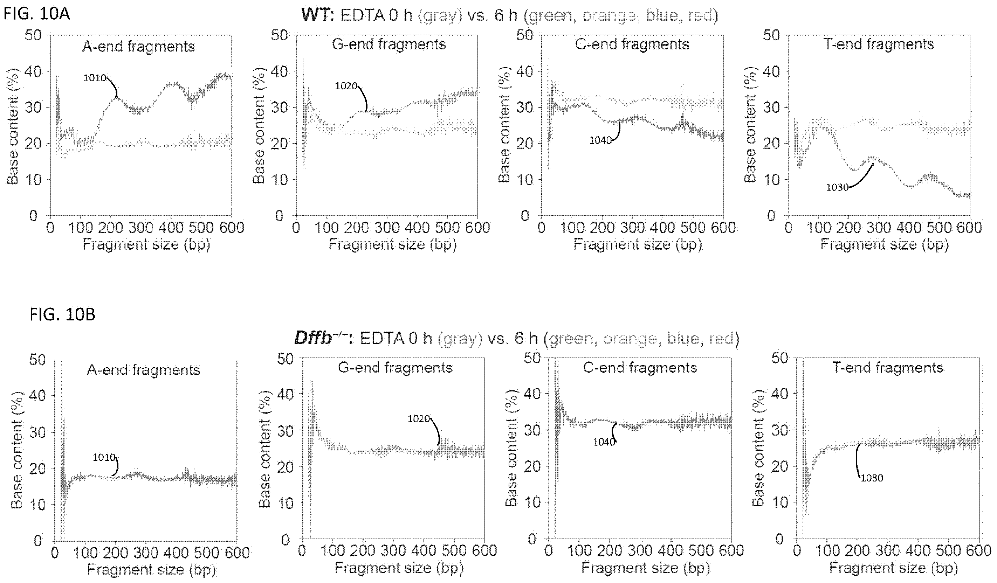

FIG. 10A shows the proportion of A-end, G-end, C-end, and T-end fragments for each fragment size compared to the respective baseline unincubated EDTA levels. In FIG. 10A, the counting is for single end, as opposed to the double end, as in FIGS. 8A-9B. Specifically, FIG. 10A shows percentages of cfDNA with A-ends 1010 (green), G-ends 1020 (orange), C-ends 1040 (blue), and T-ends 1030 (red) in WT EDTA 6 h samples enriched with fresh cfDNA compared with the baseline representation in EDTA 0 h samples (gray). As shown, the A-ended and G-ended fragments increase, and the C-end and T-end fragments decrease. Because these are percentages, when there is an increase of certain groups of content, there is a corresponding decrease in other content.

Surprisingly, the increase in long A-end fragments was concentrated at specific size ranges, with peaks at ~ 200 bp and 400 bp that were reminiscent of nucleosomal ladder sizes. G-end fragments also had a similar but weaker periodicity at these sizes. We hypothesized that these A-end (and G-end) cfDNA fragments were likely created by cleaving between nucleosomes, such that the full length of an intact nucleosomal DNA was retained. The peaks in periodicity would support a true preference for cutting at the inter-nucleosomal regions 5' to an A with a slightly smaller preference for cutting 5' to a G.

### 4. Effects of DFFB on cfDNA with A-ends

Since A-end long fragments were generated freshly from dying cells, we examined the role of apoptosis in their generation. Since DFFB is the major intracellular nuclease involved in DNA fragmentation during apoptosis, we investigated samples from Dffb-deficient mice, which have that gene knocked out in both alleles, signified by Dffb^{-/-}.

FIG. 10B shows percentages of cfDNA with A-ends (green), G-ends (orange), C-ends (blue), T-ends (red) in *Dffb*-deficient EDTA 6 h samples compared to its baseline representation in EDTA 0 h samples (gray). Comparing A-end, G-end, C-end, and T-end fragment proportions at each fragment size, there was little change in *Dffb*-deficient mice after 6 h of EDTA incubation compared with the baseline, with no periodicity in the A-end and G-end fragments. Hence, in *Dffb*-deficient mice, the increase in A-end fragments that was observed in WT mice was absent, suggesting that DFFB might have a major role in generating these A-end long fragments.

We further investigated the overall change in cfDNA after incubation and for fragment size, as well as for different regions. There was essentially no change after incubation.

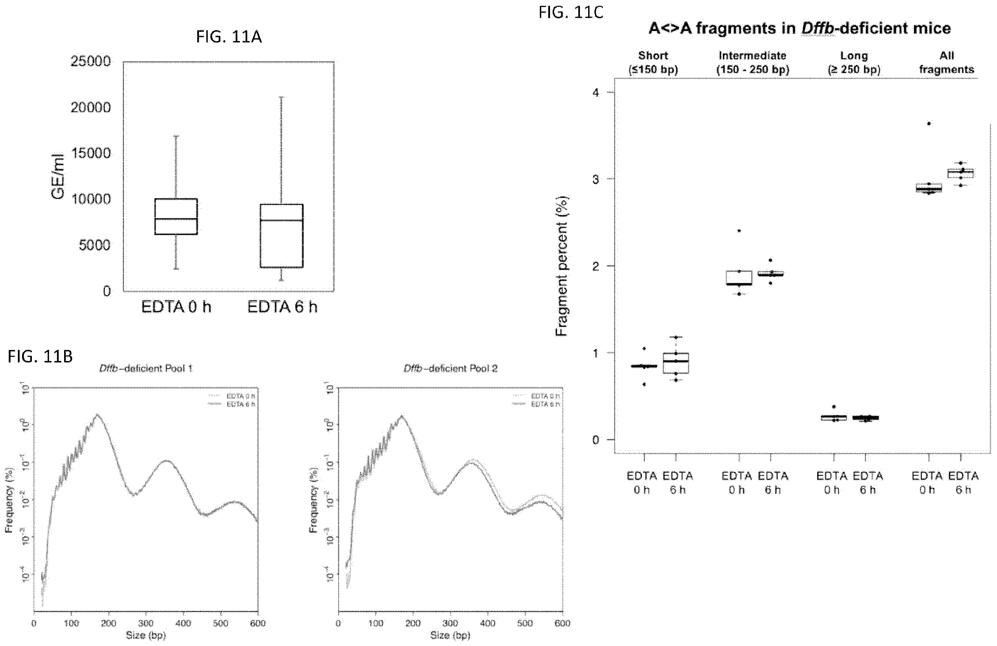

FIG. 11A shows a concentration of cfDNA in EDTA 0 h vs 6 h samples in *Dffb-*deficient mice according to embodiments of the present disclosure. After 6 h of EDTA incubation, cfDNA quantity did not significantly increase.

FIG. 11B shows size profiles in EDTA 0 h vs 6 h samples in *Dffb*-deficient mice according to embodiments of the present disclosure. There was little or no increase in long fragments.

FIG. 11C shows A<>A fragment proportions in *Dffb*-deficient mice compared between EDTA 0 h and EDTA 6 h among short, intermediate and long fragments according to embodiments of the present disclosure. A<>A fragment percentages did not increase after 6 h of EDTA incubation in *Dffb*-deficient mice, unlike in WT mice, as shown in FIG. 8A.

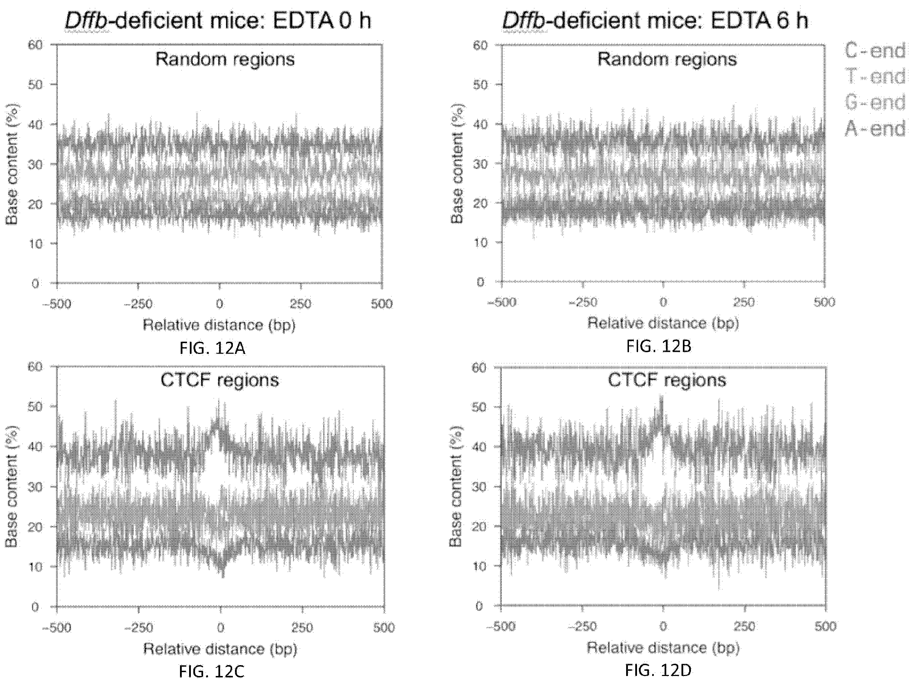

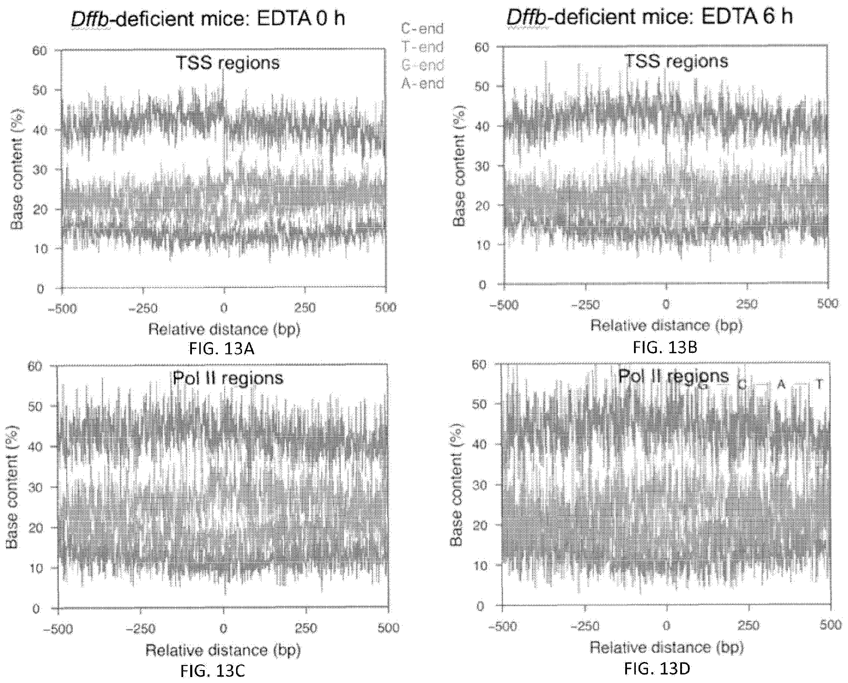

FIGS. 12A-12D shows base content proportions in *Dffb*-deficient mice in EDTA 0 h and 6 h samples for random regions and CTCF regions according to embodiments of the present disclosure. FIGS. 13A-13D shows base content proportions in *Dffb*-deficient mice in EDTA 0 h and 6 h samples for TSS regions and Pol II regions according to embodiments of the present disclosure. In random genomic regions, CTCF, TSS, and Pol II regions, the A-end fragments did not increase.

If the change in FIG. 10A was not seen, this would show an animal (e.g., human or mouse) had a deficiency in a nuclease, e.g., DFFB. Such a change can be analyzed by incubating at two different times (e.g., 0 hours and 6 hours), and comparing the size profiles at those two different times. The lack of the change may indicate a deficiency in any one of the nucleases that perform intracellular cutting, with a further analysis potentially providing details as to which nuclease.

### III. EFFECT OF DNASE1L3 ON TYPICAL CFDNA

While the above analysis characterizes the end base content and size profiles of freshly generated cfDNA, this section analyzes the process in which the typical C-end predominance was produced in plasma cfDNA. This clear preference for C-ends in all sizes of circulating cfDNA fragments seen in FIG. 4 suggests the presence of a nuclease that prefers to cleave 5' to a C. Previously, we had demonstrated that cfDNA from WT mice had a high frequency of fragments ending in CCNN motifs and that this preference for CCNN motifs in cfDNA fragment ends was reduced in *Dnase1l3*-deficient mice (Serpas, L. et al. (2019), Proceedings of the National Academy of Sciences 116, 641-649). We hypothesized that the nuclease responsible for the C-end preference might also be DNASE1L3. To investigate this hypothesis, we compared the specific A<>A, G<>G, C<>C, and T<>T fragment proportions between *Dnase1l3*-deficient mice (Dnase1l3^{-/-}) and WT mice

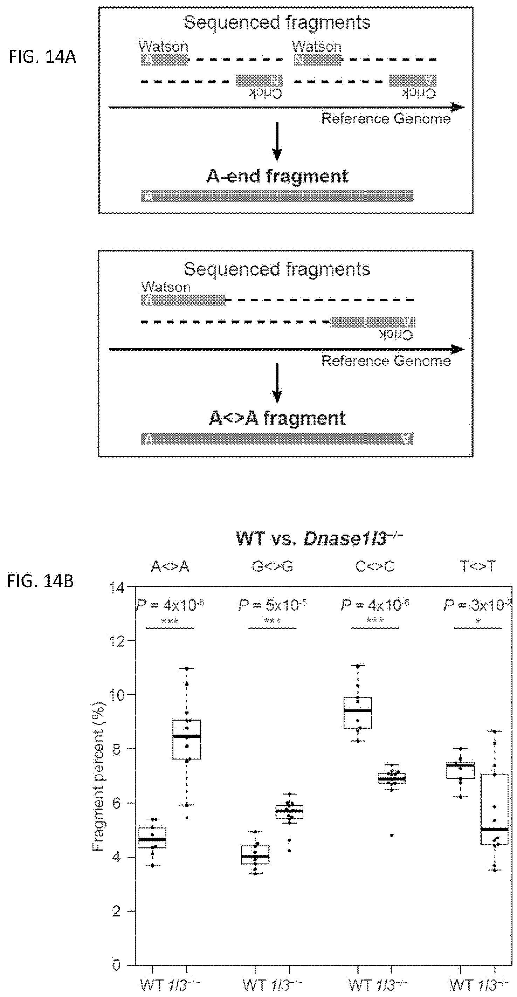

FIGS. 14A shows the construction of an A<>A fragment according to embodiments of the present disclosure. FIG. 14A shows an A-end fragment and an A<>A fragment. An A-end fragment has an A at the 5' end of the Watson strand or at the 5' end of the Crick strand. The other end can be signified with N, since the base could be any base. An A<>A fragment has an A at the 5' end of the Watson strand and an A at the 5' end of the Crick strand. Such nomenclature also applies to C<>C, G<>G, and T<>T, all of which are used throughout the disclosure.

FIG. 14B shows end base contents of *Dnase1l3*-deficient samples compared to WT samples according to embodiments of the present disclosure. The base content data is for double-sided ends for the same base. FIG. 14B shows A<>A, G<>G, C<>C, and T<>T fragment percentages in WT vs *Dnase1l3*-deficient (*1l3*^{*-*/*-*}) mice (both EDTA 0 h). The vertical axis is the fragment percent in the sample. The horizontal axis corresponds to WT and *1l3*^{*-*/*-*} for the four categories (other categories, e.g., A<>T, not shown). The P-value is calculated by the Mann-Whitney *U* test. The percentages of A<>A and G<>G increase for *1l3*^{*-*/*-*} (i.e., were higher than in WT), while the percentage of C<>C decreases significantly and the percentage of T<>T decreases for *1l3*^{*-*/*-*} (i.e., were lower than in WT). Such changes are consistent with *Dnase1l3* having a preference for cutting C since the lack of *Dnase1l3* would not cut at C, while other nucleases with other base cutting preferences would still exists and cut at those other bases.

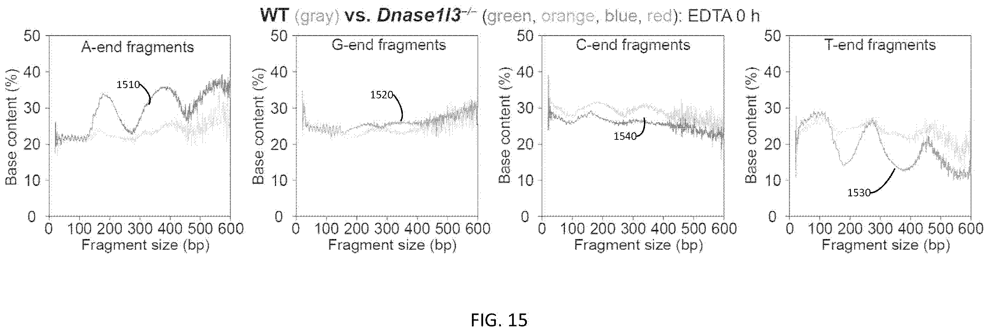

FIG. 15 shows end base contents of *Dnase1l3*-deficient samples compared to WT samples per fragment size according to embodiments of the present disclosure. FIG. 15 shows percentages of A-ends 1510 (green), G-ends 1520 (orange), C-ends 1540 (blue), and T-ends 1530 (red) in DNASE1L3-deficient EDTA 0 h cfDNA compared with the baseline representation of WT EDTA 0 h cfDNA (gray).

In FIG. 15, comparing the A-end, G-end, C-end, and T-end fragment proportions of each fragment size between the *Dnase1l3*-deficient mice and WT mice in EDTA 0 h samples, there is a decrease in C-end fragments at all fragment sizes, consistent with our findings that C<>C fragments decrease. The A-end fragments also demonstrate a nucleosomal periodic pattern with peaks in frequency ~200 bp and 400 bp. Accordingly, in the *Dnase1l3*-deficient mice, there is an increase in the A-end fragments, particularly at these peaks. There is a corresponding decrease in T-end fragments, particular at these peaks. This nucleosomal periodic pattern of A-end fragments is similar to the one observed previously in WT EDTA 6 h samples enriched with fresh cfDNA (FIG. 10A). Thus, the outcome of the DFFB cutting would be the A-end fragments with the periodic pattern, which usually would be quickly turned into C-ends by DNASE1L3. But, because the DNASE1L3 is not there, there is an A-end fragment increase. Also, as a result, the A-end becomes the dominant species as opposed to the C-end.

These results suggest that DNASE1L3 generates both C- and T-end fragments, with a greater preference for C-ends since C<>C fragment percentages are more significantly reduced.

Hence, it appeared that DNASE1L3 deficiency resulted in exposing the profile of fresh cfDNA. In a substrate-enzyme-product relationship, when the enzyme is deficient, the product would decrease and the substrate would increase. Thus, DNASE1L3-deficient cfDNA seemed to have revealed its substrate cfDNA profile, which appeared to be the cfDNA profile created by DFFB. This suggests that at least some cutting by DNASE1L3 occurs in circulating blood while DFFB cutting tends to occur within the cell.

With a more detailed look at the fragment types using both ends of a cfDNA fragment, we found that only A<>A, A<>G, and A<>C fragments demonstrated this nucleosomal periodic pattern in both *Dnase1l3*-deficient samples and WT EDTA 6 h samples enriched with fresh cfDNA.

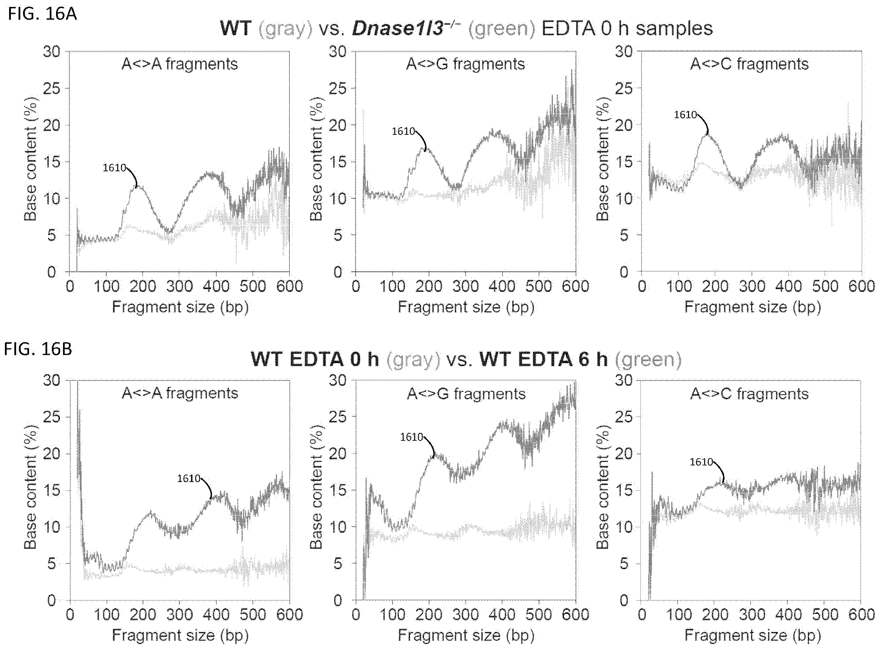

FIG. 16A shows percentages of A<>A, A<>G, and A<>C fragments in *Dnase1l3-*deficient EDTA 0 h cfDNA compared with the baseline representation of WT EDTA 0 h cfDNA (gray) according to embodiments of the present disclosure. FIG. 16B shows percentages of A<>A, A<>G, and A<>C fragments in WT EDTA 6 h samples enriched with fresh cfDNA compared to the baseline representation of WT EDTA 0 h cfDNA (gray) according to embodiments of the present disclosure. The data 1610 is for the *Dnase1l3*-deficient samples. The gray lines for the two figures correspond to different batches for WT EDTA 0 h.

There were a number of notable differences between the fragments of these two sample types. In *Dnase1l3*-deficient mice, the periodic pattern of the A<>A, A<>G, and A<>C fragments was very prominent (FIG. 16A). Since DNASE1L3 activity is absent in *Dnase1l3-*deficient mice, this prominence in the cfDNA likely reflects the true preference for nucleosomal periodic cutting in the remaining active intracellular nucleases, notably DFFB.

On the other hand, the periodic pattern seen in the fresh cfDNA was attenuated, which was especially noticeable amongst A<>C fragments (FIG. 16B). Since DNASE1L3 activity is retained in the generation of fresh cfDNA compared with *Dnase1l3*-deficient mice, this difference indicates that DNASE1L3 would play a role in creating A<>C fragments, which might be an intermediate step to creating C<>C fragments. These results also indicate that DNASE1L3 attenuates the preferential cutting of the DFFB nuclease by cutting after DFFB. Thus, it can be inferred that DNASE1L3 cutting occurs predominantly as a subsequent step to DFFB cutting, and that DNASE1L3 might not only have a role, but may actually be a dominant player in creating the typical profile with C-end predominance in cfDNA (FIG. 4).

### IV. EFFECTS OF DNASE1 ON CFDNA (WITH HEPARIN)

While we have demonstrated the steps involved in creating a typical cfDNA fragment with C-end predominance, we also explore how a cfDNA fragment might be further digested, so that a full picture of the homeostasis of cfDNA can be constructed. While C-end fragments continue to be the most prevalent even in short fragments <150 bp, we noted an enrichment of T-end fragments in sizes ~50-150 bp and ~ 250 bp in the typical cfDNA profile (FIG. 4). These peaks were not concordant with either the C-end fragments, which were related to DNASE1L3 preference or the A-end fragments which were related to DFFB cutting preference. With our theory that fragment ends correlated with nuclease preference, we explored whether or not these T-ends might be related to DNASE1 preference.

### A. Effect of deletion in Dnase1

To identify DNASE1's cutting preference, we collected whole blood from *Dnase1*^{*-*/}*⁻, Dnase1*^{*+*/*-*}, and WT mice, pooled the samples within a type, and equally distributed each pool into tubes for 0 h or 6 h incubation with heparin. Heparin was used instead of EDTA since it is known to enhance DNASE1 activity while inhibiting DNASE1L3 (Napirei, M. et al., (2005), The Biochemical journal 389, 355-364). Heparin has also been shown to displace nucleosomes.

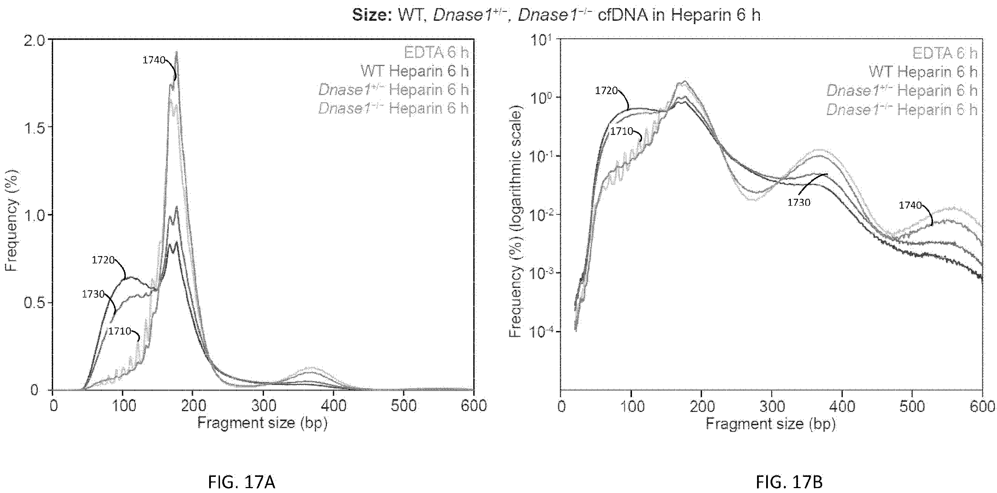

FIGS. 17A-17B show size profile of cfDNA of WT, *Dnase1*^{*+*/*-*}, and *Dnase1*^{*-*/*-*} mice with incubation in heparin according to embodiments of the present disclosure. Regular (FIG. 17A) and logarithmic (FIG. 17B) scales are provided. FIG. 17A shows cfDNA size profiles for blood with EDTA after 6 h (grey) 1710, as well as data for blood treated with heparin after 6 h for WT, (blue) 1720, *Dnase1*^{*+*/*-*} (green) 1730, and *Dnase1*^{*-*/*-*} (red) 1740 mice. We found that in WT and *Dnase1*^{*+*/*-*} mice, 6 h of heparin incubation resulted in a striking increase in short fragments with a reduction in the 166 bp peak and a loss of nucleosomal pattern. In *Dnase1*^{*-*/*-*}, no size changes occurred, and the size pattern was essentially the same as cfDNA from EDTA blood.

To show that this effect is due to Dnase1, the blue curve 1720 (WT heparin 6 h) can be compared to the red curve 1740 (Dnase1^{-/-}, which is plasma collected from mice with homozygous knockout of Dnase1). When Dnase1 is not present, there is no increase in the very short DNA molecules. And there is still an emergence (although less) of the very short DNA molecules in the green curve 1730 for Dnase1^{+/-}, which is heterozygous such that only one allele has the gene missing. The logarithmic plot helps to show the change in the amounts of longer fragments.

Accordingly, embodiments can detect a disorder in Dnase1 (e.g., a deletion) by treating a sample with heparin and comparing the sample to a WT size distribution.

We also examined these samples for a difference in fragment end proportions.

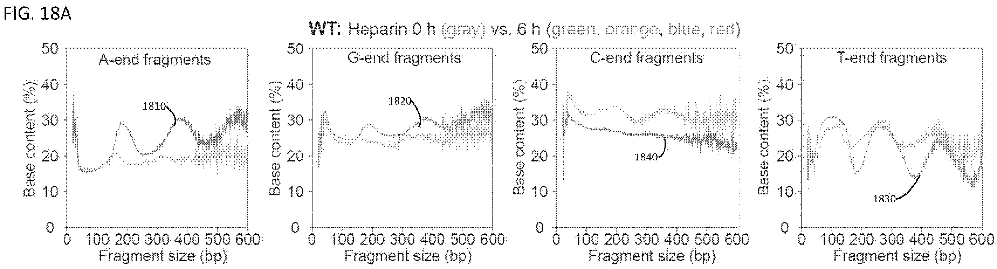

FIGS. 18A-18B show size profiles and base content of cfDNA of WT and *Dnase1*^{-/-}mice with incubation in heparin according to embodiments of the present disclosure. The data for the end fragments is for single-ended data.

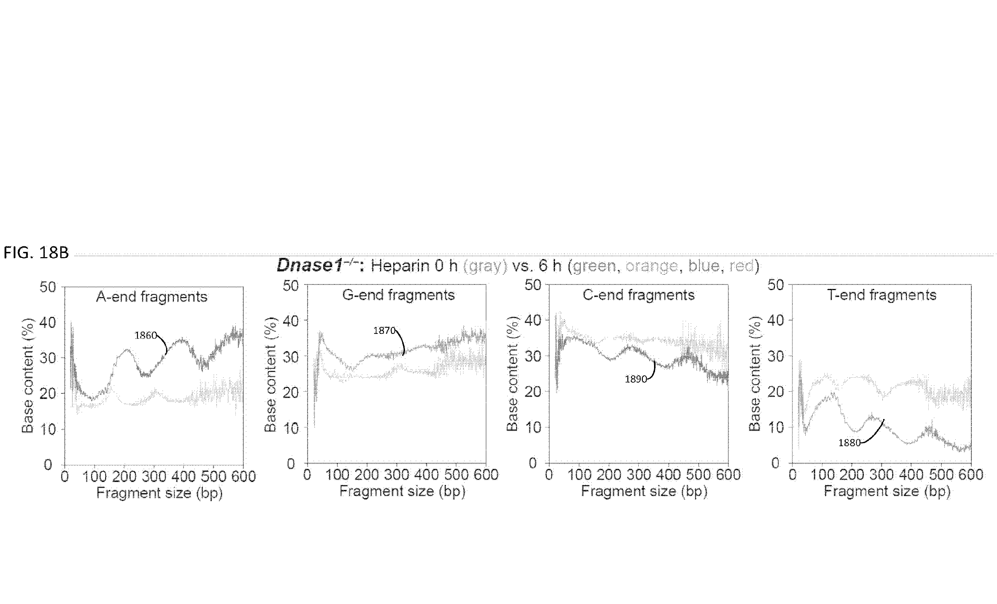

FIG. 18A shows percentages of A-ends 1810 (green), G-ends 1820 (orange), C-ends 1840 (blue), and T-ends 1830 (red) of WT Heparin 6 h samples compared to its baseline representation in Heparin 0 h (gray). FIG. 18B shows percentages of A-ends 1860 (green), G-ends 1870 (orange), C-ends 1890 (blue), and T-ends 1880 (red) in Heparin 6 h cfDNA of *Dnase1*^{-/-} mice compared to its baseline representation in Heparin 0 h (gray).

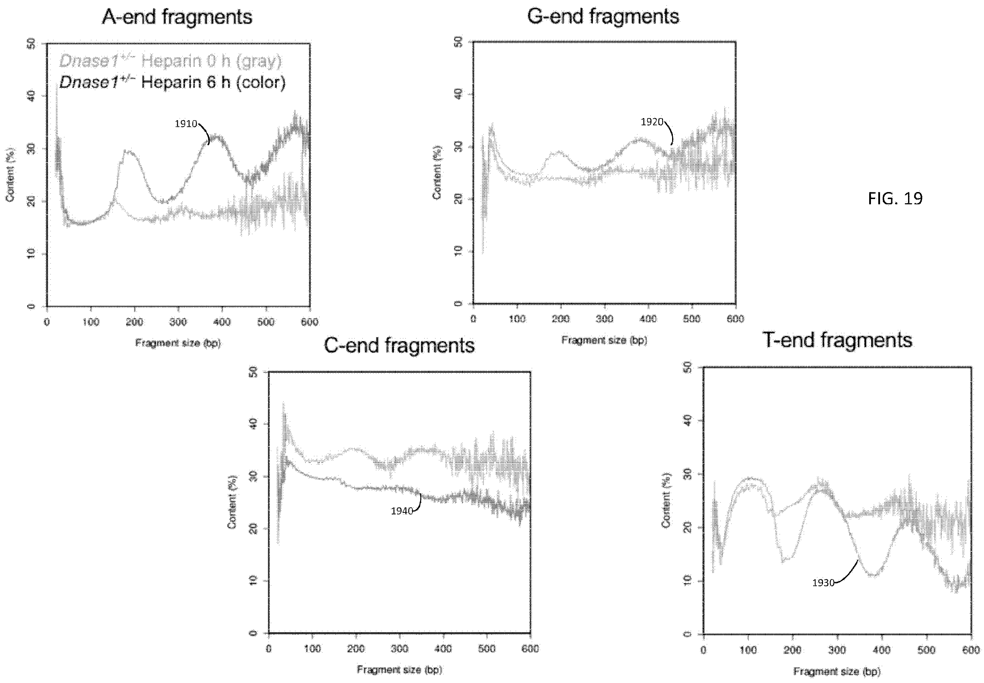

FIG. 19 shows size profiles and base content of cfDNA of *Dnase1*^{*+*/*-*} mice with incubation in heparin according to embodiments of the present disclosure.. Heparin effect in WT, *Dnase1*^{*+*/*-*}, *Dnase1*^{*-*/*-*} mice. FIG. 19 shows percentages of cfDNA with A-ends 1910 (green), G-ends 1920 (orange), C-ends 1940 (blue), and T-ends 1930 (red) in *Dnase1*^{*+*/*-*} cfDNA after 6 h heparin incubation compared with its baseline at 0 h incubation (gray).

In WT and *Dnase1*^{*+*/*-*} mice after 6 h heparin incubation, T-end fragment proportions increased in fragments sized ~50-150 bp (FIG. 18A, FIG. 19). In contrast, in *Dnase1*^{*-*/*-*} mice, this increase was absent (FIG. 18B). These observations supported our hypothesis that DNASE1 might prefer to create T-end fragments. In general, the base content for T-ends were higher for the WT and *Dnase1*^{*+*/*-*} than for *Dnase1*^{*-*/*-*}. In addition, the long A-end fragments with nucleosomal periodicity was present after 6 h heparin incubation in WT, *Dnase1*^{*+*/*-*}, and *Dnase1*^{*-*/*-*} mice. Such an observation of the A-end fragments is consistent with an increase in cfDNA due to cell death of cells in the blood sample, similar to EDTA.

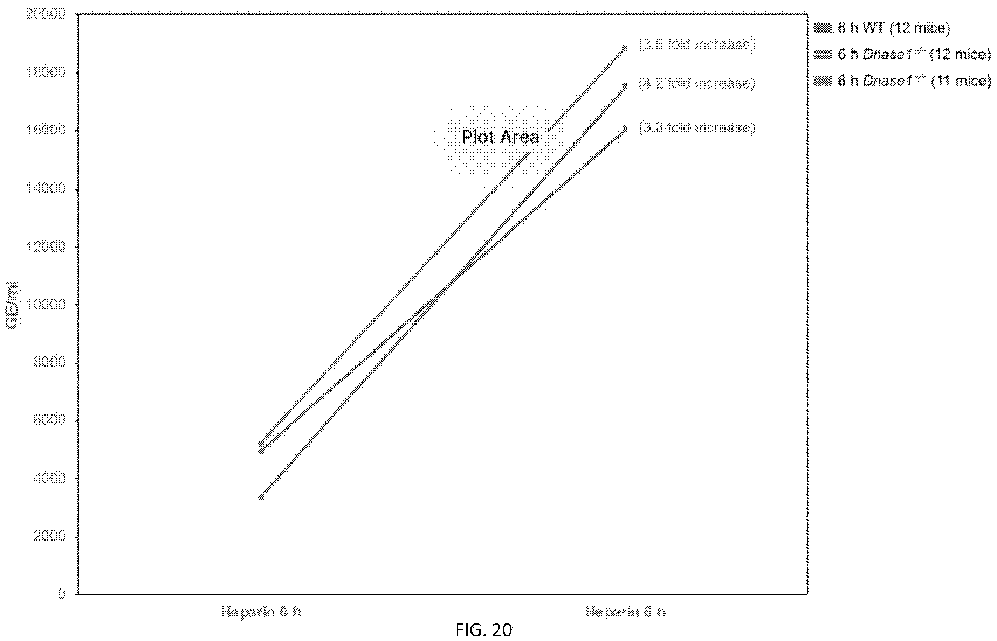

FIG. 20 shows cfDNA quantity for WT, *Dnase1*^{*+*/*-*}, and *Dnase1*^{*-*/*-*} mice with in 0 h and 6 h samples in heparin according to embodiments of the present disclosure. The concentration of cfDNA in genomic equivalents per ml is on the vertical axis, the horizontal axis has the different times for incubation with heparin. As can be seen, the amount of cfDNA increases with incubation time.

Combining the increase in cfDNA amount in all three genotypes with the literature on heparin incubation inducing apoptosis (Manaster, J. et al., (1996), British Journal of Haematology 94, 48-52), the presence of the A-end DFFB signature from freshly apoptotic cfDNA was consistent. An increase of cfDNA with fresh A-end fragments from *DFFB* were quickly digested to short T-end fragments (due to heparin enhancement of DNASE1 in WT mice), suggesting that DNASE1 preferred to cut 5' to T.

### B. Periodicity from fragments cut from nucleosomes

We analyzed the periodicity of fragments with EDTA, heparin, and varying time of incubation. The results are consistent with DNASE1 having a preference to cut T-ends, and with heparin disrupting the nucleosome structure in plasma.

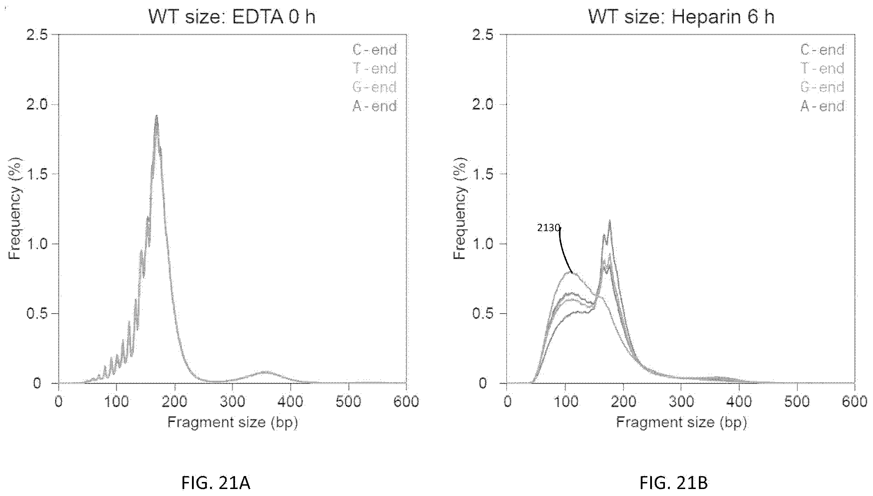

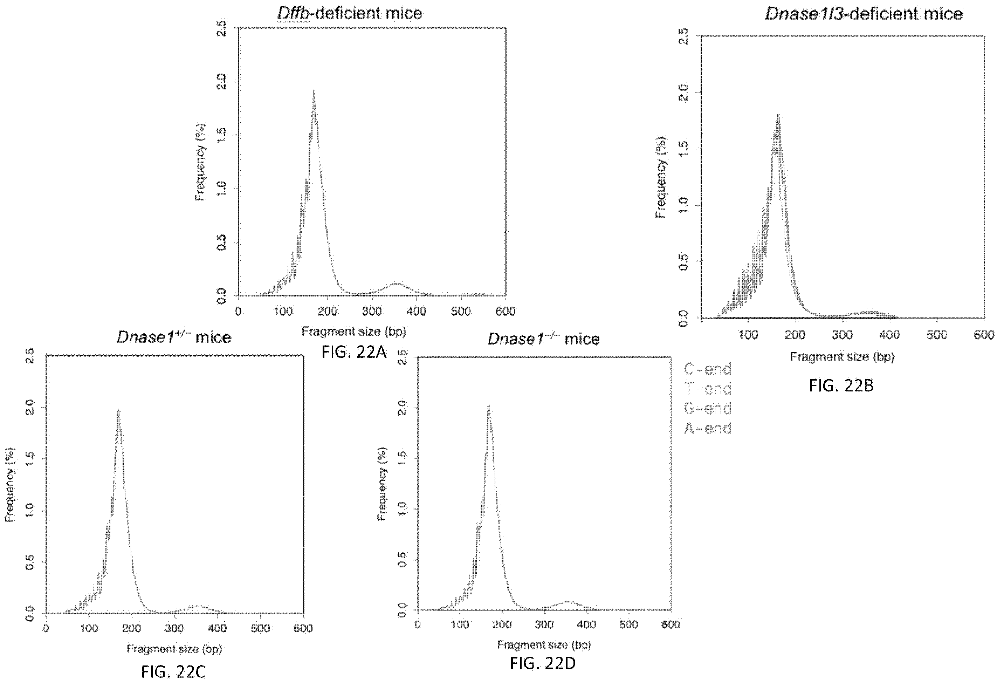

FIG. 21A shows a cfDNA size profile of A-end, G-end, C-end, and T-end fragments in an EDTA 0 h WT sample according to embodiments of the present disclosure. The frequencies are determined within a particular ending base type, e.g., each frequency value at a particular size for G-ends is normalized by the total number of G-ended fragments. Notably, all A-end, G-end, C-end, and T-end fragment types demonstrated a 10 bp periodicity for frequency in the short ≤150 bp fragments among all mice genotypes (WT, *Dnase1l3*^{*-*/}*⁻, Dffb*^{*-*/}*⁻, Dnase1*^{*+*/*-*}, and *Dnase1*^{*-*/*-*}). FIGS. 22A-22D show cfDNA size profiles of A-end, G-end, C-end, and T-end fragments in EDTA 0 h sample of *Dffb*^{*-*/}*⁻, Dnase1l3*^{*-*/}*⁻, Dnase1*^{*+*/*-*}, and *Dnase1*^{*-*/*-*} mice according to embodiments of the present disclosure. The 10 bp periodicity in the peak values is particularly prominent in FIG. 22B for *Dnase1l3*^{*-*/}*⁻.*

Other than the C-end preference for all cfDNA sizes, there was no particular end preference related to the 10 bp period fragments. Thus, it would be unlikely that a single particular nuclease would be responsible for the 10 bp periodicity. In fact, the prevailing theory for the 10 bp periodicity is that the 10 bp periodicity is a result of nuclease digestion of DNA within an intact nucleosome. This was postulated from the combined effect of restricted nuclease access to the DNA wrapped around histones with the periodic exposure of one strand of DNA over the other due to 10 bp per turn structure of the DNA helix (Klug, A., and Lutter, L.C. (1981), Nucleic Acids Res 9, 4267-4283).

FIG. 21B shows a cfDNA size profile of A-end, G-end, C-end, and T-end fragments in a Heparin 6 h WT sample according to embodiments of the present disclosure. In our heparin model, which disrupted the nucleosome structure in plasma, the 10 bp periodicity was abolished in all fragment types after 6 h heparin incubation in WT. Further, the T-end 2130 increases among the small fragments. This increase in T-end 2130 as a result of heparin disrupting the nucleosome structure is consistent with DNASE1 being prevalent in plasma (as opposed to within the intact cell) and having a preference for cutting T ends. Such changes for T-ended fragments at sizes around 50-150 bp with heparin incubation can be used to detect genetic disorders with DNASE1, e.g., if the expected increase for the T-ended fragments does not occur.

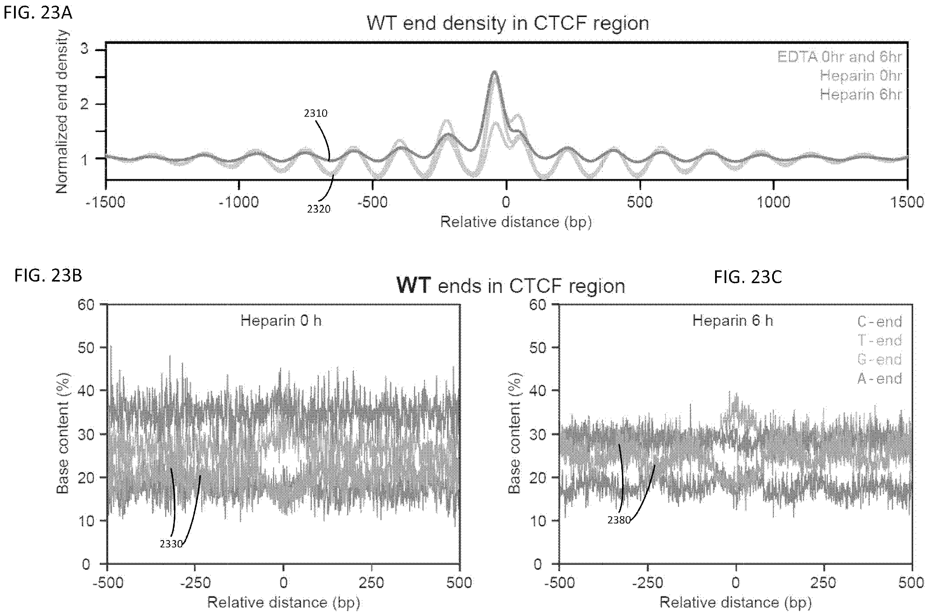

FIG. 23A shows fragment end density in the CTCF region in the Heparin 6 h sample (red line 2310) compared to the baseline samples (EDTA 0 h and 6 h, Heparin 0 h) (gray lines 2320) according to embodiments of the present disclosure. The gray lines 2320 are from the three identified samples. These three lines show some different around position 0, but have similar periodicity in the peaks away from position 0.

A CTCF region is special in that the nucleosomal spacing is very clear. Looking at the gray lines 2320 (EDTA and heparin with no incubation), there is a very good periodicity, but the wave pattern is reduced in the presence of heparin (red line 2310), which disrupts the nucleosomal structure so that cutting may occur at places in the nucleosomal DNA that are usually relatively inaccessible. Accordingly, at the well-phased nucleosomes in the CTCF region, fragment ends within the nucleosome increase with heparin 6 h incubation in WT. Thus, the disrupted nucleosome structure (as a result of heparin incubation) resulted in intra-nucleosomal DNA being cut.

FIGS. 23B-23C show 5' end base representation in the CTCF region of Heparin 0 h and 6 h samples of WT according to embodiments of the present disclosure. We explored which fragment types would contribute to the intra-nucleosomal fragments mentioned above. In WT heparin 6 h, a periodicity in T-end fragments corresponding to the intranucleosomal position was apparent (FIG. 23C). Also, there was an increase in the T-end fragments 2330 having on average about 20% with a low of about 15% (at position 0) in WT heparin 0 h (FIG. 23B) to T-end fragments 2380 having a low of 20% at position 0 with peaks at 30%. These results together support that heparin enhances DNASE1 and disrupts the nucleosomal structure, allowing DNASE1 with T-end preference to cleave intranucleosomally.

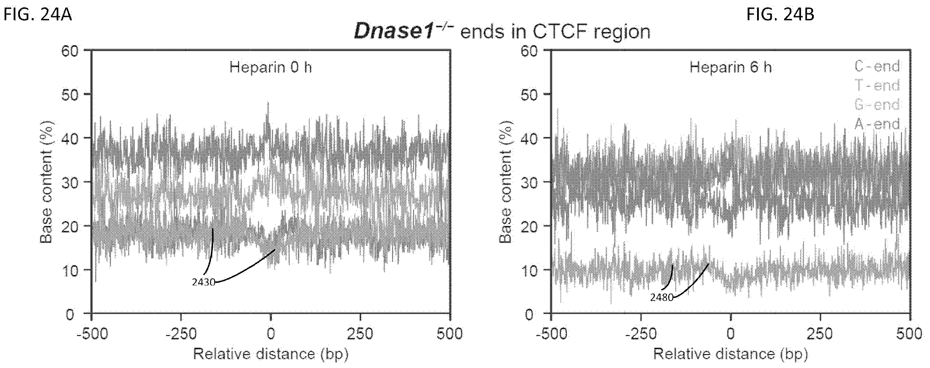

FIGS. 24A-24B show 5' end base representation in the CTCF region of Heparin 0 h and 6 h samples of *Dnase1*^{*-*/*-*} mice according to embodiments of the present disclosure. The effect seen in the periodicity and the increase in T-end fragments with WT (FIGS. 23B-23C) was absent in *Dnase1*^{*-*/*-*} mice (FIGS. 24A-24B). This can be seen in the 0 h T-end fragments 2430 and the 6 h T-end fragments 2480. Since DNASE1 is not present due the *Dnase1*^{*-*/*-*} genetic disorder in the mice, the fragments that are free from the nucleosomes as a result of the heparin incubation are not being cut by DNASE1 at T-ends. Thus, the periodicity is missing, and proportion of the 6 h T-end fragments 2480 decrease relative to the 0 h T-end fragments 2430, with a corresponding an increase in A-ends and G-ends.

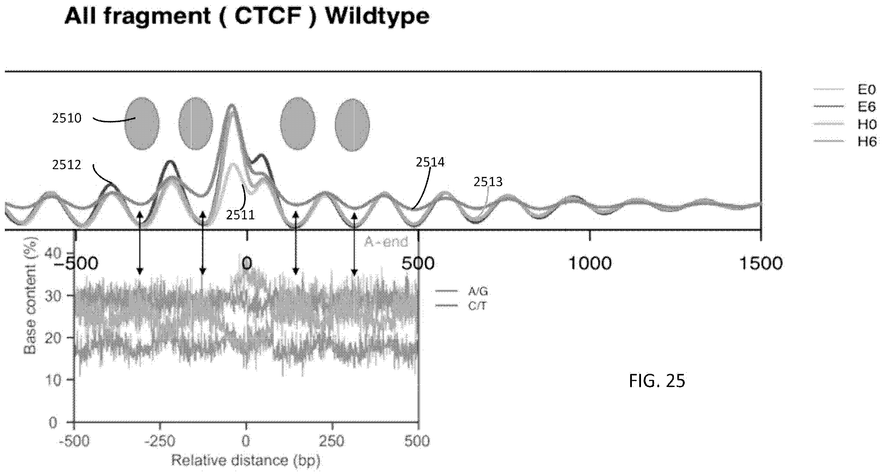

FIG. 25 shows FIGS. 23A and 23C overlaid to show that the T-end fragment peaks correspond to the intranucleosomal areas between nucleosomes 2510 with increased end density in Heparin 6 h according to embodiments of the present disclosure. Line 2511 corresponds to EDTA 0 h. Line 2512 corresponds to EDTA 6 h. Line 2513 corresponds to heparin 0 h. Line 2514 corresponds to heparin 6 h.

Since the linker areas are already cut by other enzymes (C/G/A ends) and the T-cutting enzyme is a weak competitor, the linker regions are still richer in C/G ends compared with T ends. (This internucleosomal cutting in the cell is still guided by the presence of nucleosomes). However, once the nucleosomes are in plasma and exposed to heparin, the structure gets disrupted, and then the intranucleosomal regions can be cut by the heparin-enhanced DNASE1 with a large T-end preference.

The other bases (i.e., not T) in FIG. 23C do not show a clear periodicity with incubation in heparin (or EDTA) because C-end creating DNASE1L3 dominates most of the time. DNASE1L3 can also cut intranucleosomally and so a very clear pattern is not observed. There is a chance with a higher sequencing depth one can see a periodic pattern in the other ends, especially A-ends in EDTA 6 h-there is a slight hint of it in FIG. 7B.

### V. CUTTING PREFERENCES OF NUCLEASES IN CELL AND PLASMA

The above observations allow a determination of the base end cutting preferences for DFFB, DNASE1, and DNASE1L3, as well as whether the nucleases have a prevalence for cutting within a cell or within an extracellular environment, such as plasma.

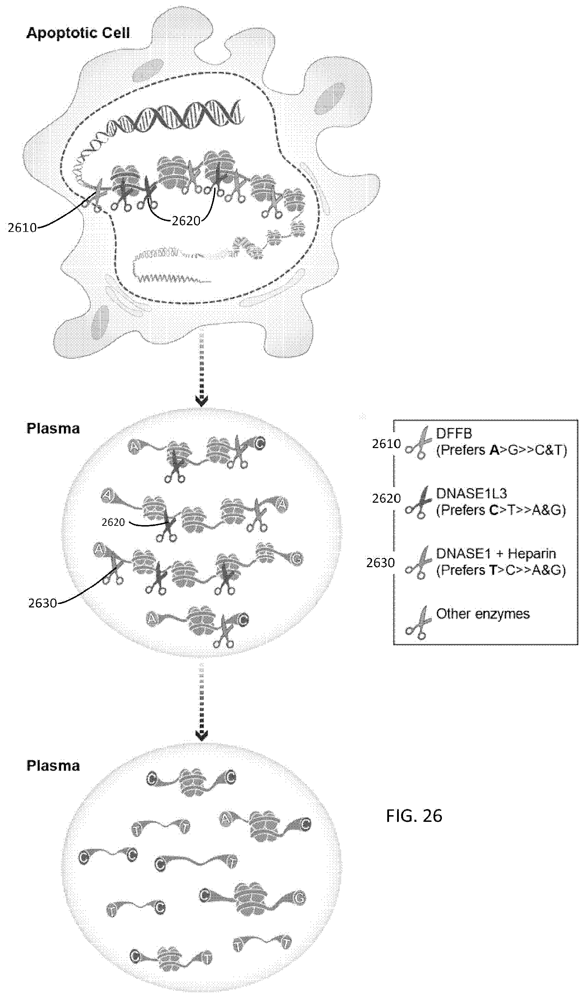

FIG. 26 shows a model of cfDNA generation and digestion with cutting preferences shown for nucleases DFFB, DNASE1, and DNASE1L3 according to embodiments of the present disclosure. DFFB generates fresh cfDNA (i.e., by cutting within the cell), where the cutting is preferred for A-ends, resulting in cfDNA that is A-end enriched. DNASE1L3 generates the predominantly C-end enriched cfDNA seen in a typical ending profile. Such cutting occurs intracellular and extracellular. DNASE1 with the help of heparin and endogenous proteases can further digest cfDNA into T-end fragments in an extracellular environment (e.g., plasma).

FIG. 26 shows an apoptotic cell with DFFB (green scissors 2610) and DNASE1L3 (blue scissors 2620) shown in the cell. The legend shows the preferential order for cutting of the three nucleases for different bases. DFFB is shown acting only in the cell. DNASE1L3 is shown as acting in the cell and also in plasma. DNASE1 (red scissors 2630) with heparin is shown acting in plasma. The resulting fragments with ending bases are shown, with different colors for the corresponding nucleases. The DNA molecules become shorter after being cut in the cell, and then even shorter after being cut in the plasma.

From this work on cfDNA fragment ends in different mouse models, we can piece together a model outlining the fragmentation process that generated cfDNA. In our analysis of the newly released cfDNA spontaneously created after incubating whole blood in EDTA, we have demonstrated that the fresh longer cfDNA are enriched for A-end fragments. In particular, A<>A, A<>G, and A<>C fragments demonstrate a strong nucleosomal periodicity at ~200 bp and 400 bp. When this same experimental model is applied to the whole blood of *Dffb*-deficient mice, no long A-end fragment enrichment is seen. Thus, we can conclude that DFFB is likely responsible for generating these A-end fragments.

This hypothesis is substantiated by literature published on the DFFB enzyme, which plays a major role in DNA fragmentation during apoptosis (Elmore, S. (2007), Toxicologic pathology 35, 495-516; Larsen, B.D. and Sørensen, C.S. (2017), The FEBS Journal 284, 1160-1170). Enzyme characterization studies have shown that DFFB creates blunt double-strand breaks in open internucleosomal DNA regions with a preference for A and G nucleotides (purines) (Larsen, B.D. and Sørensen, C.S. (2017), The FEBS Journal 284, 1160-1170; Widlak, P., and Garrard, W.T. (2005), Journal of cellular biochemistry 94, 1078-1087; Widlak, P. et al., (2000), The Journal of biological chemistry 275, 8226-8232)). This biology of blunt doublestranded cutting only at internucleosomal linker regions would explain the nucleosomal patterning in A<>A, A<>G, and A<>C fragments, e.g., as exemplified by FIG. 16B.

In this work, we have also demonstrated that typical cfDNA in plasma obtained before incubation predominantly end in C across all fragment sizes; this C-end overrepresentation is consistent in multiple different regions across the genome. Because the typical profile of cfDNA is so different from fresh cfDNA, we can infer that 1) one or more other nucleases (i.e., other than DFFB) create(s) this profile, 2) this nuclease or these nucleases dominate(s) the cleaving process in typical cfDNA, and 3) this process largely occurs after the generation of fresh A-end fragments (e.g., from DFFB).

Since this C-end predominance is lost in *Dnase1l3*-deficient mice, we believe that one nuclease responsible for creating this C-end fragment overrepresentation is DNASE1L3. While there is no existing enzymatic study that investigates the specific nucleotide cleavage preference of DNASE1L3, DNASE1L3 is known to cleave chromatin with high efficiency to almost undetectable levels without proteolytic help (Napirei, M. et al., (2009), The FEBS Journal 276, 1059-1073); Sisirak, V. et al. (2016), Cell 166, 88-101). The fairly uniform abundance of C-end fragments among all fragment sizes suggests that DNASE1L3 can cleave all DNA, even intranucleosomal DNA efficiently.

DNASE1L3 has interesting properties: it is expressed in the endoplasmic reticulum to be secreted extracellularly as one of the major serum nucleases, and it translocates to the nucleus upon cleavage of its endoplasmic reticulum-targeting motif after apoptosis is induced (Errami, Y. et al. (2013), The Journal of biological chemistry 288, 3460-3468); Napirei, M. et al., (2005), The Biochemical journal 389, 355-364)). In its role as an apoptotic intracellular endonuclease, it has been suggested that DNASE1L3 cooperates with DFFB in DNA fragmentation (Errami, Y. et al. (2013), The Journal of biological chemistry 288, 3460-3468); Koyama, R. et al., (2016), Genes to Cells 21, 1150-1163)). When comparing the fragment end profiles of fresh cfDNA (e.g., in FIG. 16B) with that of *Dnase1l3*-deficient mice (e.g., in FIG. 16A), there is a noticeable attenuation of the periodicity in A-end fragments, and especially in the A<>C fragment. We suspect this attenuation is due to the coexisting intracellular activity of DNASE1L3 and DFFB during the generation of freshly fragmented DNA from apoptosis in WT versus in *Dnase1l3-*deficient mice.

As a plasma nuclease, DNASE1L3 would help digest the DNA in circulation that had escaped phagocytosis after apoptosis. Hence, DNASE1L3 would likely exert its effect on fragmented cfDNA after intracellular fragmentation had occurred. In a two-step process, inhibiting the second step should reveal the usually transient outcome of the first step (i.e., the intracellular fragmentation). The plasma of *Dnase1l3*-deficient mice would have this second step of DNASE1L3 action inhibited and expose the cfDNA profile of the first step, the intracellular DNA fragmentation from apoptosis. This is exactly what we found, with the cfDNA fragment profile of *Dnase1l3*-deficient mice (e.g., FIG. 16A) remarkably similar to that found in freshly generated cfDNA (e.g.., FIG. 16B). Thus, DNASE1L3 digestion within the plasma would be a subsequent step that results in the typical homeostatic cfDNA.

While we previously found that the size profile of cfDNA from *Dnase1*-deficient mice did not appear to be substantially different from that of WT mice (FIG. 17A), DNASE1 is known to prefer cleaving 'naked' DNA and can only cleave chromatin with proteolytic help *in vivo* (Cheng, T.H.T. et al., (2018), Clin Chem 64, 406-408; Napirei, M. et al., (2009), The FEBS Journal 276, 1059-1073)). Using heparin to replace the function of *in vivo* proteases to enhance DNASE1 activity, we have demonstrated that DNASE1 prefers to cut DNA into T-end fragments (FIG. 18B compared to FIG. 18A). The increase in T-end fragments with heparin incubation is predominantly subnucleosomally-sized (50-150 bp), suggesting that DNASE1 has a role in generating short < 150 bp fragments (FIG. 18A). Knowing that DNASE1 prefers to cleave naked DNA into T-end fragments, we can infer from the typical cfDNA profile that the T-end fragment peaks in 50-150 bp and 250-300 bp range may be mostly naked.

The use of heparin incubation and end analysis have also provided a unique insight into the origin of the 10 bp periodicity. Since every fragment type demonstrates a 10 bp periodicity (FIG. 21A), we show that no one specific nuclease is completely responsible for the 10 bp periodicity in short fragments. Instead, we demonstrate that for all fragment types, the 10 bp periodicity is abolished when heparin is used (FIG. 21B). In addition to enhancing DNASE1 activity, heparin disrupts the nucleosomal structure (Villeponteau, B. (1992), The Biochemical journal 288 (Pt 3), 953-958), as shown in FIG. 23A. While many have postulated that the 10 bp periodicity originates from the cutting of DNA within an intact nucleosomal structure, we believe that this work provides supportive evidence, showing that no 10 bp periodicity occurs in the presence of a disrupted nucleosome.

Recently, Watanabe *et al.* induced *in vivo* hepatocyte necrosis and apoptosis with acetaminophen overdose and anti-Fas antibody treatments in mice deficient in *Dnase1L3* and *Dffb* (Watanabe, T. et al., (2019), Biochemical and biophysical research communications 516, 790-795). While Watanabe *et al.* claims to have shown that cfDNA is generated by DNASE1L3 and DFFB, their data only shows that serum cfDNA does not appear to increase after hepatocyte injury in *Dnase1l3-* and *Dffb*-double knockout mice. Even then, the degree of hepatocyte injury from their methods is hugely variable even in wildtype with surprisingly low correlation with cfDNA amount in their apoptotic anti-Fas antibody experiments. In addition to these inconsistencies that gives uncertainty to the degree of apoptosis induced in their knockout mice, they have none of the detail on fragment ends offered in this study.

In this study, we have demonstrated that the typical cfDNA fragment might be created in two major steps: 1) intracellular DNA fragmentation by DFFB, intracellular DNASE1L3, and other apoptotic nucleases, and 2) extracellular DNA fragmentation by serum DNASE1L3. Then, likely with *in vivo* proteolysis, DNASE1 can further degrade cfDNA into short T-end fragments (compare difference T-end graphs between FIG. 18A and 18B). We believe that this first model has included a number of key nucleases involved in cfDNA generation, but the model can be further refined in the future. For example, other potential apoptotic nucleases include endonuclease G, AIF, topoisomerase II, and cyclophilins, with probably more to be discovered (Nagata, S. (2018), Annual review of immunology 36, 489-517; Samejima, K. and Earnshaw, W.C. (2005), Nature Reviews: Molecular Cell Biology 6, 677-688; Yang, W. (2011), Quarterly reviews of biophysics 44, 1-93). Further studies into these nucleases with double knockout models would further refine this model and may reveal a nuclease with G-end preference. In this work, we have definitively linked the action of distinct nucleases to the cfDNA fragment end profile.

With this link between nuclease biology and cfDNA physiology established, there are many important and practical implications to the field of cfDNA. Firstly, aberrations in nuclease biology with pathological consequences may be reflected in abnormal cfDNA profiles (Al-Mayouf et al. (2011), Nat Genet 43, 1186-1188; Jimenez-Alcazar, M. et al. (2017), Science (New York, NY) 358, 1202-1206; Ozcakar, Z.B. et al., (2013), Arthritis Rheum 65, 2183-2189)). Secondly, plasma end motif analysis is a powerful approach for investigating cfDNA biology and may have diagnostic applications. And lastly, the pre-analytical variables such as anticoagulant type and time delay in blood separation are vital confounders to bear in mind when mining cfDNA for epigenetic and genetic information. Example applications for such cfDNA profiling are described below.

Additionally, even though the data is provided for mice, such biological functionality is common to all organisms that have blood or other cell-free samples.

### VI. METHODS FOR DETECTION OF GENETIC DISORDERS OF NUCLEASES

As described above, various techniques can be used to detect genetic disorders, e.g., associated with a nuclease. The genetic disorders can relate to a mutation (e.g., a deletion) of a nuclease corresponding to a particular gene. Such a mutation can cause the nuclease to not exist or to function in an irregular manner. A normal/reference cfDNA profile (e.g., by fragment ends and/or by size) can be determined for when the genetic disorder does not exist, and a comparison can be made for a new sample. The normal/reference cfDNA profiles can be determined from other subjects or for the same subject, but with different conditions (e.g., sample taken at an earlier time or with a different amount of incubation). Examples of such methods are described in the following flowcharts. Techniques described for one flowchart are applicable to other flowcharts, and are not repeated for the sake of being concise.

### A. Detecting genetic disorder using incubation over time

Different amounts of incubation of a sample can result in different cfDNA profiles depending on whether the genetic disorder exists. As a particular cfDNA profile behavior can depend on whether a particular nuclease expressed and functioning properly, a change in such behavior from normal can indicate the genetic disorder exists.

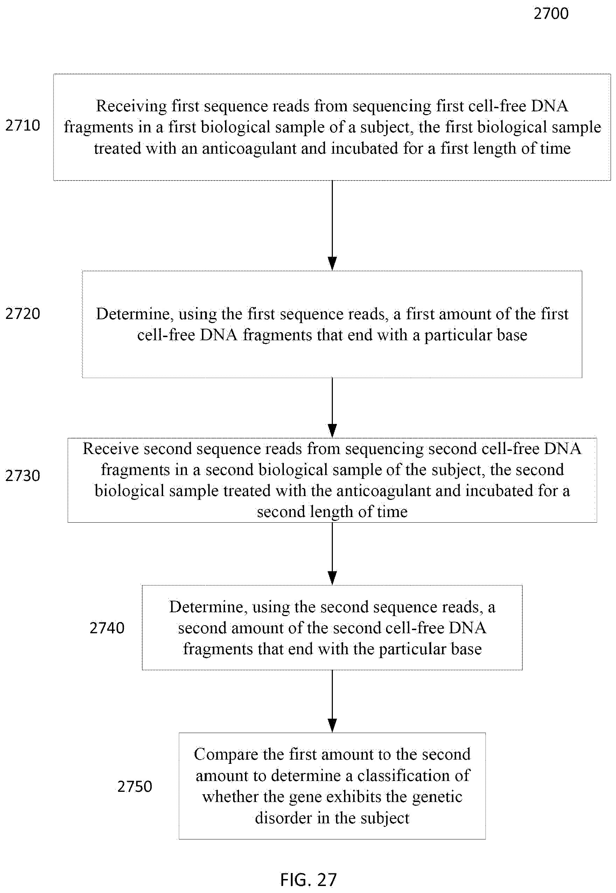

FIG. 27 shows a flowchart illustrating a method 2700 for detecting a genetic disorder for a gene associated with a nuclease using biological samples including cell-free DNA according to embodiments of the present disclosure. Method 2700 and others method herein can be performed entirely or partially with a computer system, including being controlled by a computer system. As examples, a gene can be associated with a nuclease by coding for the nuclease, having epigenetic markers for its transcription, having its RNA transcripts present, having variably spliced RNA, or having its RNA variably translated. The genetic disorder may be in only certain tissue (e.g., tumor tissue). Accordingly, the detection of the genetic disorder may be used to determine a level of cancer.

At block 2710, first sequence reads are obtained from sequencing first cell-free DNA fragments in a first biological sample of a subject are received. Example biological samples are provided herein, e.g., blood, plasma, serum, urine, and saliva. The sequencing may be performed in various ways, e.g., as described herein. Example sequencing techniques include massively parallel sequencing or next-generation sequencing, using single molecule sequencing, and/or using double- or single-stranded DNA sequencing library preparation protocols. The skilled person will appreciate the variety of sequencing techniques that may be used. As part of the sequencing, it is possible that some of the resulting sequence reads may correspond to cellular nucleic acids.

The sequencing may be targeted sequencing as described herein. For example, a biological sample can be enriched for DNA fragments from a particular region, such as CTCF regions, TSS regions, Dnase hypersensitivity sites, or Pol II regions. The enriching can include using capture probes that bind to a portion of, or an entire genome, e.g., as defined by a reference genome. As another example, the enriching can use primers to amplify (e.g., via PCR, rolling circle amplification, or multiple displacement amplification (MDA) certain regions of the genome.

The first biological sample can be treated with an anticoagulant and incubated for a first length of time. The incubation can be at a certain temperature or higher, e.g., above 5°, 10°, 15°, 20°, 25°, or 30° Celsius. Storage at lower temperatures may not count as part of the incubation time. The first length of time can be zero. In other implementations, the first biological sample is incubated for the first length of time without being treated with an anticoagulant. As examples, the anticoagulant can be EDTA or heparin. The EDTA can help to inhibit plasma nucleases (e.g., DNASE1 and DNASE1L3) to preserve cfDNA for analysis.

At block 2720, the first sequence reads are used to determine a first amount of the first cell-free DNA fragments that end with a particular base. The particular base can be determined by identifying an end of the first sequence read corresponding to an end of the fragment, which for paired end sequence can be determined using an orientation of the of the read (e.g., the first base sequenced). A particular fragment end can be used, e.g., the 5' end or the 3' end. The first amount can be determined for a particular end motif that includes the particular base. Thus, the first amount can be for a particular ending sequence that may be for more than one base. The first amount is an example of a parameter value.

In some embodiments, the first amount can be for DNA fragments that have a first end motif (e.g., a first base) at one end of the fragment and that have a second end motif (e.g., a second base) at the other end of the fragment.

In some implementations, the first cell-free DNA fragments are filtered before determining the first amount, e.g., only fragments from a certain region (e.g., CTCF) may be used to determine the first amount. The first sequence reads may be aligned to a reference genome. Then, a first set of sequence reads can be identified that end at a particular location or at a specified distance from the particular location in the reference genome, where the particular location corresponds to a particular coordinate or a genomic position with a specified property in the reference genome. The first amount can then be determined as an amount of the first set of sequence reads that end with the particular base. The genomic position can be a center of a CTCF region. As other examples, genomic positions can be associated with open chromatin regions, Pol II regions, TSS regions, and/or hypersensitive sites for a particular enzyme (e.g., a particular DNase).

At block 2730, second sequence reads obtained from sequencing second cell-free DNA fragments in a second biological sample of the subject are received. The second biological sample can be treated with the anticoagulant and incubated for a second length of time that is greater than the first length of time. In other implementations, the second biological sample can be incubated without being treated by the anticoagulant. The length of time can include a temperature factor, e.g., a higher temperature can act as a weighting factor multiplied by a time unit to obtain the length of time. In this manner, a greater/same amount of cell death can occur in a sample/shorter amount of time due to the incubation at a higher temperature.

At block 2740, the second sequence reads are used to determine a second amount of the second cell-free DNA fragments that end with the particular base. In some implementations, the first amount and the second amount are of cell-free DNA fragments having both ends with the particular base. The second amount can also be determined for a particular end motif that includes the particular base. Thus, the second amount can be for a particular ending sequence that may be for more than one base. In some embodiments, the first amount can be for DNA fragments that have a first end motif (e.g., a first base) at one end of the fragment and that have a second end motif (e.g., a second base) at the other end of the fragment.

The amounts can be determined as a percentage, also referred to herein as a base content or a frequency. In other implementations, the amounts can be raw amounts that are not directly normalized using (e.g., dividing by) a measured amount of DNA fragments (e.g., as measured by sequence reads). Instead, indirect normalization can occur by using a same size sample or by sequencing a same number of DNA fragments for the two samples.

The amounts can relate to sizes of the DNA fragments. For instance, the first sequence reads can be used to determine first sizes of the first cell-free DNA fragments that end with the particular base or larger end motif. The first amount can be determined using a first set of the first cell-free DNA fragments having a particular size. The second sequence reads can be used to determine second sizes of the second cell-free DNA fragments that end with the particular base or larger end motif. The second amount can be determined using a second set of the second cell-free DNA fragments having the particular size. The particular size can be a size range. Example uses of size can be found in FIG. 10A relative to FIG. 10B as well as other similar figures.

At block 2750, the first amount is compared to the second amount to determine a classification of whether the gene exhibits the genetic disorder in the subject. In some implementations, comparing the first amount to the second amount includes determining whether the first amount differs from the second amount by at least a threshold amount, and can include which amount is larger than the other when there is a statistically significant difference or other separation value. Accordingly, the classification can be that the genetic disorder exists when the first amount is within a threshold of the second amount.

In some embodiments, the comparison of the amounts can include determining a separation value between the first amount and the second amount. The separation value can be compared to a reference value (e.g., a cutoff) to determine the classification. The reference value can be a calibration value determined using calibration (reference) samples, which have known classifications and can be analyzed collectively to determine a reference value or calibration function (e.g., when the classifications are continuous variables). The first amount and second amounts are examples of a parameter value that can be compared to a reference/calibration value. Such techniques can be used for all methods herein, and further details are provided in other sections.

The classification can be a level or severity of the disorder, e.g., from whether a coding gene for the nuclease is missing in both chromosomes, in only one chromosome, are missing in only certain tissue, or the mutation reduces expression but does not eliminate the existence of the nuclease. Such a partial reduction in the expression of the nuclease can occur when the mutation (e.g., a deletion) is only in certain tissue or when the mutation is within a supporting region, e.g., in a non-coding region such as miRNA that affects the level of expression of the nuclease. The different levels or severity of the genetic disorder, as a result of differing amounts of difference relative to the reference level. Multiple reference levels can be used to determine the difference classifications.

In some examples, when the first amount is within a threshold of the second amount, the classification can be that the genetic disorder exists, e.g., as in FIG. 10B. As shown in FIG. 10B, there is not a significant difference in the amount of fragments for any of the ending bases, but there is a significant difference for all of the bases for the WT shown in FIG. 10A. In various implementations, the amounts can be aggregated for all sizes or for a particular set of sizes, or differences at each size can be aggregated. For example, a threshold amount for A-ended fragments at 200 bases can be about 5% as the difference for the WT is around 10% and the difference for Dffb^{-/-} is within about a percent. An example lack of change in an amount of certain DNA fragments with specified end motif(s) can also be found in the comparison of FIG. 8A to FIG. 11C, illustrating that both ends of a fragment can be used. Another example lack of change in an amount of certain DNA fragments with specified end motif(s) can also be found in the comparison of FIGS. 12A-12D and -13 to FIGS. 4B and 4C, illustrating that analysis can be of DNA fragments (sequence reads) that in a particular type of region, and even at a particular position within the particular type of region.

In other examples, when the second amount is less than the first amount by at least a threshold (e.g., for T-ends), the classification can be that the genetic disorder exists, e.g., as in FIGS. 24A-24B, contrasted where WT has second amount greater for T-ends (FIGS. 23B and 23C). In other examples, the classification can be that the genetic disorder exists when the second amount is greater (e.g., for A-ends), e.g., as in FIGS. 24A-24B, contrasted where the WT has about the same for the first and second amounts, e.g., as in FIGS. 23A and 23B.

In other examples, both the WT and the mutation can cause a same change (e.g., an increase or a decrease) of DNA fragments with a particular end motif, but the amount of change can be different. For example, FIGS. 16A and 16B show a larger increase for the WT for A<>G fragments at 20 bp than for A<>G fragments for *Dnase1l3*^{*-*/}*⁻.*

The type of genetic disorder being tested can provide the type of criteria used for determining whether the disorder exists, as the cfDNA behavior will be different.

As an example, the genetic disorder can include a deletion of the gene. As examples, the genes can be DFFB, DNASE1L3, or DNASE1. The nuclease can be one that cuts intracellular DNA, e.g., DFFB or DNASE1L3. The nuclease can be one that cuts extracellular DNA, e.g., DNASE1 or DNASE1L3.

### B. Detecting genetic disorder using reference value

As described above, a difference or other separation value (e.g., whether small or large) in a particular base content between samples with different incubations can be used to classify a genetic disorder for a gene associated with a nuclease. Alternatively, the measured amount of a particular base can be compared to a reference value. Such a reference value can correspond to the amount of the particular base measured in a healthy subject.

For instance, a comparison of FIG. 12A (DFFB deficiency) in EDTA 0 h to FIG. 2C (WT) in EDTA 0 h shows a decrease in A-end content in the *Dffb-deficient* mice for random regions. Thus, a comparison of a measured A-end content in a *Dffb-deficient* can be compared to a reference value for WT, where the disorder is determined when the measured amount is lower than the reference value by a statistically significant amount. Such a difference exists without any incubation. Similar differences exist for CTCF regions (FIG. 12B vs. FIG. 2E), for TSS regions (FIG. 13A vs. FIG. 3B), and for Pol II regions (FIG. 13C vs. FIG. 3D). Decreases in G-end content is also seen as a result of the DFFB deficiency.

Another example can be seen in FIG. 15. The DNASE1L3 deficiency results in decreases in T-end fragments and C-end fragments, and results in increases in A-end fragments and G-end fragments. One implementation can use a reference T-end content for the WT (e.g., for all sizes or just a specific size range) and determine whether the measured T-end content is statistically lower, which would provide a classification of a disorder for DNASE1L3. FIG. 16A provides further examples of such differences; in this case, examples for when the amount is for both ends. FIG. 14B provides another example.

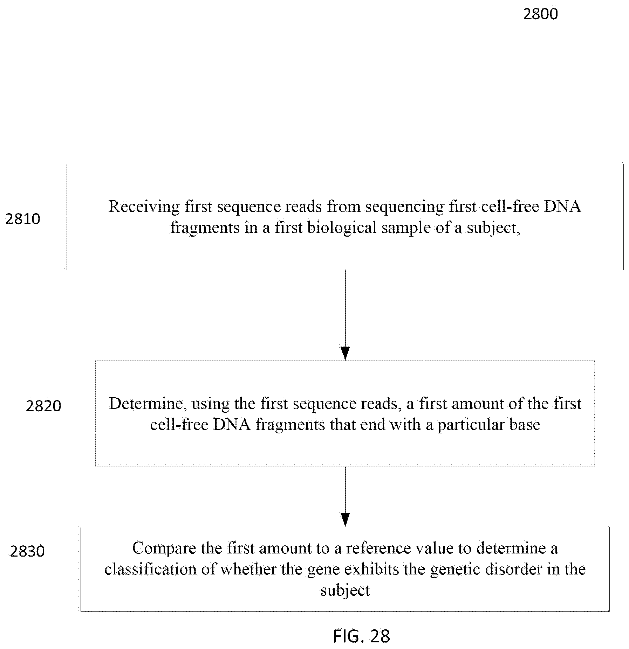

FIG. 28 shows a flowchart illustrating a method 2800 for detecting a genetic disorder for a gene associated with a nuclease using a biological sample including cell-free DNA according to embodiments of the present disclosure. Similar techniques as used for method 2700 may be used in method 2800. As examples, the gene is DNASE1L3, DFFB, or DNASE1.

At block 2810, first sequence reads obtained from sequencing first cell-free DNA fragments in a first biological sample of a subject are received. The sequencing may be performed in various ways, e.g., as described herein. The first biological sample can be treated with an anticoagulant and incubated for at least a specified amount of time, e.g., as described for FIG. 18B relative to FIG. 18A. Similar techniques as used for block 2710 may be used in block 2810.

At block 2820, the first sequence reads are used to determine a first amount of the first cell-free DNA fragments that end with a particular base. Similar techniques as used for block 2720 may be used in block 2820. For example, certain sizes of sequence reads can be used for determining the amount that end with a particular base. As another example, the amount can be determined for a particular end motif that includes the particular base.

At block 2830, the first amount is compared to a reference value to determine a classification of whether the gene exhibits the genetic disorder in the subject. In various embodiments, comparing the first amount to the second amount can include: (1) determining whether the first amount differs from the reference value by at least a threshold amount or the difference is less than the threshold amount; (2) determining whether the first amount is less than the reference value by at least a threshold amount; or (3) determining whether the first amount is greater than the reference value by at least a threshold amount. The first amount is an example of a parameter value and the reference value can be a calibration value or determined from calibration values of calibration samples. Further details are provided for other methods but equally apply to method 2800.

### C. Detecting genetic disorder using size

As described above, fragments of a certain size can be used to determine the amount of sequence reads with the particular base. In some implementations, size may be used along without a determination of a base content or other measured amount of fragments that end in a particular base. Such an example is shown in FIGS. 17A and 17B, which includes incubation with an anticoagulant (e.g., heparin). The subjects with the genetic disorder (various levels of DNASE1 deficiencies in this case) have different frequencies of DNA fragments at certain sizes. For example, from 50-150 bp, the WT (reference value) has higher frequencies than the *Dnase1*^{*+*/*-*} subject, which in turn has higher frequencies than the *Dnase1*^{*-*/*-*} subject. The opposite relationship exists for frequencies of DNA fragments in the size range 150-230 bp.

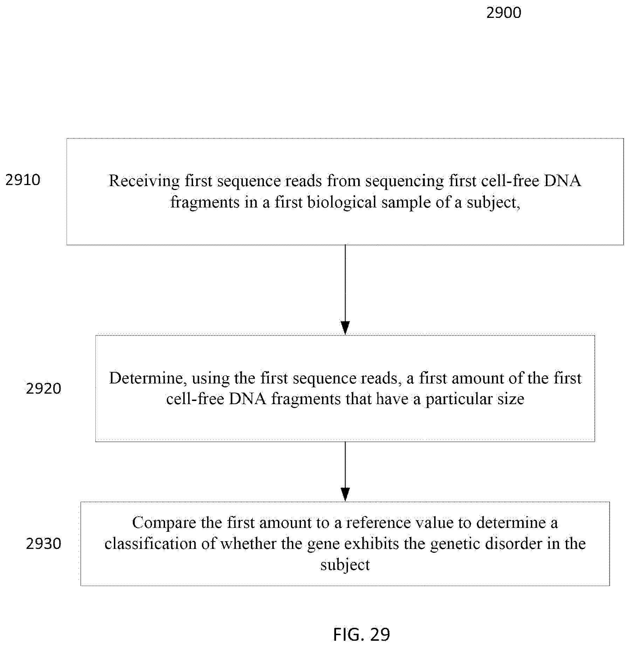

FIG. 29 shows a flowchart illustrating a method 2900 for detecting a genetic disorder for a gene associated with a nuclease using a biological sample including cell-free DNA according to embodiments of the present disclosure. Similar techniques as used for method 2700 and 2800 may be used in method 2900.

At block 2910, first sequence reads obtained from sequencing first cell-free DNA fragments in a first biological sample of a subject are received. The biological sample can be treated with an anticoagulant and incubated for at least a specified amount of time. As example, the anticoagulant can be heparin.

At block 2920, the first sequence reads can be used to determine a first amount of the first cell-free DNA fragments that have a particular size, e.g., as described in FIGS. 17A and 17B. The particular size can be a range. For example, a size range can be greater than or less than a size cutoff, e.g., 100 bp, 150 bp, or 200 bp. As other examples, the size range can be specified by a minimum and a maximum size, e.g., 50-80, 50-100, 50-150, 100-150, 100-200, 150-200, 150-230, 200-300, or 300-400 bases, as well as other ranges. The width of the size range can vary, e.g., to be 50, 100, 150, or 200 bases. As examples, the first amount can be a raw count or be normalized, e.g., as a frequency using a total number of sequence reads or DNA fragments analyzed.

At block 2930, the first amount is compared to a reference value to determine a classification of whether the gene exhibits the genetic disorder in the subject. A separation value can be determined between the first amounts and the reference value. In one example, the gene is DNASE1. The classifications of method 2900 can be the same as described for other methods, e.g., being of different levels or severity of the genetic disorder, as a result of differing amounts of difference relative to the reference level. Multiple reference levels can be used to determine the difference classifications.

The first amount is an example of a parameter value. The reference value can be part of a calibration data point that is determined from one or more calibration samples having known efficacy for a given measurement of the parameter (e.g., for a given calibration value). The known efficacy can be determined using blood clotting tests, as described later.

In various embodiments of methods 2700-2900, wherein the reference value can be determined from one or more reference samples that do not have the genetic disorder and/or determined from one or more reference samples that have the genetic disorder.

### VII. DETERMINING EFFICACY OF DOSAGE OF ANTICOAGULANT

Some people are treated with anticoagulants, e.g., for deep venal thrombosis (DVT), which results in clots in some veins. One treatment is heparin. Some embodiments can determine whether the anticoagulant is working. As examples, the effect of heparin can be seen with an increase in cfDNA quantity and/or an increase in DNASE1 activity and/or an increase in short fragments. This can be seen in the size profile or the shift in median size or the increase in fragments of a particular size, e.g., less than 150 bp.

### A. Determining efficacy using amount of a particular base at fragment ends

In some embodiments, the efficacy can be determined using an amount (e.g., base content) of a particular base at fragment ends.

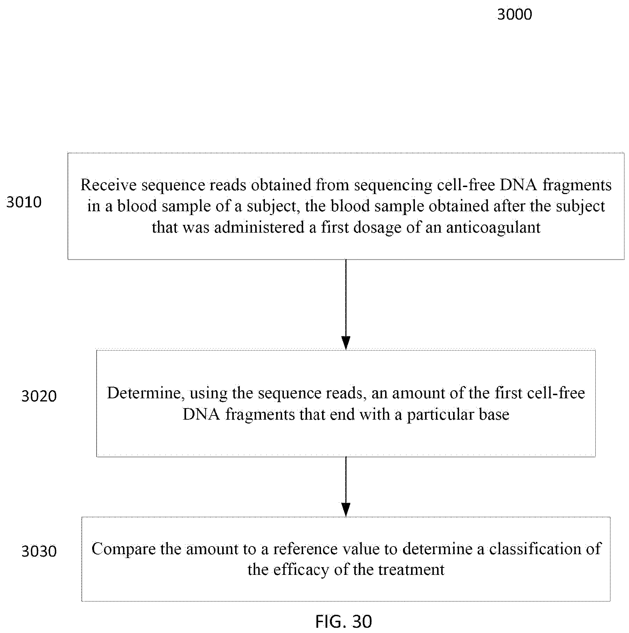

FIG. 30 shows a flowchart illustrating a method 3000 for determining an efficacy of a treatment of a subject having blood disorder according to embodiments of the present disclosure. Similar techniques as used for other methods may be used in method 3000.

At block 3010, sequence reads obtained from sequencing cell-free DNA fragments in a blood sample of the subject are received. The blood sample is obtained after the subject that was administered a first dosage of an anticoagulant. The anticoagulant can be heparin. Method 3000 can include administering the first dosage of the anticoagulant to the subject.

Prior to receiving the sequence reads, the blood sample can be obtained from the subject, and a sequencing of the cell-free DNA fragments in the blood sample can be performed to obtain the sequence reads.

At block 3020, the sequence reads can be used to determine an amount of the cell-free DNA fragments that end with a particular base. As examples, the amount can be at a particular size (e.g., as shown in FIG. 18B) or at (or adjacent to) particular coordinates or genomic position having a specified property, e.g., as shown in FIGS. 7B-7D. The effect of an anticoagulant on the amount of a particular base at an end of the fragments can be seen in FIG. 18A. For example, an increase in the A-end fragments would be expected in total and for certain size ranges. As with other methods, the particular base may be part of a larger end motif, e.g., a 2-mer, 3-mer, etc. Further, the particular base can be required to be on both ends of a DNA fragment, or a particular pair of different end motifs can be used to select a particular set of DNA fragments.

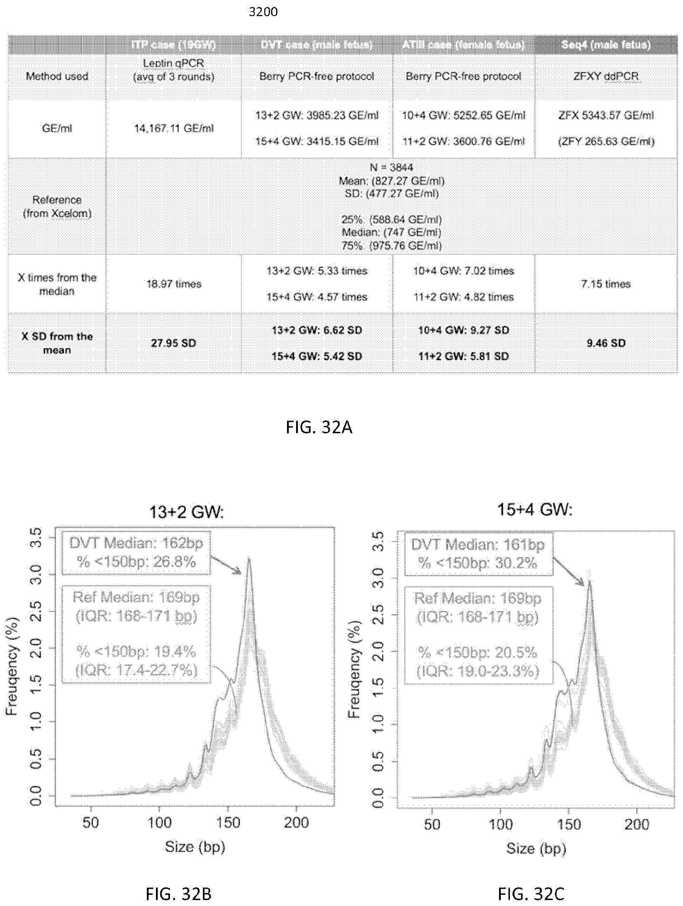

Besides an amount of the cell-free DNA fragments that end with a particular base, a total amount of cfDNA (i.e., for any ends) can be determined and used, e.g., as shown later in FIG. 32A. The measured amount in this method and other methods can be normalized, e.g., using a property of the sample (e.g., volume or mass of the sample) or using another amount of cell-free DNA fragments or sequence reads satisfying specified criteria (e.g., a total amount of DNA fragment in the sample or a number of fragments with a different end motif).

At block 3030, the amount can be compared to a reference value to determine a classification of the efficacy of the treatment. The reference value can be determined in various ways, e.g., as described herein. For instance, an expected amount can be determined for patients that respond as desired. The amount of difference between the amount and the reference value can provide the classification. If the difference is sufficient small (e.g., less than a cutoff), then the first dosage can be classified as effective. If the difference is greater than the cutoff, then the first dosage can be determined as not effective. There may be different levels of ineffective dosage, e.g., intermediate or large inefficacy, which may be determined by using one or more additional cutoff values.

If the amount does not match the reference value (e.g., within a specified range of the reference value), a second dosage of the anticoagulant can be administered to the subject based on the comparison, the second dosage being greater than the first dosage. In other examples, the second dosage can be less than the first dosage, e.g., if the amount overshoots the reference value.

The amount is an example of a parameter value. The reference value can be part of a calibration data point that is determined from one or more calibration samples having known efficacy for a given measurement of the parameter (e.g., for a given calibration value). The known efficacy can be determined using blood clotting tests, as described later. Further details are provided for other methods and sections but equally apply to method 3000.

As an example, the reference value can correspond to a measurement previously performed in the subject before administering the anticoagulant. The change in the amount from the previous measurement can indicate an efficacy of the dosage of the anticoagulant. In another implementation, the reference value can correspond to the amount measured in a healthy subject. An efficacious dosage can be one that brings the amount to within a threshold of the reference value for the healthy subject. In yet another implementation, the reference value can correspond to the amount measured in a subject that has the blood disorder (e.g., as may be previously measured in the subject before administering the anticoagulant or measured in another subject who has the blood disorder).

### B. Determining efficacy using size of fragments

In some embodiments, the efficacy can be determined using the sizes of fragment ends.

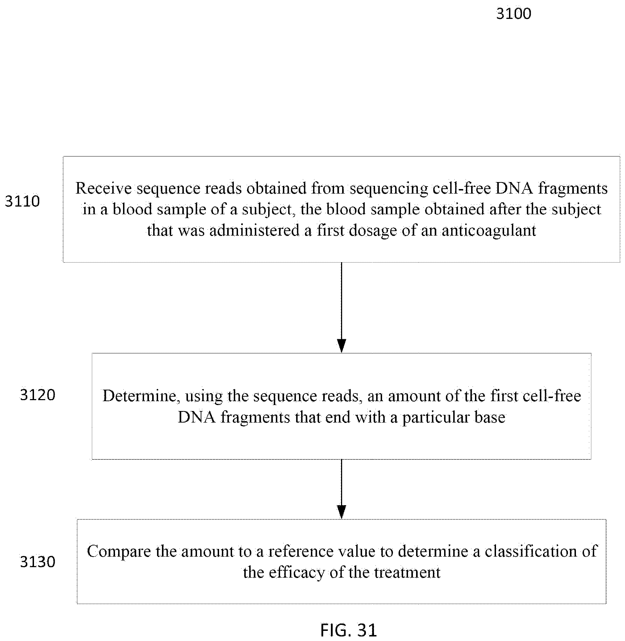

FIG. 31 shows a flowchart illustrating a method 3100 for determining an efficacy of a treatment of a subject having blood disorder according to embodiments of the present disclosure. Similar techniques as used for other methods may be used in method 3100.

At block 3110, sequence reads obtained from sequencing cell-free DNA fragments in a blood sample of the subject are received. The blood sample is obtained after the subject that was administered a first dosage of an anticoagulant. The anticoagulant can be heparin. Method 3100 can include administering the first dosage of the anticoagulant to the subject.

At block 3120, the sequence reads can be used to determine an amount of the cell-free DNA fragments that have a particular size. Block 3120 may be performed in a similar manner as block 1120 in method 1100. The effect on the size can be as illustrated in FIGS. 17A and 17B.

At block 3130, the amount can be compared to a reference value to determine a classification of the efficacy of the treatment. The reference value can be determine in a similar manner as for method 3000. The first amount is an example of a parameter value and the reference value can be a calibration value or determined from calibration values of calibration samples. Further details are provided for other methods but equally apply to method 3100.

If the amount does not match the reference value (e.g., within a specified range of the reference value), a second dosage of the anticoagulant can be administered to the subject based on the comparison, the second dosage being greater than the first dosage. In other examples, the second dosage can be less than the first dosage, e.g., if the amount overshoots the reference value.

### C. Results

FIG. 32A shows a table 3200 for four cases treated with heparin according to embodiments of the present disclosure. Each column corresponds to a different patient. The first row identifies the hemostatic disorders of each of the four patient. ITP is immune thrombocytopenic purpura: immune-mediated destruction of platelets leading to a bleeding tendency. DVT is deep vein thrombosis. ATIII is antithrombin III deficiency: without antithrombin III in the coagulation cascade, there is no inhibition of thrombin, Factor IXa, Factor Xa, etc. leading to a thrombotic (clot forming) tendency (i.e., DVT). Seq4 has unknown clinical case details other than being given heparin.

The second row lists the method using to determine the concentration of cfDNA in the plasma samples. The third row shows the concentration of cell-free DNA in GE/ml. The fourth row shows the reference value determined from 3,844 reference samples that are not treated with an anticoagulant and that do not have a blood disorder. The fifth and sixth row shows the difference in the measured value in the second row to the reference values in the third row. As one can see, there is a significant increase. The last row shows significant deviations from the mean for cell-free DNA quantity, which shows that the dosage of heparin is affecting the amount of cell-free DNA resulting in a significant increase.

As shown in rows five and six, the amount of cell-free DNA increases significantly as the heparin works to prevent coagulation. Thus, the total amount of DNA can be used to determine an efficacy of dosage. As described below, the absolute or fold decrease in the cfDNA can be determined and compared to a target to determine the efficacy of a current dose and/or to determine how much the dosage should increase or decrease. If the parameter is too high, the dosage can be decrease to meet the target.

FIGS. 32B-32C show data for two samples taken at different times for the DVT patient who as treated with heparin according to embodiments of the present disclosure. The different times are specified by week and day of the pregnancy. FIGS. 32B-32C shows plots of frequency vs. size relative for a subject to a reference. As can be seen in FIG. FIGS. 32B-32C, the subjects' size distributions shifted to smaller size, as indicating an effect of heparin, consistent with FIG. 17A. Other embodiments can use other anticoagulants, such as Warfarin or factor Xa inhibitor (e.g., for atrial fibrillation).

Blood clotting tests can be used as calibration data for each subject with a particular dosage of the anticoagulant to identify what change in amount or size correlates to an effective change in the amount/size. For example, correlation studies done in a group of patients (e.g., DVT patients) who are given anticoagulants can determine the fold change in total amount of cfDNA, change in amount having a particular end motif, or change in size profile that may result in the optimal speed of clearance of a DVT clot. The measured change (absolute or fold) can correspond to a calibration value that corresponds to the target or measure property (e.g., optimal speed for clearance). This value or range of values for amount/size can be a target for treatment for monitoring therapy. Blood of a subject may be allowed to undergo clotting in vitro, and then anticoagulants can be titrated in vitro for the dose in which the anticoagulant is effective. The cfDNA amount/size can be measured in the sample after the clot is dissolved, and these values or a range of values can be the treatment target for the subject. For example, a clotting test can identify that the subject is clotting at the proper amount, and the corresponding amount/size can be used as the reference (calibration) value, which may be used to classify the efficacy of a current dosage.

The dosage can vary per person in order to achieve the effective change, which is why such techniques can be advantageous as they allow measurement of the resulting changes. Such a change in the size or amount of fragments can measures the actual effects within the body, as opposed to just expecting every person to react in the same way to the same dose.

### VIII. MONITORING ACTIVITY OF A NUCLEASE

Some embodiments can be used to monitor the activity of a nuclease, e.g., DFFB, DNASE1, and DNASE1L3. Such activity can be from internal nucleases (i.e., as a natural process of the body) and/or from the result of adding a nuclease, e.g., DNASE1. Such monitoring can be used to determine a change in a genetic disorder for the efficacy of a treatment. For example, DNASE1 can be used to treat a subject. An effect of the treatment can be measured by analyzing the T-end fragment percentage or size. In some embodiments, DNASE1 (e.g., exogenously added) can be used to treat auto-immune conditions, such as SLE. Depending on the determination of the activity, the dosage of treatment of the nuclease can be changed.

The determination of abnormal nuclease activity (e.g., above or below a reference value corresponding to normal/healthy values) can indicate a level of pathology alone or in combination with other factors. The pathology can be cancer.

### A. Effect of adding DNASE1 to samples

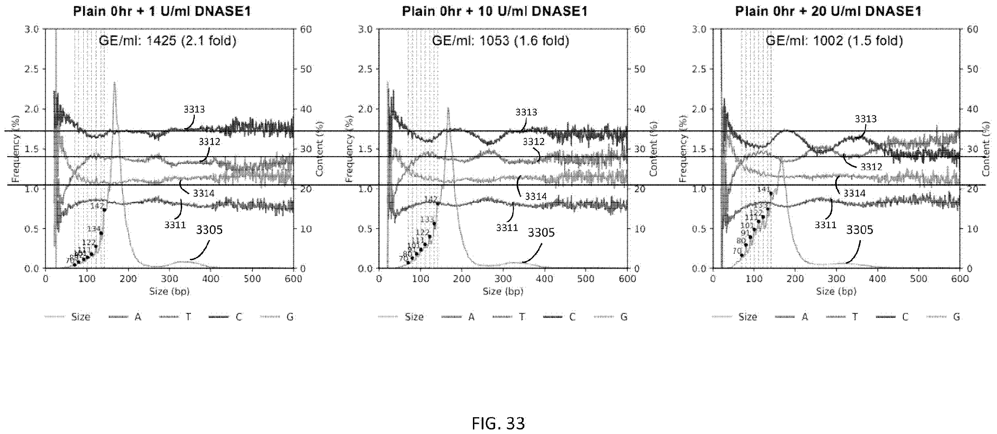

FIG. 33 shows plots of content percentage for the different ends vs. size of the fragment for different dosages of DNASE1 according to embodiments of the present disclosure. Base content percentage is on the right vertical axis, and the horizontal axis is for size per bp. Green line 3311 corresponds to frequency of A-end fragments. Red line 3312 corresponds to frequency of T-end fragments. Blue line 3313 corresponds to frequency of C-end fragments. Grey line 3314 corresponds to frequency of G-end fragments. DNASE1 was administered in vitro.

FIG. 33 also shows a frequency plot for the size of all fragments according to embodiments of the present disclosure. The frequency is on the left vertical axis. The yellow line 3305 corresponds to the size of all fragments. The concentration of GE/ml is provided for each sample. The plots are for three different doses 1 U/ml (unit per ml), 10 U/ml, and 20 U/ml of administering DNASE1, which were added in vitro to the sample after obtaining the plasma from the subject.

The T-end fragments 3312 increase with DNASE1 dose. As shown, the red line 3312 increases from left to right with the higher dosage. This dependency of base content (total or per size) on nuclease activity can allow a classification of a test sample as having a particular activity. The total amount of T-end could be used or a particular amount at a particular size or size range. Any of the features described elsewhere in this disclosure and that depend on nuclease activity can be used (e.g., content for other bases at certain sizes or across all fragment sizes) to measure nuclease activity, e.g., using reference values determined in other samples having a known classification.

A size profile 3305 can also reflect DNASE1 activity. For example, an increase in smaller DNA fragments can show an increase in DNASE1 activity. The number of smaller DNA fragments increases with higher dosage of DNASE1, as can be seen in the progression from left to right in the figure, with more small DNA fragments with the highest dose of 20 U/ml.

Any of the data from any of these plots can be used as a reference value or compared to a reference value. For example, the frequency of DNA fragments at a particular size range (including a specific size) can be determined for each of the doses. Then, a measurement for a new sample can be compared to each of these reference values to determine a relative amount of activity in the test sample. Such a classification of nuclease activity can be qualitative (e.g., low, medium, or high) or quantitative (a particular numerical value). Since these samples correspond to a known activity, they can act as calibration values for determining an activity in the test sample. If desired, interpolation or regression can be used to estimate a particular activity for the measured value in the test sample.

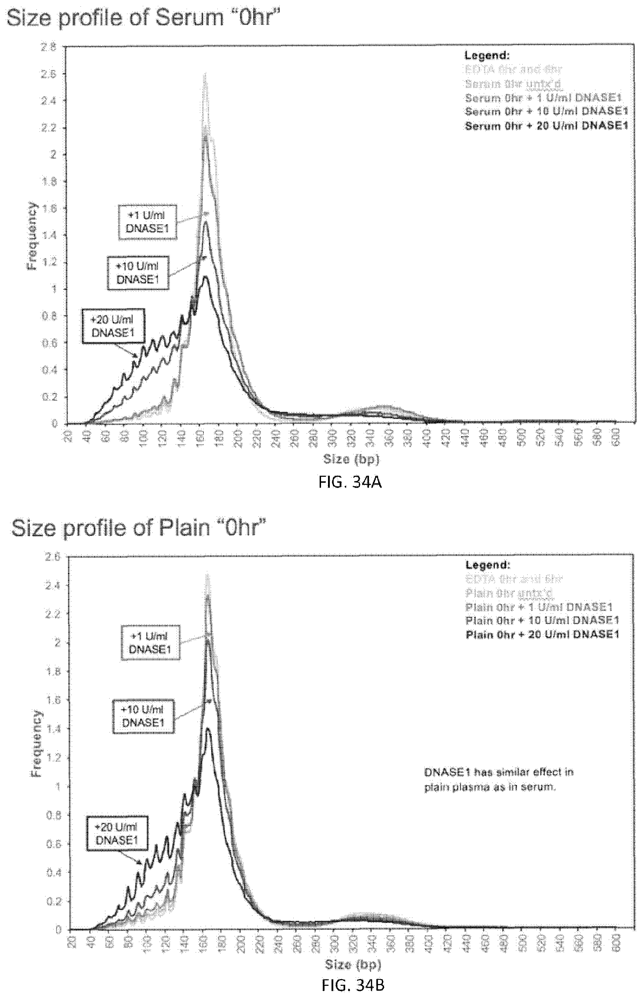

FIG. 34A shows a size profile for serum that is treated with DNASE1 compared to untreated and to EDTA treated (at 9 and 6 hours) according to embodiments of the present disclosure. The more DNASE1 added, the greater shift to smaller DNA fragments. This also shows the dependency of size on the nuclease activity, consistent with FIG. 33. FIG. 34B shows a similar effect in plasma. As denoted in FIGS. 34A and 34B, plain plasma is blood put into a plain (anticoagulant-free) falcon tube and separated immediately at 4° C. In contrast, serum samples that were allowed to clot in an anticoagulant free falcon tube for > 1 h.

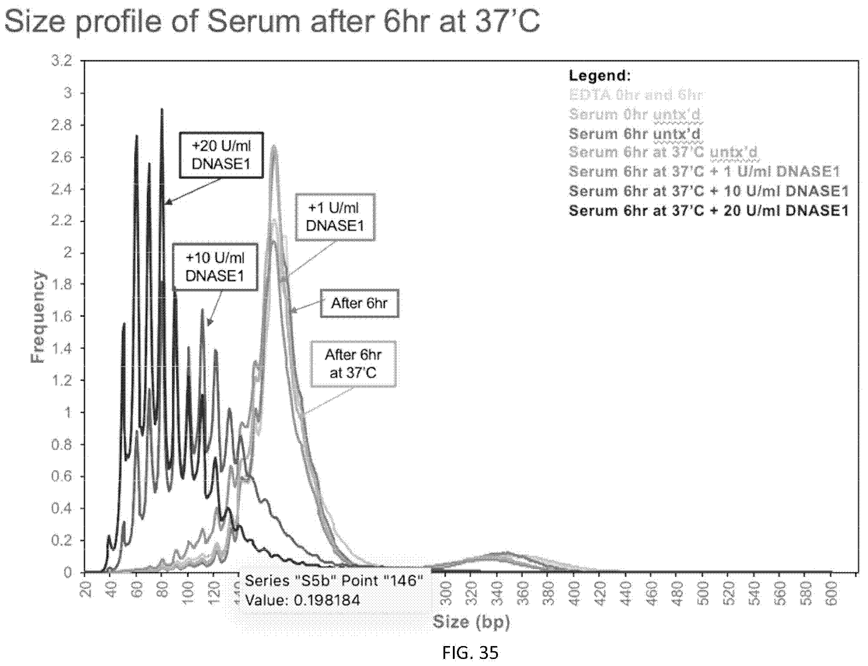

FIG. 35 shows the effect of different doses of DNASE1 on serum after 6 hours according to embodiments of the present disclosure. In the legend, "untx'd" corresponds to "untreated." The effect on size shows even more pronounced shift in the size profile to smaller DNA fragments. FIG. 35 shows that the dependency on size exists when there is incubation. Since the effect is larger than at no incubation (0 h), the difference in the reference values obtained from each sample can be larger, thereby allowing greater classification (discrimination) accuracy since the difference in the reference values for the samples with known classifications will be larger.

### B. DNASE1 activity in urine

Other cell-free samples can be used for any of the methods described herein. As an example urine can be used. The amount of nucleases in plasma can differ from blood, resulting in a different cfDNA profile, including size.

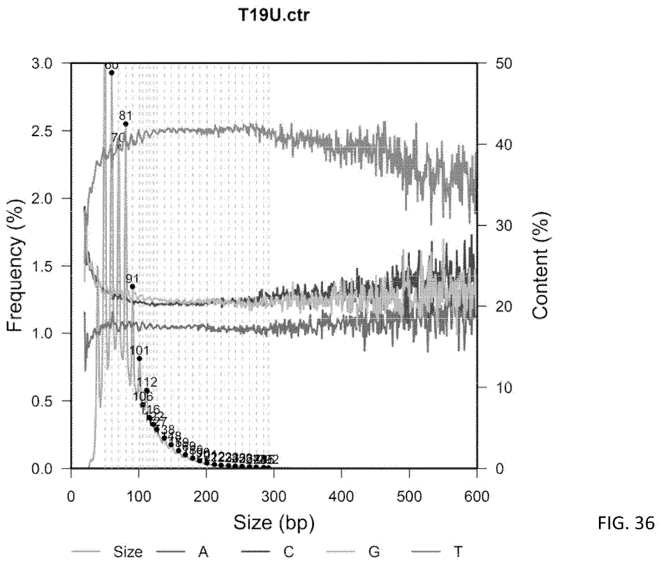

FIG. 36 shows the frequency vs. size and base content vs size in a urine sample according to embodiments of the present disclosure. The T-ends are the highest, as a result of the preference DNASE1 has to cut T-ends. The high T prevalence in urine compared to blood indicates a higher relative activity of DNASE1 in urine than in blood.

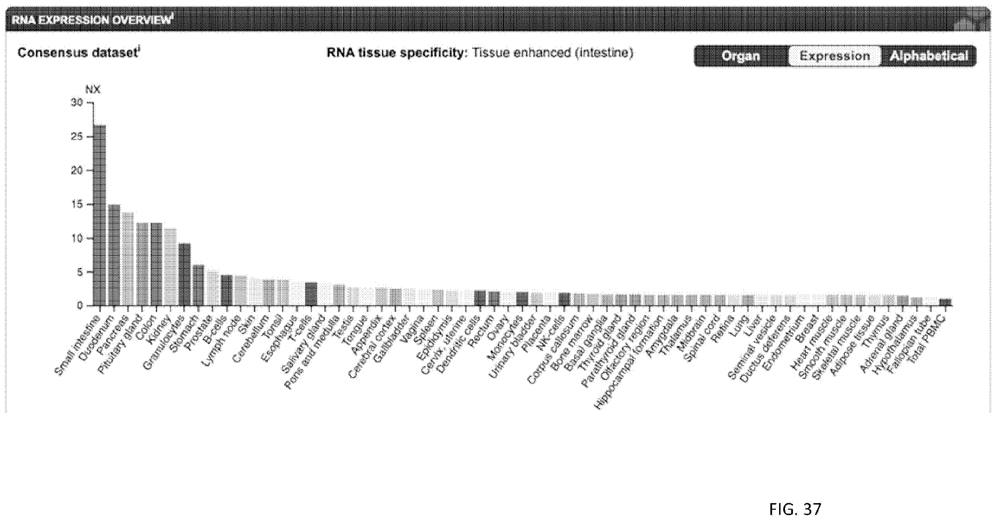

FIG. 37 shows the DNASE1 expression for different tissues. The kidney expression is relatively high compared to blood cells. The higher expression for kidney cells would show itself in urine. This illustrates the correlation of DNASE1 activity and T-end frequency.

### C. Monitoring using amount of a particular base at fragment ends

Accordingly, some embodiments can monitor nuclease activity using an amount of DNA fragments having a particular base at the end. Various figures herein show example data for such monitoring suing samples of one or more subjects.

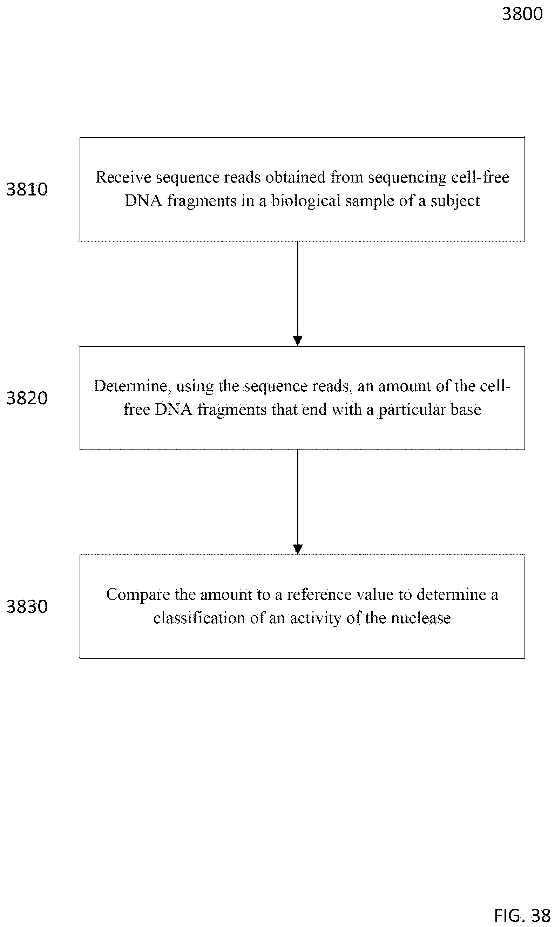

FIG. 38 is a flowchart illustrating a method 3800 for monitoring activity of a nuclease using a biological sample including cell-free DNA according to embodiments of the present disclosure. Aspects of method 3800 can be performed in a similar manner as other methods described herein.

At block 3810, sequence reads are received. The sequence reads can be obtained from sequencing cell-free DNA fragments in a biological sample of a subject.

At block 3820, an amount of the cell-free DNA fragments that end with a particular base are determined using the sequence reads. As with other methods, the particular base may be part of a larger end motif, e.g., a 2-mer, 3-mer, etc. Further, the particular base can be required to be on both ends of a DNA fragment, or a particular pair of different end motifs can be used to select a particular set of DNA fragments.

The amount is an example of a parameter value. The measured amount in this method and other methods can be normalized, e.g., using a property of the sample (e.g., volume or mass of the sample) or using another amount of cell-free DNA fragments or sequence reads satisfying specified criteria (e.g., a total amount of DNA fragment in the sample or a number of fragments with a different end motif). Such normalization can be performed for any of the amounts (parameters) described herein.

At block 3830, the amount is compared to a reference value to determine a classification of an activity of the nuclease. In some embodiments, if the activity is below the reference value, the subject can be classified as having a disorder. In such a case, the subject can be treated, e.g., as described herein. The classification can be a numerical classification value, which can be compared to a cutoff to determine a second classification of whether a gene associated with the nuclease exhibits a genetic disorder in the subject.

The reference value can be a calibration value determined using calibration (reference) samples, which have known classifications and can be analyzed collectively to determine a reference value or calibration function (e.g., when the classifications are continuous variables). For example, the nuclease activity can be a continuous variable, and the comparison of the amount to the reference value can be determine by inputting the amount to a calibration function, e.g., as is described herein.

### D. Monitoring using size of fragments

Embodiments can also provide monitor nuclease activity using an amount of DNA fragments at a particular size range, including at a particular size value. Various figures herein show example data for such monitoring suing samples of one or more subjects.

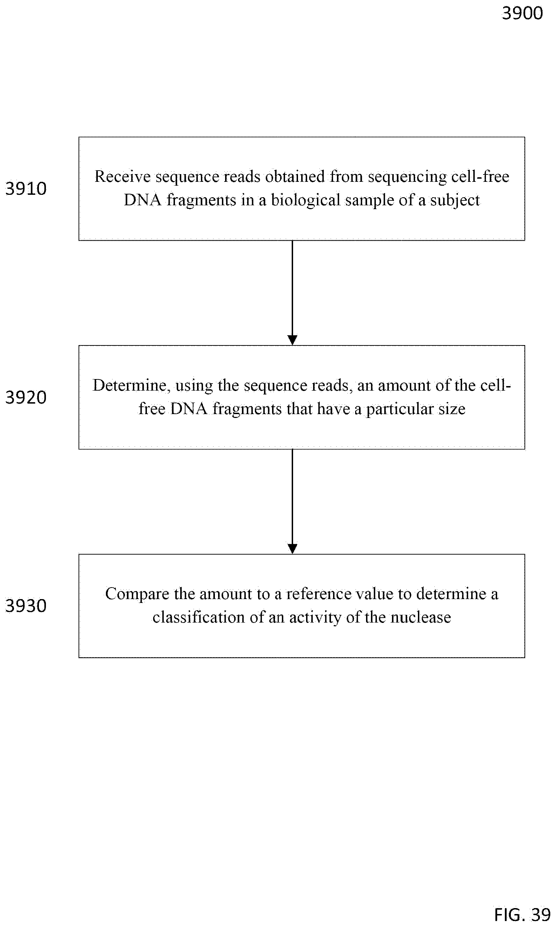

FIG. 39 is a flowchart illustrating a method for monitoring activity of a nuclease using a biological sample including cell-free DNA according to embodiments of the present disclosure. Aspects of method 3800 can be performed in a similar manner as other methods described herein.

At block 3910, sequence reads are received. The sequence reads can be obtained from sequencing cell-free DNA fragments in a biological sample of a subject. The biological sample can be treated with an anticoagulant and incubated for at least a specified amount of time.

At block 3920, an amount of the cell-free DNA fragments that have a particular size are determined using the sequence reads. As with other methods, the particular base may be part of a larger end motif, e.g., a 2-mer, 3-mer, etc. Further, the particular base can be required to be on both ends of a DNA fragment, or a particular pair of different end motifs can be used to select a particular set of DNA fragments. The amount is an example of a parameter value.

At block 3930, the amount is compared to a reference value to determine a classification of an activity of the nuclease. In some embodiments, if the activity is below the reference value, the subject can be classified as having a disorder. In such a case, the subject can be treated, e.g., as described herein.

Regardless of the amount of a particular base or use of size, the reference value can be determined from a calibration sample having a first classification of the activity of the nuclease. If the amount is similar to the reference value, then the biological sample (and the subject from whom it was obtained) can be identified as having the first classification for the nuclease activity. As examples, the first classification can be normal, increased, or decreased.

In various embodiments, comparing the amount to the reference value can include determining whether the amount differs from the reference value by at least a threshold amount. Comparing the amount to the reference value includes determining whether the amount is less than the reference value by at least a threshold amount. Comparing the amount to the reference value includes determining whether the amount is greater than the reference value by at least a threshold amount.

As examples, the nuclease can be DFFB, DNASE1L3, or DNASE1. The biological sample can be obtained from a subjected treated with the nuclease. The method can further include determining a classification of the efficacy of the treatment based on the comparison of the amount to the reference value.

### IX. CALIBRATION OF CLASSIFICATIONS

As described herein, the reference values can be determined using one or more reference (calibration) samples that have a known classification. For example, the reference samples can be known to be healthy or known to have a genetic disorder. As other examples, the reference/calibration samples can have known or measured nuclease activities or efficacy values for a given calibration value (e.g., a parameter including any of the amounts described herein).

The one or more calibration values can be one or more reference values or be used to determine a reference value. The reference values can correspond to particular numerical values for the classifications. For example, calibration data points (calibration value and measured property, such as nuclease activity or level of efficacy) can be analyzed via interpolation or regression to determine a calibration function (e.g., a linear function). Then, a point of the calibration function can be used to determine the numerical classification as an input based on the input of the measured amount or other parameter (e.g., a separation value between two amounts or between a measured amount and a reference value). Such techniques may be applied to any of the method described herein.

For an example with methods 3000 and 3100, the reference value can be determined using one or more reference samples having a known or measured classification for the efficacy of the treatment. The efficacy of treatment for the one or more reference samples can be measured by performing a clotting test on the one or more reference samples. The corresponding amount (e.g., the amount in block 3020 or 3120) can be measured in the one or more reference samples, thereby providing calibration data points comprising the two measurements for the reference/calibration samples. The one or more reference samples can be a plurality of reference samples. A calibration function can be determined that approximates calibration data points corresponding to the measured efficacies and measured amounts for the plurality of reference samples, e.g., by interpolation or regression.

For an example with methods 3800 and 3900, the reference value can be determined using one or more reference samples having a known or measured classification for the activity of the nuclease. The activity of the nuclease for the one or more reference samples can be measured as described herein, e.g., fluorometric or spectrophotometric measurement of cfDNA quantity, which may be done on its own or before, after, and/or in real-time with, the addition of a nuclease-containing sample. Another example is using radial enzyme diffusion methods. The corresponding amount (e.g., the amount in block 3820 or 3920) can be measured in the one or more reference samples, thereby providing calibration data points comprising the two measurements for the reference/calibration samples. The one or more reference samples can be a plurality of reference samples. A calibration function can be determined that approximates calibration data points corresponding to the measured activities and measured amounts for the plurality of reference samples, e.g., by interpolation or regression.

### X. TREATMENT

Embodiments may further include treating the genetic disorder or low nuclease activity (e.g., lower than a threshold) in the patient after determining a classification for the subject. The classification for the subject after treatment may or may not involve adding anticoagulants in vivo or in vitro to enhance the cfDNA end profile. Further, the treatment can be determined as an alternative to a current treatment (e.g., an anticoagulant) when the current dosage has low efficacy, e.g., an increase in dosage or a different anticoagulant can be used. Treatment can be provided according to a determined level of a disorder, any identified mutations, and/or a tissue of origin. For example, an identified mutation (e.g., for polymorphic implementations) can be targeted with a particular drug or chemotherapy. The tissue of origin can be used to guide a surgery or any other form of treatment. And, the level of a disorder can be used to determine how aggressive to be with any type of treatment, which may also be determined based on the level of disorder. A disorder (e.g., cancer) may be treated by chemotherapy, drugs, diet, therapy, and/or surgery. In some embodiments, the more the value of a parameter (e.g., amount or size) exceeds the reference value, the more aggressive the treatment may be.

Treatments may include transurethral bladder tumor resection (TURBT). This procedure is used for diagnosis, staging and treatment. During TURBT, a surgeon inserts a cystoscope through the urethra into the bladder. The tumor is then removed using a tool with a small wire loop, a laser, or high-energy electricity. For patients with NMIBC, TURBT may be used for treating or eliminating the cancer. Another treatment may include radical cystectomy and lymph node dissection. Radical cystectomy is the removal of the whole bladder and possibly surrounding tissues and organs.

Treatment may include chemotherapy, which is the use of drugs to destroy cancer cells, usually by keeping the cancer cells from growing and dividing. The drugs may involve, for example but are not limited to, mitomycin-C (available as a generic drug), gemcitabine (Gemzar), and thiotepa (Tepadina) for intravesical chemotherapy. The systemic chemotherapy may involve, for example but not limited to, cisplatin gemcitabine, methotrexate (Rheumatrex, Trexall), vinblastine (Velban), doxorubicin, and cisplatin.

In some embodiments, treatment may include immunotherapy. Immunotherapy may include immune checkpoint inhibitors that block a protein called PD-1. Inhibitors may include but are not limited to atezolizumab (Tecentriq), nivolumab (Opdivo), avelumab (Bavencio), durvalumab (Imfinzi), and pembrolizumab (Keytruda).

Treatment embodiments may also include targeted therapy. Targeted therapy is a treatment that targets the cancer's specific genes and/or proteins that contributes to cancer growth and survival. For example, erdafitinib is a drug given orally that is approved to treat people with locally advanced or metastatic urothelial carcinoma with FGFR3 or FGFR2 genetic mutations that has continued to grow or spread of cancer cells.

Some treatments may include radiation therapy. Radiation therapy is the use of high-energy x-rays or other particles to destroy cancer cells. In addition to each individual treatment, combinations of these treatments described herein may be used. In some embodiments, when the value of the parameter exceeds a threshold value, which itself exceeds a reference value, a combination of the treatments may be used.

### XI. EXPERIMENTAL MODEL AND SUBJECT DETAILS

### A. Mice

Plasma DNA data for *Dnase1l3*^{-/-} mice were retrieved from the European Genome-phenome Archive (EGA; accession number EGAS00001003174) (Serpas, L. et al. (2019), Proceedings of the National Academy of Sciences 116, 641-649). Mice carrying a targeted allele of *Dnase1* [*Dnase1*^{tm1.1(KOMP)Vlcg}] and mice carrying a targeted allele of *Dffb* [*Dffb*^{C57BL/6N-Dffbem1Wtsi}] both on B6 background were obtained from the Knockout Mouse Project Repository of the University of California at Davis. See "Key Resources Table" for details. The mice were maintained in the Laboratory Animal Center of The Chinese University of Hong Kong (CUHK). All experimental procedures were approved by the Animal Experimentation Ethics committee of CUHK and performed in compliance with "Guide for the Care and Use of Laboratory Animals" (8th edition, 2011) established by the National Institutes of Health. Male and female mice of 13-17 weeks were used for experiments. An analysis on the influence of sex and gender on the results were not done since their blood samples were pooled.

### B. Murine sample collection

Mice were killed and exsanguinated by cardiac puncture. Blood from each mouse was pooled and immediately distributed evenly into experimental conditions: EDTA with 0 h incubation and EDTA with 6 h incubation, or heparin with 0 h incubation and heparin with 6 h incubation. For the *Dffb*^{-/-} experiments, 5 pools of blood were created, each containing blood from 2-4 mice using a total of 14 WT and 14 *Dffb*^{-/-} mice. For the *Dnase1*^{-/-} experiments, one pool was created for each genotype, from a total of 12 WT, 12 *Dnase1*^{+/-}, and 11 *Dnase1*^{-/-}mice. The EDTA tubes were commercially bought 1.3 mL K3E micro tubes (Sarstedt). Heparin tubes were 2 mL microcentrifuge tubes with 18 IU heparin (Sigma-Aldrich) per mL blood added. Incubation was done at room temperature (12-20 °C) on a rocker.

After the room temperature (RT) incubation time was completed, the blood samples were separated by a double centrifugation protocol (1,600 x g for 10 minutes at 4 °C, then recentrifugation of the plasma at 16,000 x g for 10 minutes at 4 °C) (Chiu, R.W.K. et al., (2001), Clinical Chemistry 47, 1607-1613). The resulting plasma was collected, yielding 0.4-1.5 mL of plasma for each condition and time point.

### C. Plasma DNA extraction and library preparation

Plasma DNA was extracted with the QIAamp Circulating Nucleic Acid Kit (Qiagen) according to the manufacturer's protocol. Indexed plasma DNA libraries were constructed using a TruSeq DNA Nano Library Prep Kit according to the manufacturer's instructions. The adaptor-ligated DNA was enriched with 8 cycles of PCR and analyzed on Agilent 4200 TapeStation (Agilent Technologies) using the High Sensitivity D1000 ScreenTape System (Agilent Technologies) for quality control and gel-based size determination. Libraries were quantified by the Qubit dsDNA high sensitivity assay kit (Thermo Fisher Scientific) before sequencing.

### D. DNA sequencing and alignment

Multiplexed DNA libraries were sequenced for 2 x 75 bp paired-end reads on the NextSeq 500 platform (Illumina). Sequences were assigned to their corresponding samples based on their six-base index sequence. Using the Short Oligonucleotide Alignment Program 2 (SOAP2), the paired-end reads from mouse plasma were aligned to the reference mouse genome (NCBI build 37/UCSC mm9; non-repeat-masked) (Li, R. et al., (2009), Bioinformatics 25, 1966-1967). Up to two nucleotide mismatches were allowed. Only paired-end reads aligned to the same chromosome in the correct orientation and spanning an insert size of < 600 bp were retained for downstream analysis. Paired-end reads sharing the same start and end genomic coordinates were deemed PCR duplicates and were discarded from downstream analysis.

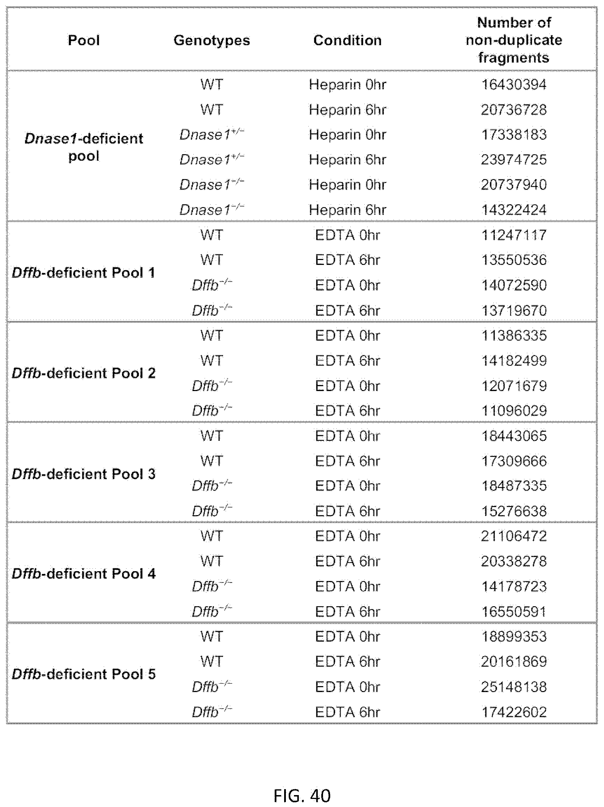

FIG. 40 summarizes the number of non-duplicate fragments obtained for each condition according to embodiments of the present disclosure. The genome coordinates of the aligned ends were used to deduce the size of the whole fragment of the sequenced cfDNA. The deletions of the *Dnase1* and *Dffb* genes were observed after alignment in the *Dnase1*^{-/-} and *Dffb*^{-/-} mice data, respectively.

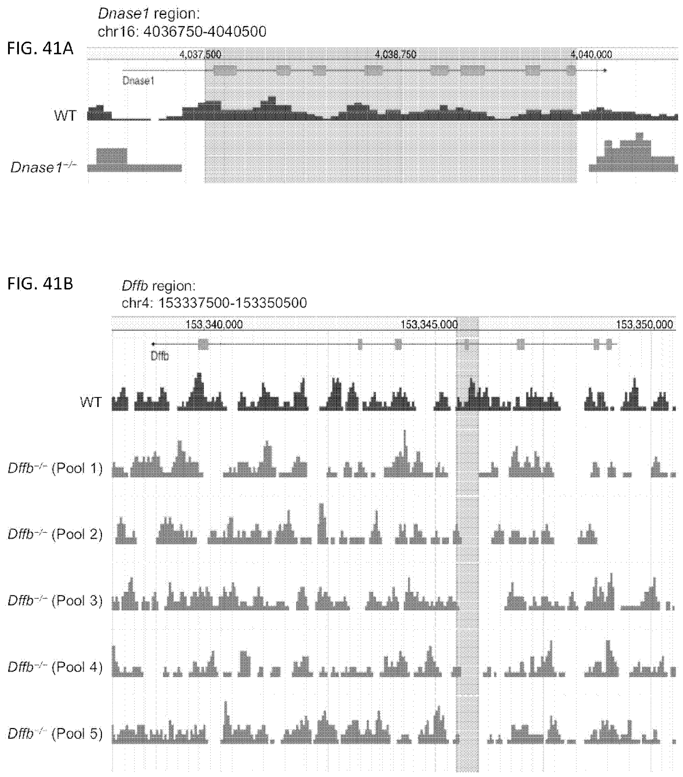

FIGS. 41A-41B show the sequenced read coverage for plasma of WT (blue), *Dnase1*^{-/-}mice (A, red) and *Dffb*^{-/-} mice (Pool 1-5) (B, red). Knockout regions highlighted in yellow.FIG. 41A shows a deletion in the Dnase1 gene for both copies (Dnase1^{-/-}). The WT is on the first row and shows a regular count of sequence reads aligning to the region for the Dnase1 gene. The second row shows a lack of sequence reads for the sample with the deletion. FIG. 41B shows the deletions for the Dffb gene in both copies. The lack of read counts in the region for the Dffb gene is marked by the vertical bar.

### E. Base-end analysis and fragment type analysis

CTCF and Pol II regions were downloaded from the mouse ENCODE project (Shen, Y. et al. (2012), Nature 488, 116-120). The transcription start sites (TSS) of all genes in the reference mouse genome UCSC mm9 were downloaded from UCSC. 10,000 random nonoverlapping regions with 10,000 bp length were randomly selected across the whole genome by BEDTools (v2.27.1) (Quinlan, A.R. and Hall, I.M. (2010), Bioinformatics 26, 841-842). We used a window size ± 500 bp. For the end density analysis, the end density of ± 1500 bp window of CTCF regions was normalized by the median end counts in ± 3000 bp CTCF regions.

For the random, CTCF, and Pol II regions, only cfDNA fragments oriented in the direction of the Watson strand was used for analysis. For the TSS region, only cfDNA fragments oriented in the same direction as the TSS region were used. At each position in these regions, the first nucleotide on the 5' end was identified for each fragment and the base-end percentage was calculated (e.g. A-end fragments / All fragments, with all fragments including A-end, G-end, C-end, and T-end fragments). To analyze the base end percentages by fragment size, both 5' ends (on the respective Watson or Crick strands) of a cfDNA fragment were counted per fragment and the base end percentages at each size were calculated.

For fragment type analysis, each fragment was assigned to a fragment type based on their two ending nucleotides. These fragments where both ends were identified were denoted with their end nucleotides and the symbol <> in between, such that a fragment with both ends as A would be designated as A<>A. All fragments include A<>A, A<>G, A<>C, A<>T, C<>C, C<>G, C<>T, G<>G, G<>T, T<>T fragments. Each fragment type percentages was calculated (e.g. A<>A fragment percent = A<>A fragments / All fragments).

### F. cfDNA quantification

Heparin was found to have significant positive interference with the Qubit dsDNA high sensitivity assay (ThermoFisher Scientific) (data not shown). Instead, the Bio-Rad QX200 Droplet Digital PCR (ddPCR) platform was used for all cfDNA quantification since the heparin interference of DNA target molecules can be ameliorated by the reaction partitioning of ddPCR (Dingle, T.C. et al., (2013), Clin Chem 59, 1670-1672). Heparin samples were diluted 5-fold and at least four wells per sample were done. Mouse cfDNA was quantified by the mouse TaqMan Copy number reference assay (ThermoFisher Scientific) targeting the transferrin receptor gene (Tfrc).

### G. Quantification and statistical analysis

Analysis was performed using custom-built programs written in Python and R languages. Statistical differences were calculated using Mann-Whitney *U* tests unless otherwise specified. A *P* value of less than 0.05 was considered statistically significant and all probabilities were two-tailed.

### XII. EXAMPLE SYSTEMS

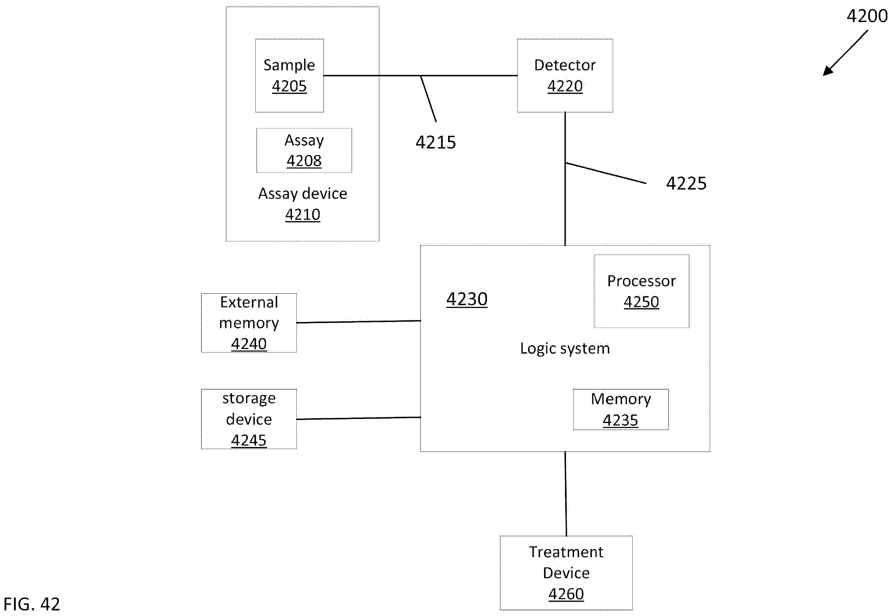

FIG. 42 illustrates a measurement system 4200 according to an embodiment of the present disclosure. The system as shown includes a sample 4205, such as cell-free DNA molecules within an assay device 4210, where an assay 4208 can be performed on sample 4205. For example, sample 4205 can be contacted with reagents of assay 4208 to provide a signal of a physical characteristic 4215. An example of an assay device can be a flow cell that includes probes and/or primers of an assay or a tube through which a droplet moves (with the droplet including the assay). Physical characteristic 4215 (e.g., a fluorescence intensity, a voltage, or a current), from the sample is detected by detector 4220. Detector 4220 can take a measurement at intervals (e.g., periodic intervals) to obtain data points that make up a data signal. In one embodiment, an analog-to-digital converter converts an analog signal from the detector into digital form at a plurality of times. Assay device 4210 and detector 4220 can form an assay system, e.g., a sequencing system that performs sequencing according to embodiments described herein. A data signal 4225 is sent from detector 4220 to logic system 4230. As an example, data signal 4225 can be used to determine sequences and/or locations in a reference genome of DNA molecules. Data signal 4225 can include various measurements made at a same time, e.g., different colors of fluorescent dyes or different electrical signals for different molecule of sample 4205, and thus data signal 4225 can correspond to multiple signals. Data signal 4225 may be stored in a local memory 4235, an external memory 4240, or a storage device 4245.

Logic system 4230 may be, or may include, a computer system, ASIC, microprocessor, etc. It may also include or be coupled with a display (e.g., monitor, LED display, etc.) and a user input device (e.g., mouse, keyboard, buttons, etc.). Logic system 4230 and the other components may be part of a stand-alone or network connected computer system, or they may be directly attached to or incorporated in a device (e.g., a sequencing device) that includes detector 4220 and/or assay device 4210. Logic system 4230 may also include software that executes in a processor 4250. Logic system 4230 may include a computer readable medium storing instructions for controlling measurement system 4200 to perform any of the methods described herein. For example, logic system 4230 can provide commands to a system that includes assay device 4210 such that sequencing or other physical operations are performed. Such physical operations can be performed in a particular order, e.g., with reagents being added and removed in a particular order. Such physical operations may be performed by a robotics system, e.g., including a robotic arm, as may be used to obtain a sample and perform an assay.

Measurement system 4200 may also include a treatment device 4260, which can provide a treatment to the subject. Treatment device 4260 can determine a treatment and/or be used to perform a treatment. Examples of such treatment can include surgery, radiation therapy, chemotherapy, immunotherapy, targeted therapy, hormone therapy, and stem cell transplant. Logic system 4230 may be connected to treatment device 4260, e.g., to provide results of a method described herein. The treatment device may receive inputs from other devices, such as an imaging device and user inputs (e.g., to control the treatment, such as controls over a robotic system).

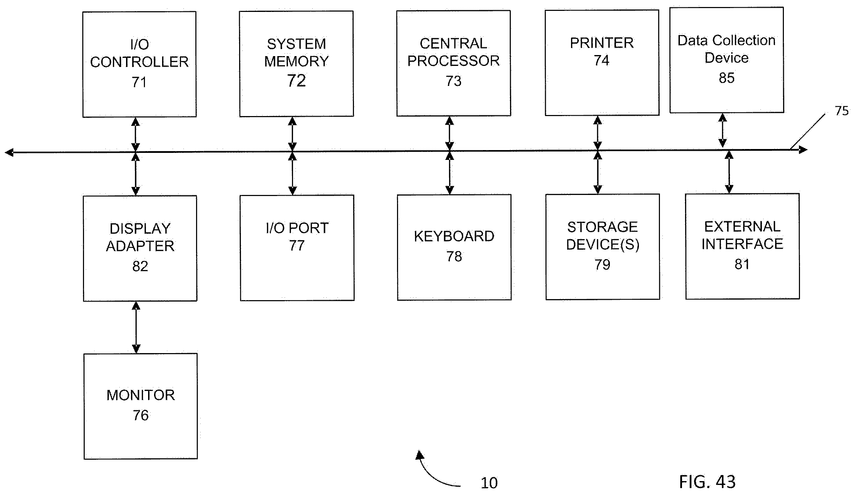

Any of the computer systems mentioned herein may utilize any suitable number of subsystems. Examples of such subsystems are shown in FIG. 43 in computer system 10. In some embodiments, a computer system includes a single computer apparatus, where the subsystems can be the components of the computer apparatus. In other embodiments, a computer system can include multiple computer apparatuses, each being a subsystem, with internal components. A computer system can include desktop and laptop computers, tablets, mobile phones and other mobile devices.

The subsystems shown in FIG. 43 are interconnected via a system bus 75. Additional subsystems such as a printer 74, keyboard 78, storage device(s) 79, monitor 76 (e.g., a display screen, such as an LED), which is coupled to display adapter 82, and others are shown. Peripherals and input/output (I/O) devices, which couple to I/O controller 71, can be connected to the computer system by any number of means known in the art such as input/output (I/O) port 77 (e.g., USB, FireWire^{®}). For example, I/O port 77 or external interface 81 (e.g. Ethernet, Wi-Fi, etc.) can be used to connect computer system 10 to a wide area network such as the Internet, a mouse input device, or a scanner. The interconnection via system bus 75 allows the central processor 73 to communicate with each subsystem and to control the execution of a plurality of instructions from system memory 72 or the storage device(s) 79 (e.g., a fixed disk, such as a hard drive, or optical disk), as well as the exchange of information between subsystems. The system memory 72 and/or the storage device(s) 79 may embody a computer readable medium. Another subsystem is a data collection device 85, such as a camera, microphone, accelerometer, and the like. Any of the data mentioned herein can be output from one component to another component and can be output to the user.

A computer system can include a plurality of the same components or subsystems, e.g., connected together by external interface 81, by an internal interface, or via removable storage devices that can be connected and removed from one component to another component. In some embodiments, computer systems, subsystem, or apparatuses can communicate over a network. In such instances, one computer can be considered a client and another computer a server, where each can be part of a same computer system. A client and a server can each include multiple systems, subsystems, or components.

Aspects of embodiments can be implemented in the form of control logic using hardware circuitry (e.g. an application specific integrated circuit or field programmable gate array) and/or using computer software with a generally programmable processor in a modular or integrated manner. As used herein, a processor can include a single-core processor, multi-core processor on a same integrated chip, or multiple processing units on a single circuit board or networked, as well as dedicated hardware. Based on the disclosure and teachings provided herein, a person of ordinary skill in the art will know and appreciate other ways and/or methods to implement embodiments of the present invention using hardware and a combination of hardware and software.

Any of the software components or functions described in this application may be implemented as software code to be executed by a processor using any suitable computer language such as, for example, Java, C, C++, C#, Objective-C, Swift, or scripting language such as Perl or Python using, for example, conventional or object-oriented techniques. The software code may be stored as a series of instructions or commands on a computer readable medium for storage and/or transmission. A suitable non-transitory computer readable medium can include random access memory (RAM), a read only memory (ROM), a magnetic medium such as a hard-drive or a floppy disk, or an optical medium such as a compact disk (CD) or DVD (digital versatile disk) or Blu-ray disk, flash memory, and the like. The computer readable medium may be any combination of such storage or transmission devices.

Such programs may also be encoded and transmitted using carrier signals adapted for transmission via wired, optical, and/or wireless networks conforming to a variety of protocols, including the Internet. As such, a computer readable medium may be created using a data signal encoded with such programs. Computer readable media encoded with the program code may be packaged with a compatible device or provided separately from other devices (e.g., via Internet download). Any such computer readable medium may reside on or within a single computer product (e.g. a hard drive, a CD, or an entire computer system), and may be present on or within different computer products within a system or network. A computer system may include a monitor, printer, or other suitable display for providing any of the results mentioned herein to a user.

Any of the methods described herein may be totally or partially performed with a computer system including one or more processors, which can be configured to perform the steps. Thus, embodiments can be directed to computer systems configured to perform the steps of any of the methods described herein, potentially with different components performing a respective step or a respective group of steps. Although presented as numbered steps, steps of methods herein can be performed at a same time or at different times or in a different order. Additionally, portions of these steps may be used with portions of other steps from other methods. Additionally, any of the steps of any of the methods can be performed with modules, units, circuits, or other means of a system for performing these steps.

The specific details of particular embodiments may be combined in any suitable manner without departing from the scope of embodiments of the invention. However, other embodiments of the invention may be directed to specific embodiments relating to each individual aspect, or specific combinations of these individual aspects.

The above description of example embodiments of the present disclosure has been presented for the purposes of illustration and description. It is not intended to be exhaustive or to limit the disclosure to the precise form described, and many modifications and variations are possible in light of the teaching above.

A recitation of "a", "an" or "the" is intended to mean "one or more" unless specifically indicated to the contrary. The use of "or" is intended to mean an "inclusive or," and not an "exclusive or" unless specifically indicated to the contrary. Reference to a "first" component does not necessarily require that a second component be provided. Moreover, reference to a "first" or a "second" component does not limit the referenced component to a particular location unless expressly stated. The term "based on" is intended to mean "based at least in part on."

### XIII. REFERENCES

Al-Mayouf, S.M., Sunker, A., Abdwani, R., Abrawi, S.A., Almurshedi, F., Alhashmi, N., Al Sonbul, A., Sewairi, W., Qari, A., Abdallah, E., et al. (2011). Loss-of-function variant in DNASE1L3 causes a familial form of systemic lupus erythematosus. Nat Genet 43, 1186-1188. Chan, K.C.A., Woo, J.K.S., King, A., Zee, B.C.Y., Lam, W.K.J., Chan, S.L., Chu, S.W.I., Mak, C., Tse, I.O.L., Leung, S.Y.M., et al. (2017). Analysis of Plasma Epstein-Barr Virus DNA to Screen for Nasopharyngeal Cancer. New England Journal of Medicine 377, 513-522. Chandrananda, D., Thorne, N.P., and Bahlo, M. (2015). High-resolution characterization of sequence signatures due to non-random cleavage of cell-free DNA. BMC Medical Genomics 8, 29.
Cheng, T.H.T., Lui, K.O., Peng, X.L., Cheng, S.H., Jiang, P., Chan, K.C.A., Chiu, R.W.K., and Lo, Y.M.D. (2018). DNase1 Does Not Appear to Play a Major Role in the Fragmentation of Plasma DNA in a Knockout Mouse Model. Clin Chem 64, 406-408.
Chiu, R.W.K., Chan, K.C.A., Gao, Y., Lau, V.Y.M., Zheng, W., Leung, T.Y., Foo, C.H.F., Xie, B., Tsui, N.B.Y., Lun, F.M.F., et al. (2008). Noninvasive prenatal diagnosis of fetal chromosomal aneuploidy by massively parallel genomic sequencing of DNA in maternal plasma. Proceedings of the National Academy of Sciences of the United States of America 105, 20458-20463.
Chiu, R.W.K., Poon, L.L.M., Lau, T.K., Leung, T.N., Wong, E.M.C., and Lo, Y.M.D. (2001). Effects of Blood-Processing Protocols on Fetal and Total DNA Quantification in Maternal Plasma. Clinical Chemistry 47, 1607-1613.
Dingle, T.C., Sedlak, R.H., Cook, L., and Jerome, K.R. (2013). Tolerance of droplet-digital PCR vs real-time quantitative PCR to inhibitory substances. Clin Chem 59, 1670-1672.
Elmore, S. (2007). Apoptosis: a review of programmed cell death. Toxicologic pathology 35, 495-516.
Errami, Y., Naura, A.S., Kim, H., Ju, J., Suzuki, Y., El-Bahrawy, A.H., Ghonim, M.A., Hemeida, R.A., Mansy, M.S., Zhang, J., et al. (2013). Apoptotic DNA fragmentation may be a cooperative activity between caspase-activated deoxyribonuclease and the poly(ADP-ribose) polymerase-regulated DNAS1L3, an endoplasmic reticulum-localized endonuclease that translocates to the nucleus during apoptosis. The Journal of biological chemistry 288, 3460-3468.
Ivanov, M., Baranova, A., Butler, T., Spellman, P., and Mileyko, V. (2015). Non-random fragmentation patterns in circulating cell-free DNA reflect epigenetic regulation. BMC genomics 16, S1.
Jimenez-Alcazar, M., Rangaswamy, C., Panda, R., Bitterling, J., Simsek, Y.J., Long, A.T., Bilyy, R., Krenn, V., Renne, C., Renne, T., et al. (2017). Host DNases prevent vascular occlusion by neutrophil extracellular traps. Science (New York, NY) 358, 1202-1206.
Klug, A., and Lutter, L.C. (1981). The helical periodicity of DNA on the nucleosome. Nucleic Acids Res 9, 4267-4283.
Koyama, R., Arai, T., Kijima, M., Sato, S., Miura, S., Yuasa, M., Kitamura, D., and Mizuta, R. (2016). DNase γ, DNase I and caspase-activated DNase cooperate to degrade dead cells. Genes to Cells 21, 1150-1163.
Larsen, B.D., and Sørensen, C.S. (2017). The caspase-activated DNase: apoptosis and beyond. The FEBS Journal 284, 1160-1170.
Li, R., Yu, C., Li, Y., Lam, T.-W., Yiu, S.-M., Kristiansen, K., and Wang, J. (2009). SOAP2: an improved ultrafast tool for short read alignment. Bioinformatics 25, 1966-1967.
Lo, Y.M.D., Chan, K.C.A., Sun, H., Chen, E.Z., Jiang, P., Lun, F.M.F., Zheng, Y.W., Leung, T.Y., Lau, T.K., Cantor, C.R., et al. (2010). Maternal Plasma DNA Sequencing Reveals the Genome-Wide Genetic and Mutational Profile of the Fetus. Science Translational Medicine 2, 61ra91-61ra91.
Lo, Y.M.D., Corbetta, N., Chamberlain, P.F., Rai, V., Sargent, I.L., Redman, C.W.G., and Wainscoat, J.S. (1997). Presence of fetal DNA in maternal plasma and serum. The Lancet 350, 485-487.
Manaster, J., Chezar, J., Shurtz-Swirski, R., Shapiro, G., Tendler, Y., Kristal, B., Shasha, S.M., and Sela, S. (1996). Heparin induces apoptosis in human peripheral blood neutrophils. British Journal of Haematology 94, 48-52.
Nagata, S. (2018). Apoptosis and Clearance of Apoptotic Cells. Annual review of immunology 36, 489-517.
Napirei, M., Ludwig, S., Mezrhab, J., Klöckl, T., and Mannherz, H.G. (2009). Murine serum nucleases - contrasting effects of plasmin and heparin on the activities of DNase1 and DNase1-like 3 (DNase1l3). The FEBS Journal 276, 1059-1073.
Napirei, M., Wulf, S., Eulitz, D., Mannherz, H.G., and Kloeckl, T. (2005). Comparative characterization of rat deoxyribonuclease 1 (Dnase1) and murine deoxyribonuclease 1-like 3 (Dnase1l3). The Biochemical journal 389, 355-364.
Ozcakar, Z.B., Foster, J., 2nd, Diaz-Horta, O., Kasapcopur, O., Fan, Y.S., Yalcinkaya, F., and Tekin, M. (2013). DNASE1L3 mutations in hypocomplementemic urticarial vasculitis syndrome. Arthritis Rheum 65, 2183-2189.
Quinlan, A.R., and Hall, I.M. (2010). BEDTools: a flexible suite of utilities for comparing genomic features. Bioinformatics 26, 841-842.
Samejima, K., and Earnshaw, W.C. (2005). Trashing the genome: the role of nucleases during apoptosis. Nature Reviews: Molecular Cell Biology 6, 677-688.
Serpas, L., Chan, R.W.Y., Jiang, P., Ni, M., Sun, K., Rashidfarrokhi, A., Soni, C., Sisirak, V., Lee, W.-S., Cheng, S.H., et al. (2019). Dnase1l3 deletion causes aberrations in length and end-motif frequencies in plasma DNA. Proceedings of the National Academy of Sciences 116, 641-649.
Shen, Y., Yue, F., McCleary, D.F., Ye, Z., Edsall, L., Kuan, S., Wagner, U., Dixon, J., Lee, L., Lobanenkov, V.V., et al. (2012). A map of the cis-regulatory sequences in the mouse genome. Nature 488, 116-120.
Sisirak, V., Sally, B., D'Agati, V., Martinez-Ortiz, W., Özçakar, Z.B., David, J., Rashidfarrokhi, A., Yeste, A., Panea, C., Chida, Asiya S., et al. (2016). Digestion of Chromatin in Apoptotic Cell Microparticles Prevents Autoimmunity. Cell 166, 88-101.
Snyder, M.W., Kircher, M., Hill, A.J., Daza, R.M., and Shendure, J. (2016). Cell-free DNA Comprises an In Vivo Nucleosome Footprint that Informs Its Tissues-Of-Origin. Cell 164, 57-68.
Sun, K., Jiang, P., Cheng, S.H., Cheng, T.H.T., Wong, J., Wong, V.W.S., Ng, S.S.M., Ma, B.B.Y., Leung, T.Y., Chan, S.L., et al. (2019). Orientation-aware plasma cell-free DNA fragmentation analysis in open chromatin regions informs tissue of origin. Genome Research 29, 418-427.
Villeponteau, B. (1992). Heparin increases chromatin accessibility by binding the trypsin-sensitive basic residues in histones. The Biochemical journal 288 (Pt 3), 953-958.
Watanabe, T., Takada, S., and Mizuta, R. (2019). Cell-free DNA in blood circulation is generated by DNase1L3 and caspase-activated DNase. Biochemical and biophysical research communications 516, 790-795.
Widlak, P., and Garrard, W.T. (2005). Discovery, regulation, and action of the major apoptotic nucleases DFF40/CAD and endonuclease G. Journal of cellular biochemistry 94, 1078-1087.
Widlak, P., Li, P., Wang, X., and Garrard, W.T. (2000). Cleavage preferences of the apoptotic endonuclease DFF40 (caspase-activated DNase or nuclease) on naked DNA and chromatin substrates. The Journal of biological chemistry 275, 8226-8232.
Yang, W. (2011). Nucleases: diversity of structure, function and mechanism. Quarterly reviews of biophysics 44, 1-93

## Claims

1. A computer based method for monitoring activity of a nuclease using a biological sample of a subject including cell-free DNA, the method comprising:
receiving sequence reads obtained from sequencing cell-free DNA fragments in the biological sample of the subject;
determining, using the sequence reads, an amount of the cell-free DNA fragments that end with a particular base; and
comparing the amount to a reference value to determine a first classification of an activity of the nuclease.

2. The method of claim 1 , wherein the first classification is a numerical classification value, the method further comprising:
comparing the numerical classification value to a cutoff to determine a second classification of whether a gene associated with the nuclease exhibits a genetic disorder in the subject.

3. The method of claim 2, further comprising:
aligning the sequence reads to a reference genome; and
identifying a first set of sequence reads that end at a particular location or at a specified distance from the particular location in the reference genome, the particular location corresponding to a particular coordinate or a genomic position with a specified property in the reference genome,
wherein the amount corresponds to an amount of the first set of sequence reads that end with the particular base.

4. The method of any one of claim 2 or 3, wherein the genetic disorder is in tumor tissue.

5. The method of any one of the preceding claims, the biological sample is treated with an anticoagulant and incubated for at least a specified amount of time.

6. The method of any one of the preceding claims, wherein comparing the amount to the reference value includes determining whether the amount differs from the reference value by at least a threshold amount.

7. The method of any one of the preceding claims, wherein comparing the amount to the reference value includes determining whether the amount is less than the reference value by at least a threshold amount.

8. The method of any one of the preceding claims, wherein comparing the amount to the reference value includes determining whether the amount is greater than the reference value by at least a threshold amount.

9. The method of any one of the preceding claims, wherein the nuclease is DFFB, DNASE1L3, or DNASE1.

10. The method of any one of the preceding claims, wherein the subject has been provided a treatment with the nuclease before the biological sample was obtained.

11. The method of claim 10, further comprising:
determining a second classification of an efficacy of the treatment with the nuclease based on the comparison of the amount to the reference value.

12. The method of any one of the preceding claims, wherein the reference value is determined using one or more reference samples having a known or measured classification for the activity of the nuclease.

13. The method of claim 12, further comprising:
measuring the activity of the nuclease for the one or more reference samples; and
measuring the amount in the one or more reference samples.

14. The method of claim 13, wherein the one or more reference samples are a plurality of reference samples, the method further comprising:
determining a calibration function that approximates calibration data points corresponding to the measured activities and measured amounts for the plurality of reference samples.

15. A computer program comprising a plurality of instructions that, when executed, control a computer system to perform the method of any one of the preceding claims.

## Patentansprüche

1. Computerbasiertes Verfahren zum Überwachen von Aktivität einer Nuklease unter Verwendung einer biologischen Probe eines Subjekts einschließlich zellfreier DNA, wobei das Verfahren Folgendes umfasst:
Empfangen von Sequenz-Reads, die aus dem Sequenzieren von zellfreien DNA-Fragmenten in der biologischen Probe des Subjekts erhalten werden;
Bestimmen, unter Verwendung der Sequenz-Reads, einer Menge der zellfreien DNA-Fragmente, die mit einer bestimmten Base enden; und
Vergleichen der Menge mit einem Referenzwert, um eine erste Klassifizierung einer Aktivität der Nuklease zu bestimmen.

2. Verfahren nach Anspruch 1, wobei die erste Klassifizierung ein numerischer Klassifizierungswert ist, wobei das Verfahren ferner Folgendes umfasst:
Vergleichen des numerischen Klassifizierungswerts mit einem Cut-off-Wert, um eine zweite Klassifizierung davon zu bestimmen, ob ein Gen im Zusammenhang mit der Nuklease eine genetische Störung in dem Subjekt aufweist.

3. Verfahren nach Anspruch 2, ferner umfassend:
Ausrichten der Sequenz-Reads an einem Referenz-Genom; und
Identifizieren eines ersten Satzes von Sequenz-Reads, die an einer bestimmten Stelle oder in einem bestimmten Abstand zu der bestimmten Stelle in dem Referenz-Genom enden, wobei die bestimmte Stelle einer bestimmten Koordinate oder einer genomischen Position mit einer angegebenen Eigenschaft im Referenz-Genom entspricht,
wobei die Menge einer Menge des ersten Satzes von Sequenz-Reads entspricht, die mit der bestimmten Base enden.

4. Verfahren nach einem aus Anspruch 2 oder 3, wobei die genetische Störung in Tumorgewebe ist.

5. Verfahren nach einem der vorhergehenden Ansprüche, wobei die biologische Probe mit einem Antikoagulans behandelt und für mindestens eine angegebene Zeitdauer inkubiert wird.

6. Verfahren nach einem der vorhergehenden Ansprüche, wobei das Vergleichen der Menge mit dem Referenzwert das Bestimmen beinhaltet, ob sich die Menge von dem Referenzwert um mindestens eine Schwellenwertmenge unterscheidet.

7. Verfahren nach einem der vorhergehenden Ansprüche, wobei das Vergleichen der Menge mit dem Referenzwert das Bestimmen beinhaltet, ob die Menge geringer als der Referenzwert um mindestens eine Schwellenwertmenge ist.

8. Verfahren nach einem der vorhergehenden Ansprüche, wobei das Vergleichen der Menge mit dem Referenzwert das Bestimmen beinhaltet, ob die Menge größer als der Referenzwert um mindestens eine Schwellenwertmenge ist.

9. Verfahren nach einem der vorhergehenden Ansprüche, wobei die Nuklease DFFB, DNASE1L3 oder DNASE1 ist.

10. Verfahren nach einem der vorhergehenden Ansprüche, wobei dem Subjekt eine Behandlung mit der Nuklease bereitgestellt wurde, bevor die biologische Probe erhalten wurde.

11. Verfahren nach Anspruch 10, ferner umfassend:
Bestimmen einer zweiten Klassifizierung einer Wirksamkeit der Behandlung mit der Nuklease basierend auf dem Vergleich der Menge mit dem Referenzwert.

12. Verfahren nach einem der vorhergehenden Ansprüche, wobei der Referenzwert unter Verwendung von einer oder mehreren Referenzproben mit einer bekannten oder gemessenen Klassifizierung für die Aktivität der Nuklease bestimmt wird.

13. Verfahren nach Anspruch 12, ferner umfassend:
Messen der Aktivität der Nuklease für die eine oder mehreren Referenzproben; und
Messen der Menge in der einen oder den mehreren Referenzproben.

14. Verfahren nach Anspruch 13, wobei die eine oder mehreren Referenzproben eine Vielzahl von Referenzproben sind, wobei das Verfahren ferner Folgendes umfasst:
Bestimmen einer Kalibrierfunktion, die sich Kalibrierdatenpunkte, die den gemessenen Aktivitäten und gemessenen Mengen für die Vielzahl von Referenzproben entsprechen, annähert.

15. Computerprogramm, umfassend eine Vielzahl von Anweisungen, die, wenn sie ausgeführt werden, ein Computersystem steuern, um das Verfahren nach einem der vorhergehenden Ansprüche durchzuführen.

## Revendications

1. Procédé informatique pour surveiller l'activité d'une nucléase à l'aide d'un échantillon biologique d'un sujet comprenant de l'ADN acellulaire, le procédé comprenant :
la réception de lectures de séquence obtenues à partir du séquençage de fragments d'ADN acellulaire dans l'échantillon biologique du sujet ;
la détermination, à l'aide des lectures de séquence, d'une quantité de fragments d'ADN acellulaires qui se terminent par une base particulière ; et
la comparaison de la quantité à une valeur de référence pour déterminer une première classification d'une activité de la nucléase.

2. Procédé selon la revendication 1, dans lequel la première classification est une valeur de classification numérique, le procédé comprenant en outre :
la comparaison de la valeur de classification numérique à un seuil pour déterminer une seconde classification indiquant si un gène associé à la nucléase présente un trouble génétique dans le sujet.

3. Procédé selon la revendication 2, comprenant en outre :
l'alignement des lectures de séquence sur un génome de référence ; et
l'identification d'un premier ensemble de lectures de séquence qui se terminent à un emplacement particulier ou à une distance spécifiée de l'emplacement particulier dans le génome de référence, l'emplacement particulier correspondant à une coordonnée particulière ou à une position génomique avec une propriété spécifiée dans le génome de référence,
dans lequel le montant correspond à un montant du premier ensemble de lectures de séquence qui se terminent par la base particulière.

4. Procédé selon l'une quelconque des revendications 2 ou 3, dans lequel le trouble génétique se situe dans le tissu tumoral.

5. Procédé selon l'une quelconque des revendications précédentes, l'échantillon biologique est traité avec un anticoagulant et incubé pendant au moins une durée spécifiée.

6. Procédé selon l'une quelconque des revendications précédentes, dans lequel la comparaison du montant à la valeur de référence comprend la détermination si le montant diffère de la valeur de référence d'au moins un montant seuil.

7. Procédé selon l'une quelconque des revendications précédentes, dans lequel la comparaison du montant à la valeur de référence comprend la détermination si le montant est inférieur à la valeur de référence d'au moins un montant seuil.

8. Procédé selon l'une quelconque des revendications précédentes, dans lequel la comparaison du montant à la valeur de référence comprend la détermination si le montant est supérieur à la valeur de référence d'au moins un montant seuil.

9. Procédé selon l'une quelconque des revendications précédentes, dans lequel la nucléase est DFFB, DNASE1L3 ou DNASE1.

10. Procédé selon l'une quelconque des revendications précédentes, dans lequel le sujet a reçu un traitement avec la nucléase avant l'obtention de l'échantillon biologique.

11. Procédé selon la revendication 10, comprenant en outre :
la détermination d'une seconde classification d'une efficacité du traitement avec la nucléase basée sur la comparaison de la quantité à la valeur de référence.

12. Procédé selon l'une quelconque des revendications précédentes, dans lequel la valeur de référence est déterminée en utilisant un ou plusieurs échantillons de référence ayant une classification connue ou mesurée pour l'activité de la nucléase.

13. Procédé selon la revendication 12, comprenant en outre :
la mesure de l'activité de la nucléase pour les un ou plusieurs échantillons de référence ; et
la mesure de la quantité dans les un ou plusieurs échantillons de référence.

14. Procédé selon la revendication 13, dans lequel les un ou plusieurs échantillons de référence sont une pluralité d'échantillons de référence, le procédé comprenant en outre :
la détermination d'une fonction d'étalonnage qui se rapproche des points de données d'étalonnage correspondant aux activités mesurées et aux quantités mesurées pour la pluralité d'échantillons de référence.

15. Programme informatique comprenant une pluralité d'instructions qui, lorsqu'elles sont exécutées, commandent un système informatique pour exécuter le procédé selon l'une quelconque des revendications précédentes.
